# EUROPEAN PATENT APPLICATION

(11) **EP 1 555 316 A2**
(43) Date of publication of application: **20.07.2005**
(21) Application number: 04078487.8
(22) Date of filing: 10.02.1999
(51) Int. Cl.: C12N 15/10, C07K 19/00, C07K 14/705, C07K 14/54, C07K 14/02, C12N 5/10, G01N 33/566, A61K 48/00

(54) **Antigen library immunization**

(30) Priority: 11.02.1998 US 21769; 11.02.1998 US 74294 P; 23.10.1998 US 105509 P
(62) Divisional of application: 99932510.3
(71) Applicant: Maxygen, Inc., Redwood City, CA 94063 (US)
(72) Inventor: Punnonen, Julha, Belmont, CA 94002 (US); Bass, Steven H., Hillsborough, CA 94010 (US); Whalen, Robert Gerald, 75015 Paris (FR); Howard, Russell, Los Altos Hills, CA 94022 (US); Stemmer, Willem P., Los Gatos, CA 95030 (US)
(74) Representative: Salka, Jeffrey

(57) **Abstract**

This invention is directed to antigen library immunization, which provides methods for obtaining antigens having improved properties for therapeutic and other uses. The methods are useful for obtaining improved antigens that can induce an immune response against pathogens, cancer, and other conditions, as well as antigens that are effective in modulating allergy, inflammatory and autoimmune diseases.

SEQUENCE LISTING

<110> Maxygen, Inc.

<120> Antigen Library Immunization

<130> 0157ep120

<140> 04078487.8
<141> 1999-02-10

<150> US 09/021,769
<151> 1998-02-11

<150> US 60/074,294
<151> 1998-02-11

<150> US 60/105,509
<151> 1998-10-23

<160> 25

<170> PatentIn Ver. 2.0

<210> 1
<211> 1185
<212> DNA
<213> Herpes Simplex Virus 1

<220>
<223> glycoprotein D-1 (gD-1) from Herpes Simplex Virus
      1 (HSV-1)

<400> 1
atggggggga ctgccgccag gttgggggcc gtgattttgt ttgtcgtcat agtgggcctc 60

catggggtcc gcggcaaata tgccttggcg gatgcctctc tcaagatggc cgaccccaat 120

cgctttcgcg gcaaagacct tccggtcctg gaccagctga ccgaccctcc gggggtccgg 180

cgcgtgtacc acatccaggc gggcctaccg gacccgttcc agccccccag cctcccgatc 240

acggtttact acgccgtgtt ggagcgcgcc tgccgcagcg tgctcctaaa cgcaccgtcg 300

gaggcccccc agattgtccg cggggcctcc gaagacgtcc ggaaacaacc ctacaacctg 360

accatcgctt ggtttcggat gggaggcaac tgtgctatcc ccatcacggt catggagtac 420

accgaatgct cctacaacaa gtctctgggg gcctgtccca tccgaacgca gccccgctgg 480

aactactatg acagcttcag cgccgtcagc gaggataacc tggggttcct gatgcacgcc 540

cccgcgtttg agaccgccgg cacgtacctg cggctcgtga agataaacga ctggacggag 600

attacacagt ttatcctgga gcaccgagcc aagggctcct gtaagtacgc cctcccgctg 660

cgcatccccc cgtcagcctg cctctccccc caggcctacc agcagggggt gacggtggac 720

agcatcggga tgctgccccg cttcatcccc gagaaccagc gcaccgtcgc cgtatacagc 780
ttgaagatcg ccgggtggca cgggcccaag gccccataca cgagcaccct gctgcccccg 840

gagctgtccg agacccccaa cgccacgcag ccagaactcg ccccggaaga ccccgaggat 900

tcggccctct tggaggaccc cgtggggacg gtggcgccgc aaatcccacc aaactggcac 960

atcccgtcga tccaggacgc cgcgacgcct taccatcccc cggccacccc gaacaacatg 1020

ggcctgatcg ccggcgcggt gggcggcagt ctcctggcag ccctggtcat ttgcggaatt 1080

gtgtactgga tgcaccgccg cactcggaaa gccccaaagc gcatacgcct cccccacatc 1140

cgggaagacg accagccgtc ctcgcaccag cccttgtttt actag 1185

<210> 2
<211> 1180
<212> DNA
<213> Herpes Simplex Virus 2

<220>
<223> glycoprotein D-1 (gD-1) from Herpes Simplex Virus
      2 (HSV-2)

<400> 2
atggggcgtt tgacctccgg cgtcgggacg gcggccctgc tagttgtcgc ggtgggactc 60

cgcgtcgtct gcgccaaata cgccttagca gacccctcgc ttaagatggc cgatcccaat 120

cgatttcgcg ggaagaacct tccggttttg gaccagctga ccgacccccc cggggtgaag 180

cgtgtttacc acattcagcc gagcctggag gacccgttcc agccccccag catcccgatc 240

actgtgtact acgcagtgct ggaacgtgcc tgccgcagcg tgctcctaca tgccccatcg 300

gaggcccccc agatcgtgcg cggggcttcg gacgaggccc gaaagcacac gtacaacctg 360

accatcgcct ggtatcgcat gggagacaat tgcgctatcc ccatcacggt tatggaatac 420

accgagtgcc cctacaacaa gtcgttgggg gtctgcccca tccgaacgca gccccgctgg 480

agctactatg acagctttag cgccgtcagc gaggataacc tgggattcct gatgcacgcc 540

cccgccttcg agaccgcggg tacgtacctg cggctagtga agataaacga ctggacggag 600

atcacacaat ttatcctgga gcaccgggcc cgcgcctcct gcaagtacgc tctccccctg 660

cgcatccccc cggcagcgtg cctcacctcg aaggcctacc aacagggcgt gacggtcgac 720

agcatcggga tgttaccccg ctttactccc gaaaaccagc gcaccgtcgc cctatacagc 780

ttaaaaatcg ccgggtggca cggccccaag cccccgtaca ccagcaccct gctgccgccg 840

gagctgtccg acaccaccaa cgccacgcaa cccgaactcg ttccggaaga ccccgaggac 900

tcggccctct tagaggatcc cgccgggacg gtgtcttcgc agatcccccc aaactggcac 960

atcccgtcga tccaggacgt cgcgccgcac cacgcccccg ccgccccagc caacccgggc 1020

ctgatcatcg gcgcgctggc cggcagtacc ctggcggcgc tggtcatcgg cggtattgcg 1080

ttttgggtac gccgccggcg ctcagtggcc cccaagcgcc tacgtctccc ccacatccgg 1140

gatgacgacg cgcccccctc gcaccagcca ttgttttact 1180

<210> 3
<211> 1242
<212> DNA
<213> Herpes Simplex Virus 2

<220>
<223> glycoprotein D-2 (gD-2) from Herpes Simplex Virus
      2 (HSV-2)

<400> 3
atggggcgtt tgacctccgg cgtcgggacg gcggccctgc tagttgtcgc ggtgggactc 60

cgcgtcgtct gcgccaaata cgccttagca gacccctcgc ttaagatggc cgatcccaat 120
cgatttcgcg ggaagaacct tccggttttg gaccagctga ccgacccccc cggggtgaag 180
cgtgtttacc acattcagcc gagcctggag gacccgttcc agccccccag catcccgatc 240
actgtgtact acgcagtgct ggaacgtgcc tgccgcagcg tgctcctaca tgccccatcg 300
gaggcccccc agatcgtgcg cggggcttcg gacgaggccc gaaagcacac gtacaacctg 360
accatcgcct ggtatcgcat gggagacaat tgcgctatcc ccatcacggt tatggaatac 420
accgagtgcc cctacaacaa gtcgttgggg gtctgcccca tccgaacgca gccccgctgg 480
agctactatg acagctttag cgccgtcagc gaggataacc tgggattcct catgcacgcc 540
cccgccttcg agaccgcggg tacgtacctg cggctagtga agataaacga ctggacggag 600
atcacacaat ttatcctgga gcaccgggcc cgcgcctcct gcaagtacgc tctccccctg 660
cgcatccccc cggcagcgtg cctcacctcg aaggcctacc aacagggcgt gacggtcgac 720
agcatcggga tgttaccccg ctttatcccc gaaaaccagc gcaccgtcgc cctatacagc 780
ttaaaaatcg ccgggtggca cggccccaag cccccgtaca ccagcaccct gctgccgccg 840
gagctgtccg acaccaccaa cgccacgcaa cccgaactcg ttccggaaga ccccgaggac 900
tcggccctct tagaggatcc cgccgggacg gtgtcttcgc agatcccccc aaactggcac 960
atcccgtcga tccaggacgt cgcgccgcac cacgcccccg ccgcccccag caacccgggc 1020
ctgatcatcg gcgcgctggc cggcagtacc ctggcggcgc tggtcatcgg cggtattgcg 1080
ttttgggtac gccgccgcgc tcagatggcc cccaagcgcc tacgtctccc ccacatccgg 1140
gatgacgacg cgcccccctc gcaccagcca ttgttttact agaggagttt ccccgttccc 1200
gtgtacctct gggcccgtgt gggagggtgg ccggggtatt tg 1242

<210> 4
<211> 31
<212> DNA
<213> Artificial Sequence

<220>
<223> Description of Artificial Sequence:primer 6025F

<400> 4
caagcttctc tatcaaagca gtaagtagta c 31

<210> 5
<211> 25
<212> DNA
<213> Artificial Sequence

<220>
<223> Description of Artificial Sequence:primer 7773R

<400> 5
cttcctgctg ctcccaagaa cccaa 25

<210> 6
<211> 25
<212> DNA

<213> Artificial Sequence

<220>
<223> Description of Artificial Sequence:primer 6196F

<400> 6
atagaaagag cagaagacag tggca                                          25


<210> 7
<211> 25
<212> DNA
<213> Artificial Sequence

<220>
<223> Description of Artificial Sequence:primer 7746R

<400> 7
aacaaagctc ctattcccac tgctc                                          25


<210> 8
<211> 32
<212> DNA
<213> Artificial Sequence

<220>
<223> Description of Artificial Sequence:primer
      BssH2-6205F

<400> 8
ttggcgcgca gaagacagtg gcaatgagag tg                                  32


<210> 9
<211> 846
<212> DNA
<213> Hepatitis B virus

<220>
<221> CDS
<222> (1)..(846)
<223> PreS2-S coding region of hepatitis B virus adr
      surface antigen (HBsAg)

<400> 9
atg cag tgg aac tcc aca aca ttc cac caa gct ctg cta gac ccc aga    48
Met Gln Trp Asn Ser Thr Thr Phe His Gln Ala Leu Leu Asp Pro Arg
  1               5                  10                  15

gtg agg ggc cta tac ttt cct gct ggt ggc tcc agt tcc gga aca gta    96
Val Arg Gly Leu Tyr Phe Pro Ala Gly Gly Ser Ser Ser Gly Thr Val
             20                  25                  30

aac cct gtt ccg act act gcc tca ccc ata tcg tca atc ttc tcg agg    144
Asn Pro Val Pro Thr Thr Ala Ser Pro Ile Ser Ser Ile Phe Ser Arg
         35                  40                  45

act ggg gac cct gca ccg aac atg gag aac aca aca tca gga ttc cta    192
Thr Gly Asp Pro Ala Pro Asn Met Glu Asn Thr Thr Ser Gly Phe Leu
     50                  55                  60

gga ccc ctg ctc gtg tta cag gcg ggg ttt ttc ttg ttg aca aga atc    240
Gly Pro Leu Leu Val Leu Gln Ala Gly Phe Phe Leu Leu Thr Arg Ile
 65                  70                  75                  80

ctc aca ata cca cag agt cta cac tcg tgg tgg act tct ctc aat ttt    288
Leu Thr Ile Pro Gln Ser Leu His Ser Trp Trp Thr Ser Leu Asn Phe
                 85                  90                  95

cta ggg gca gca ccc acg tgt ctt ggc caa aat tcg cag tcc cca acc     336
Leu Gly Ala Ala Pro Thr Cys Leu Gly Gln Asn Ser Gln Ser Pro Thr
            100                 105                 110

tcc aat cac tca cca acc tct tgt cct cca att tgt cct ggt tat cgt     384
Ser Asn His Ser Pro Thr Ser Cys Pro Pro Ile Cys Pro Gly Tyr Arg
        115                 120                 125

tgg atg tgt ctg cgg cgt ttt atc ata ttc ctc ttc atc ctg ctg cta     432
Trp Met Cys Leu Arg Arg Phe Ile Ile Phe Leu Phe Ile Leu Leu Leu
    130                 135                 140

tgc ctc atc ttc ttg ttg gtt ctt ctg gac tac caa ggt atg ttg tct     480
Cys Leu Ile Phe Leu Leu Val Leu Leu Asp Tyr Gln Gly Met Leu Ser
145                 150                 155                 160

gtt tgt cct cta ctt cca aga aca tca act acc agc acg gga cca tgc     528
Val Cys Pro Leu Leu Pro Arg Thr Ser Thr Thr Ser Thr Gly Pro Cys
                165                 170                 175

aag acc tgc acg att cct gct caa gga acc tct atg ttt ccc tct tct     576
Lys Thr Cys Thr Ile Pro Ala Gln Gly Thr Ser Met Phe Pro Ser Ser
            180                 185                 190

tgc tgt aca aaa cct tcg gac gga aac tgc act tgt att ccc atc cca     624
Cys Cys Thr Lys Pro Ser Asp Gly Asn Cys Thr Cys Ile Pro Ile Pro
        195                 200                 205

tca tct tgg gct ttc gca aga ttc cta tgg gag tgg gcc tca gtc cgt     672
Ser Ser Trp Ala Phe Ala Arg Phe Leu Trp Glu Trp Ala Ser Val Arg
    210                 215                 220

ttc tcc tgg ctc agt tta cta gtg cca ttt gtt cag tgg ttc gta ggg     720
Phe Ser Trp Leu Ser Leu Leu Val Pro Phe Val Gln Trp Phe Val Gly
225                 230                 235                 240

ctt tcc ccc act gtt tgg ctt tca gtt ata tgg atg atg tgg tat tgg     768
Leu Ser Pro Thr Val Trp Leu Ser Val Ile Trp Met Met Trp Tyr Trp
                245                 250                 255

ggg cca agt ctg tac aac atc ttg agt ccc ttt tta cct cta tta cca     816
Gly Pro Ser Leu Tyr Asn Ile Leu Ser Pro Phe Leu Pro Leu Leu Pro
            260                 265                 270

att ttc ttt tgt ctt tgg gta tac att tga                             846
Ile Phe Phe Cys Leu Trp Val Tyr Ile
        275                 280

<210> 10
<211> 281
<212> PRT
<213> Hepatitis B virus

<400> 10
Met Gln Trp Asn Ser Thr Thr Phe His Gln Ala Leu Leu Asp Pro Arg
1               5                   10                  15

Val Arg Gly Leu Tyr Phe Pro Ala Gly Gly Ser Ser Ser Gly Thr Val
            20                  25                  30

Asn Pro Val Pro Thr Thr Ala Ser Pro Ile Ser Ser Ile Phe Ser Arg
        35                  40                  45

Thr Gly Asp Pro Ala Pro Asn Met Glu Asn Thr Thr Ser Gly Phe Leu
    50                  55                  60

Gly Pro Leu Leu Val Leu Gln Ala Gly Phe Phe Leu Leu Thr Arg Ile
65                  70                  75                  80

Leu Thr Ile Pro Gln Ser Leu His Ser Trp Trp Thr Ser Leu Asn Phe
                85                  90                  95

Leu Gly Ala Ala Pro Thr Cys Leu Gly Gln Asn Ser Gln Ser Pro Thr
            100                 105                 110

Ser Asn His Ser Pro Thr Ser Cys Pro Pro Ile Cys Pro Gly Tyr Arg
        115                 120                 125

Trp Met Cys Leu Arg Arg Phe Ile Ile Phe Leu Phe Ile Leu Leu Leu
    130                 135                 140

Cys Leu Ile Phe Leu Leu Val Leu Leu Asp Tyr Gln Gly Met Leu Ser
145                 150                 155                 160

Val Cys Pro Leu Leu Pro Arg Thr Ser Thr Thr Ser Thr Gly Pro Cys
                165                 170                 175

Lys Thr Cys Thr Ile Pro Ala Gln Gly Thr Ser Met Phe Pro Ser Ser
            180                 185                 190

Cys Cys Thr Lys Pro Ser Asp Gly Asn Cys Thr Cys Ile Pro Ile Pro
        195                 200                 205

Ser Ser Trp Ala Phe Ala Arg Phe Leu Trp Glu Trp Ala Ser Val Arg
    210                 215                 220

Phe Ser Trp Leu Ser Leu Leu Val Pro Phe Val Gln Trp Phe Val Gly
225                 230                 235                 240

Leu Ser Pro Thr Val Trp Leu Ser Val Ile Trp Met Met Trp Tyr Trp
                245                 250                 255

Gly Pro Ser Leu Tyr Asn Ile Leu Ser Pro Phe Leu Pro Leu Leu Pro
            260                 265                 270

Ile Phe Phe Cys Leu Trp Val Tyr Ile
        275                 280


<210> 11
<211> 846
<212> DNA
<213> Hepatitis B virus

<220>
<221> CDS
<222> (1)..(846)
<223> PreS2-S coding region of hepatitis B virus ayw
      surface antigen (HBsAg)

<400> 11
atg cag tgg aat tcc aca acc ttc cac caa act ctg caa gat ccc aga      48
Met Gln Trp Asn Ser Thr Thr Phe His Gln Thr Leu Gln Asp Pro Arg
1               5                   10                  15

gtg aga ggc ctg tat ttc cct gct ggt ggc tcc agt tca gga aca gta      96
Val Arg Gly Leu Tyr Phe Pro Ala Gly Gly Ser Ser Ser Gly Thr Val
            20                  25                  30

aac cct gtt ctg act act gcc tct ccc tta tcg tca atc ttc tcg agg      144
Asn Pro Val Leu Thr Thr Ala Ser Pro Leu Ser Ser Ile Phe Ser Arg
        35                  40                  45

att ggg gac cct gcg ctg aac atg gag aac atc aca tca gga ttc cta      192
Ile Gly Asp Pro Ala Leu Asn Met Glu Asn Ile Thr Ser Gly Phe Leu

50                  55                  60

gga ccc ctt ctc gtg tta cag gcg ggg ttt ttc ttg ttg aca aga atc      240
Gly Pro Leu Leu Val Leu Gln Ala Gly Phe Phe Leu Leu Thr Arg Ile
65                  70                  75                  80

ctc aca ata ccg cag agt cta gac tcg tgg tgg act tct ctc aat ttt      288
Leu Thr Ile Pro Gln Ser Leu Asp Ser Trp Trp Thr Ser Leu Asn Phe
                85                  90                  95

cta ggg gga act acc gtg tgt ctt ggc caa aat tcg cag tcc cca acc      336
Leu Gly Gly Thr Thr Val Cys Leu Gly Gln Asn Ser Gln Ser Pro Thr
            100                 105                 110

tcc aat cac tca cca acc tct tgt cct cca act tgt cct ggt tat cgc      384
Ser Asn His Ser Pro Thr Ser Cys Pro Pro Thr Cys Pro Gly Tyr Arg
        115                 120                 125

tgg atg tgt ctg cgg cgt ttt atc atc ttc ctc ttc atc ctg ctg cta      432
Trp Met Cys Leu Arg Arg Phe Ile Ile Phe Leu Phe Ile Leu Leu Leu
    130                 135                 140

tgc ctc atc ttc ttg ttg gtt ctt ctg gac tat caa ggt atg ttg ccc      480
Cys Leu Ile Phe Leu Leu Val Leu Leu Asp Tyr Gln Gly Met Leu Pro
145                 150                 155                 160

gtt tgt cct cta att cca gga tcc tca aca acc agc acg gga cca tgc      528
Val Cys Pro Leu Ile Pro Gly Ser Ser Thr Thr Ser Thr Gly Pro Cys
                165                 170                 175

cgg acc tgc atg act act gct caa gga acc tct atg tat ccc tcc tgt      576
Arg Thr Cys Met Thr Thr Ala Gln Gly Thr Ser Met Tyr Pro Ser Cys
            180                 185                 190

tgc tgt acc aaa cct tcg gac gga aat tgc acc tgt att ccc atc cca      624
Cys Cys Thr Lys Pro Ser Asp Gly Asn Cys Thr Cys Ile Pro Ile Pro
        195                 200                 205

tca tcc tgg gct ttc gga aaa ttc cta tgg gag tgg gcc tca gcc cgt      672
Ser Ser Trp Ala Phe Gly Lys Phe Leu Trp Glu Trp Ala Ser Ala Arg
    210                 215                 220

ttc tcc tgg ctc agt tta cta gtg cca ttt gtt cag tgg ttc gta ggg      720
Phe Ser Trp Leu Ser Leu Leu Val Pro Phe Val Gln Trp Phe Val Gly
225                 230                 235                 240

ctt tcc ccc act gtt tgg ctt tca gtt ata tgg atg atg tgg tat tgg      768
Leu Ser Pro Thr Val Trp Leu Ser Val Ile Trp Met Met Trp Tyr Trp
                245                 250                 255

ggg cca agt ctg tac agc atc ttg agt ccc ttt tta ccg ctg tta cca      816
Gly Pro Ser Leu Tyr Ser Ile Leu Ser Pro Phe Leu Pro Leu Leu Pro
            260                 265                 270

att ttc ttt tgt ctt tgg gta tac att taa                              846
Ile Phe Phe Cys Leu Trp Val Tyr Ile
        275                 280

<210> 12
<211> 281
<212> PRT
<213> Hepatitis B virus

<400> 12
Met Gln Trp Asn Ser Thr Thr Phe His Gln Thr Leu Gln Asp Pro Arg
1               5                   10                  15

Val Arg Gly Leu Tyr Phe Pro Ala Gly Gly Ser Ser Ser Gly Thr Val

20                  25                  30

Asn Pro Val Leu Thr Thr Ala Ser Pro Leu Ser Ser Ile Phe Ser Arg
        35                  40                  45

Ile Gly Asp Pro Ala Leu Asn Met Glu Asn Ile Thr Ser Gly Phe Leu
    50                  55                  60

Gly Pro Leu Leu Val Leu Gln Ala Gly Phe Phe Leu Leu Thr Arg Ile
65                  70                  75                  80

Leu Thr Ile Pro Gln Ser Leu Asp Ser Trp Trp Thr Ser Leu Asn Phe
                85                  90                  95

Leu Gly Gly Thr Thr Val Cys Leu Gly Gln Asn Ser Gln Ser Pro Thr
            100                 105                 110

Ser Asn His Ser Pro Thr Ser Cys Pro Pro Thr Cys Pro Gly Tyr Arg
        115                 120                 125

Trp Met Cys Leu Arg Arg Phe Ile Ile Phe Leu Phe Ile Leu Leu Leu
    130                 135                 140

Cys Leu Ile Phe Leu Leu Val Leu Leu Asp Tyr Gln Gly Met Leu Pro
145                 150                 155                 160

Val Cys Pro Leu Ile Pro Gly Ser Ser Thr Thr Ser Thr Gly Pro Cys
                165                 170                 175

Arg Thr Cys Met Thr Thr Ala Gln Gly Thr Ser Met Tyr Pro Ser Cys
            180                 185                 190

Cys Cys Thr Lys Pro Ser Asp Gly Asn Cys Thr Cys Ile Pro Ile Pro
        195                 200                 205

Ser Ser Trp Ala Phe Gly Lys Phe Leu Trp Glu Trp Ala Ser Ala Arg
    210                 215                 220

Phe Ser Trp Leu Ser Leu Leu Val Pro Phe Val Gln Trp Phe Val Gly
225                 230                 235                 240

Leu Ser Pro Thr Val Trp Leu Ser Val Ile Trp Met Met Trp Tyr Trp
                245                 250                 255

Gly Pro Ser Leu Tyr Ser Ile Leu Ser Pro Phe Leu Pro Leu Leu Pro
            260                 265                 270

Ile Phe Phe Cys Leu Trp Val Tyr Ile
        275                 280

<210> 13
<211> 40
<212> DNA
<213> Artificial Sequence

<220>
<223> Description of Artificial Sequence:forward HBV ayw
      PCR primer

<400> 13
ccgggaattc ctcgacacca tgcagtggaa ttccacaacc                          40

<210> 14
<211> 39
<212> DNA
<213> Artificial Sequence

<220>

<223> Description of Artificial Sequence:reverse HBV ayw
      PCR primer

<400> 14
ccggggtacc caaagacaaa agaaaattgg taacagcgg                              39


<210> 15
<211> 849
<212> DNA
<213> Woodchuck hepatitis B virus

<220>
<221> CDS
<222> (1)..(849)
<223> Woodchuck hepatitis B virus (WHV8) surface antigen

<400> 15
atg aaa aat cag act ttt cat ctc cag ggg ttc gta gac gga tta cga      48
Met Lys Asn Gln Thr Phe His Leu Gln Gly Phe Val Asp Gly Leu Arg
  1               5                  10                  15

gac ttg aca aca acg gaa cgc caa cac aat gcc tat gga gat cct ttt      96
Asp Leu Thr Thr Thr Glu Arg Gln His Asn Ala Tyr Gly Asp Pro Phe
             20                  25                  30

aca aca cta agc cct gcg gtt cct act gta tcc acc ata ttg tct cct     144
Thr Thr Leu Ser Pro Ala Val Pro Thr Val Ser Thr Ile Leu Ser Pro
         35                  40                  45

ccc tcg acg act ggg gac cct gca ctg tca ccg gag atg tca cca tca     192
Pro Ser Thr Thr Gly Asp Pro Ala Leu Ser Pro Glu Met Ser Pro Ser
     50                  55                  60

agt ctc cta gga ctc ctc gca gga tta cag gtg gtg tat ttc ttg tgg     240
Ser Leu Leu Gly Leu Leu Ala Gly Leu Gln Val Val Tyr Phe Leu Trp
 65                  70                  75                  80

aca aaa atc cta aca ata gct cag aat cta gat tgg tgg tgg act tct     288
Thr Lys Ile Leu Thr Ile Ala Gln Asn Leu Asp Trp Trp Trp Thr Ser
                 85                  90                  95

ctc agt ttt cca ggg ggc ata cca gag tgc act ggc caa aat tcg cag     336
Leu Ser Phe Pro Gly Gly Ile Pro Glu Cys Thr Gly Gln Asn Ser Gln
            100                 105                 110

ttc caa act tgc aaa cac ttg cca acc tcc tgt cca cca act tgc aat     384
Phe Gln Thr Cys Lys His Leu Pro Thr Ser Cys Pro Pro Thr Cys Asn
        115                 120                 125

ggc ttt cgt tgg atg tat ctg cgg cgt ttt atc ata tac cta tta gtc     432
Gly Phe Arg Trp Met Tyr Leu Arg Arg Phe Ile Ile Tyr Leu Leu Val
    130                 135                 140

ctg ctg ctg tgc ctc atc ttc ttg ttg gtt ctc ctg gac tgg aaa ggt     480
Leu Leu Leu Cys Leu Ile Phe Leu Leu Val Leu Leu Asp Trp Lys Gly
145                 150                 155                 160

tta ata cct gtc tgt cct ctt caa ccc aca aca gaa aca aca gtc aat     528
Leu Ile Pro Val Cys Pro Leu Gln Pro Thr Thr Glu Thr Thr Val Asn
                165                 170                 175

tgc aga caa tgc aca atc tct gca caa gac atg tat act cct cct tac     576
Cys Arg Gln Cys Thr Ile Ser Ala Gln Asp Met Tyr Thr Pro Pro Tyr
            180                 185                 190

tgt tgt tgt tta aaa cct acg gca gga aat tgc act tgt tgg ccc atc     624
Cys Cys Cys Leu Lys Pro Thr Ala Gly Asn Cys Thr Cys Trp Pro Ile

195                 200                 205

cct tca tca tgg gct tta gga aat tac cta tgg gag tgg gcc tta gcc      672
Pro Ser Ser Trp Ala Leu Gly Asn Tyr Leu Trp Glu Trp Ala Leu Ala
    210                 215                 220

cgt ttc tct tgg ctc aat tta cta gtg ccc ttg ctt caa tgg tta gga      720
Arg Phe Ser Trp Leu Asn Leu Leu Val Pro Leu Leu Gln Trp Leu Gly
225                 230                 235                 240

gga att tcc ctc att gcg tgg ttt ttg ctt ata tgg atg att tgg ttt      768
Gly Ile Ser Leu Ile Ala Trp Phe Leu Leu Ile Trp Met Ile Trp Phe
                245                 250                 255

tgg ggg ccc gca ctt ctg agc atc tta ccg cca ttt att ccc ata ttt      816
Trp Gly Pro Ala Leu Leu Ser Ile Leu Pro Pro Phe Ile Pro Ile Phe
            260                 265                 270

gtt ctg ttt ttc ttg att tgg gta tac att tga                          849
Val Leu Phe Phe Leu Ile Trp Val Tyr Ile
        275                 280


<210> 16
<211> 282
<212> PRT
<213> Woodchuck hepatitis B virus

<400> 16
Met Lys Asn Gln Thr Phe His Leu Gln Gly Phe Val Asp Gly Leu Arg
1               5                   10                  15

Asp Leu Thr Thr Thr Glu Arg Gln His Asn Ala Tyr Gly Asp Pro Phe
            20                  25                  30

Thr Thr Leu Ser Pro Ala Val Pro Thr Val Ser Thr Ile Leu Ser Pro
        35                  40                  45

Pro Ser Thr Thr Gly Asp Pro Ala Leu Ser Pro Glu Met Ser Pro Ser
    50                  55                  60

Ser Leu Leu Gly Leu Leu Ala Gly Leu Gln Val Val Tyr Phe Leu Trp
65                  70                  75                  80

Thr Lys Ile Leu Thr Ile Ala Gln Asn Leu Asp Trp Trp Trp Thr Ser
                85                  90                  95

Leu Ser Phe Pro Gly Gly Ile Pro Glu Cys Thr Gly Gln Asn Ser Gln
            100                 105                 110

Phe Gln Thr Cys Lys His Leu Pro Thr Ser Cys Pro Pro Thr Cys Asn
        115                 120                 125

Gly Phe Arg Trp Met Tyr Leu Arg Arg Phe Ile Ile Tyr Leu Leu Val
    130                 135                 140

Leu Leu Leu Cys Leu Ile Phe Leu Leu Val Leu Leu Asp Trp Lys Gly
145                 150                 155                 160

Leu Ile Pro Val Cys Pro Leu Gln Pro Thr Thr Glu Thr Thr Val Asn
                165                 170                 175

Cys Arg Gln Cys Thr Ile Ser Ala Gln Asp Met Tyr Thr Pro Pro Tyr
            180                 185                 190

Cys Cys Cys Leu Lys Pro Thr Ala Gly Asn Cys Thr Cys Trp Pro Ile
        195                 200                 205

Pro Ser Ser Trp Ala Leu Gly Asn Tyr Leu Trp Glu Trp Ala Leu Ala

210                 215                 220

Arg Phe Ser Trp Leu Asn Leu Leu Val Pro Leu Leu Gln Trp Leu Gly
225                 230                 235                 240

Gly Ile Ser Leu Ile Ala Trp Phe Leu Leu Ile Trp Met Ile Trp Phe
                245                 250                 255

Trp Gly Pro Ala Leu Leu Ser Ile Leu Pro Pro Phe Ile Pro Ile Phe
            260                 265                 270

Val Leu Phe Phe Leu Ile Trp Val Tyr Ile
        275                 280


<210> 17
<211> 40
<212> DNA
<213> Artificial Sequence

<220>
<223> Description of Artificial Sequence:forward WHV PCR
      primer

<400> 17
ccgggaattc tcatctccag gggttcgtag acggattacg                          40


<210> 18
<211> 39
<212> DNA
<213> Artificial Sequence

<220>
<223> Description of Artificial Sequence:reverse WHV
      PCR primer

<400> 18
ccggggtacc caaatcaaga aaaacagaac aaatatggg                          39
<210> 19
<211> 20
<212> DNA
<213> Artificial Sequence

<220>
<223> Description of Artificial Sequence:AYWSHFOR primer

<400> 19
gccggcagga aggaaatggg                                               20


<210> 20
<211> 23
<212> DNA
<213> Artificial Sequence

<220>
<223> Description of Artificial Sequence:AYWSHREV primer

<400> 20
ctgctattgt cttcccaatc ctc                                           23


<210> 21
<211> 22
<212> DNA
<213> Artificial Sequence

<220>

<223> Description of Artificial Sequence:WHVSHFOR primer

<400> 21
cgggacatac cacgtggttt ag                                                22


<210> 22
<211> 22
<212> DNA
<213> Artificial Sequence

<220>
<223> Description of Artificial Sequence:WHVSHREV primer

<400> 22
ggcattaaag cagcgtatcc ac                                                22


<210> 23
<211> 16
<212> PRT
<213> Plasmodium berghei

<220>
<221> PEPTIDE
<222> (1)..(16)
<223> circumsporozoite (CS) protein-based B-epitope

<400> 23
Pro Pro Pro Pro Asn Pro Asn Asp Pro Pro Pro Pro Asn Pro Asn Asp
  1               5                  10                  15
<210> 24
<211> 20
<212> PRT
<213> Plasmodium yoelii

<220>
<221> PEPTIDE
<222> (1)..(20)
<223> circumsporozoite (CS) protein-based B-epitope

<400> 24
Gln Gly Pro Gly Ala Pro Gln Gly Pro Gly Ala Pro Gln Gly Pro Gly
  1               5                  10                  15

Ala Pro Gln Gly
             20


<210> 25
<211> 12
<212> PRT
<213> Plasmodium berghei

<220>
<221> PEPTIDE
<222> (1)..(12)
<223> T-helper epitope

<400> 25
Lys Gln Ile Arg Asp Ser Ile Thr Glu Glu Trp Ser
  1               5                  10

## Description

### BACKGROUND OF THE INVENTION

### Field of the Invention

This invention pertains to the field of methods for developing immunogens that can induce efficient immune responses against a broad range of antigens.

### Background

The interactions between pathogens and hosts are results of millions of years of evolution, during which the mammalian immune system has evolved sophisticated means to counterattack pathogen invasions. However, bacterial and viral pathogens have simultaneously gained a number of mechanisms to improve their virulence and survival in hosts, providing a major challenge for vaccine research and development despite the powers of modem techniques of molecular and cellular biology. Similar to the evolution of pathogen antigens, several cancer antigens are likely to have gained means to downregulate their immunogenicity as a mechanism to escape the host immune system.

Efficient vaccine development is also hampered by the antigenic heterogeneity of different strains of pathogens, driven in part by evolutionary forces as means for the pathogens to escape immune defenses. Pathogens also reduce their immunogenicity by selecting antigens that are difficult to express, process and/or transport in host cells, thereby reducing the availability of immunogenic peptides to the molecules initiating and modulating immune responses. The mechanisms associated with these challenges are complex, multivariate and rather poorly characterized. Accordingly, a need exists for vaccines that can induce a protective immune response against bacterial and viral pathogens. The present invention fulfills this and other needs.

### SUMMARY OF THE INVENTION

The present invention provides recombinant multivalent antigenic polypeptides that include a first antigenic determinant from a first disease-associated polypeptide and at least a second antigenic determinant from a second disease-associated polypeptide. The disease-associated polypeptides can be selected from the group consisting of cancer antigens, antigens associated with autoimmunity disorders, antigens associated with inflammatory conditions, antigens associated with allergic reactions, antigens associated with infectious agents. and other antigens that are associated with a disease condition.

In another embodiment, the invention provides a recombinant antigen library that contains recombinant nucleic acids that encode antigenic polypeptides. The libraries are typically obtained by recombining at least first and second forms of a nucleic acid which includes a polynucleotide sequence that encodes a disease-associated antigenic polypeptide, wherein the first and second forms differ from each other in two or more nucleotides, to produce a library of recombinant nucleic acids.

Another embodiment of the invention provides methods of obtaining a polynucleotide that encodes a recombinant antigen having improved ability to induce an immune response to a disease condition. These methods involve: (1) recombining at least first and second forms of a nucleic acid which comprises a polynucleotide sequence that encodes an antigenic polypeptide that is associated with the disease condition, wherein the first and second forms differ from each other in two or more nucleotides, to produce a library of recombinant nucleic acids; and (2) screening the library to identify at least one optimized recombinant nucleic acid that encodes an optimized recombinant antigenic polypeptide that has improved ability to induce an immune response to the disease condition.

These methods optionally further involve: (3) recombining at least one optimized recombinant nucleic acid with a further form of the nucleic acid, which is the same or different from the first and second forms, to produce a further library of recombinant nucleic acids; (4) screening the further library to identify at least one further optimized recombinant nucleic acid that encodes a polypeptide that has improved ability to induce an immune response to the disease condition; and (5) repeating (3) and (4), as necessary, until the further optimized recombinant nucleic acid encodes a polypeptide that has improved ability to induce an immune response to the disease condition.

In some embodiments, the optimized recombinant nucleic acid encodes a multivalent antigenic polypeptide and the screening is accomplished by expressing the library of recombinant nucleic acids in a phage display expression vector such that the recombinant antigen is expressed as a fusion protein with a phage polypeptide that is displayed on a phage particle surface; contacting the phage with a first antibody that is specific for a first serotype of the pathogenic agent and selecting those phage that bind to the first antibody; and contacting those phage that bind to the first antibody with a second antibody that is specific for a second serotype of the pathogenic agent and selecting those phage that bind to the second antibody; wherein those phage that bind to the first antibody and the second antibody express a multivalent antigenic polypeptide.

The invention also provides methods of obtaining a recombinant viral vector which has an enhanced ability to induce an antiviral response in a cell. These methods can include the steps of: (1) recombining at least first and second forms of a nucleic acid which comprise a viral vector, wherein the first and second forms differ from each other in two or more nucleotides, to produce a library of recombinant viral vectors; (2) transfecting the library of recombinant viral vectors into a population of mammalian cells; (3) staining the cells for the presence of Mx protein; and (4) isolating recombinant viral vectors from cells which stain positive for Mx protein, wherein recombinant viral vectors from positive staining cells exhibit enhanced ability to induce an antiviral response.

### BRIEF DESCRIPTION OF THE FIGURES

Figure 1 shows a schematic representation of a method for generating a chimeric, multivalent antigen that has immunogenic regions from multiple antigens. Antibodies to each of the non-chimeric parental immunogenic polypeptides are specific for the respective organisms (A, B, C). After carrying out the recombination and selection methods of the invention, however, a chimeric immunogenic polypeptide is obtained that is recognized by antibodies raised against each of the three parental immunogenic polypeptides.
Figure 2 shows the principle of family DNA shuffling. A family of antigen genes from related pathogens are subjected to shuffling, which results in a library of chimeric and/or mutated antigens. Screening methods are employed to identify those recombinant antigens that are the most immunogenic and/or cross-protective. These can, if desired, be subjected to additional rounds of shuffling and screening.

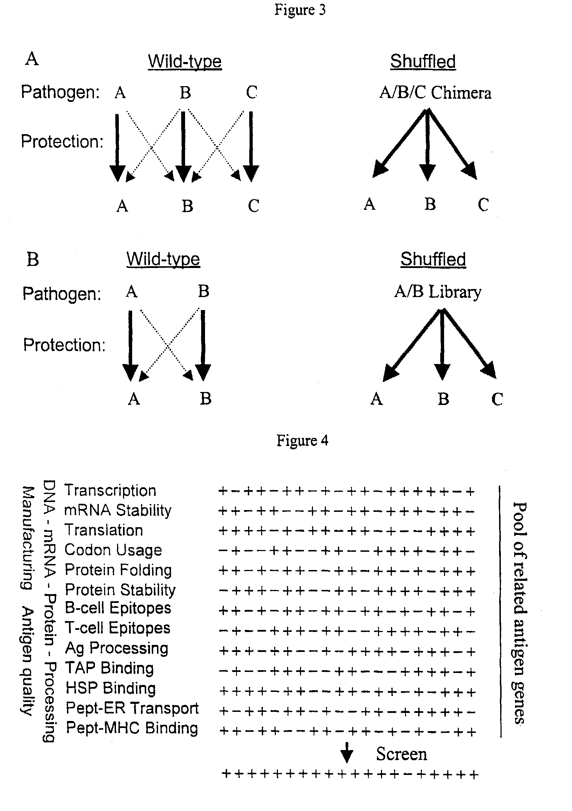

Figure 3A-Figure 3B shows a schematic for a method by which one can obtain recombinant polypeptides that can induce a broad-spectrum immune response. In Figure 3A, wild-type immunogenic polypeptides from the pathogens A, B, and C provide protection against the corresponding pathogen from which the polypeptide is derived, but little or no cross-protection against the other pathogens (left panel). After shuffling, an A/B/C chimeric polypeptide is obtained that can induce a protective immune response against all three pathogen types (right panel). In Figure 3B, shuffling is used with substrate nucleic acids from two pathogen strains (A, B), which encode polypeptides that are protective only against the corresponding pathogen. After shuffling, the resulting chimeric polypeptide can induce an immune response that is effective against not only the two parental pathogen strains, but also against a third strain of pathogen (C).
Figure 4 diagrams some of the possible factors that can determine whether a particular polynucleotide encodes an immunogenic polypeptide having a desired property, such as enhanced immunogenicity and/or cross-reactivity. Those sequence regions that positively affect a particular property are indicated as plus signs along the antigen gene, while those sequence regions that have a negative effect are shown as minus signs. A pool of related antigen genes are shuffled and screened to obtain those that recombinant nucleic acids that have gained positive sequence regions and lost negative regions. No pre-existing knowledge as to which regions are positive or negative for a particular trait is required.
Figure 5 shows a schematic representation of the screening strategy for antigen library screening.
Figure 6 shows a schematic representation of a strategy for pooling and deconvolution as used in antigen library screening.
Figure 7 is an alignment of the nucleotide sequences of glycoprotein D (gD) from HSV-1 (SEQ ID NO:1) and HSV-2 (gD-1 (SEQ ID NO:2) and gD-2 (SEQ ID NO:3)).
Figure 8A shows a diagram of a method for expressing HIV gp120 using genetic vaccine vectors and generation of a library of shuffled gp120 genes. Figure 8B shows PCR primers that are useful for obtaining gp120 nucleic acid substrates for DNA shuffling reactions. Primers suitable for generating substrates include 6025F (SEQ ID NO:4), 7773R (SEQ ID NO:5), and primers suitable for amplifying the shuffled nucleic acids include 6196F (SEQ ID NO:6) and 7746R (SEQ ID NO:7). The primer BssH2-6205F (SEQ ID NO:8) can be used to clone the resulting fragment into a genetic vaccine vector.
Figure 9 shows the domain structure of hepadnavirus envelope genes.
Figure 10 shows a schematic representation of the use of shuffling to obtain hepadnavirus proteins in which the immunogenicity of one antigenic domain is improved.
Figure 11 shows a strategy in which genes that encode the hepadnavirus proteins having one antigenic domain that has improved immunogenicity are shuffled to obtain recombinant proteins in which all three domains have improved immunogenicity.
Figure 12 shows the transmembrane organization of the HBsAg polypeptide.
Figure 13 shows a method for using phage display to obtain recombinant allergens that are not bound by pre-existing IgE.

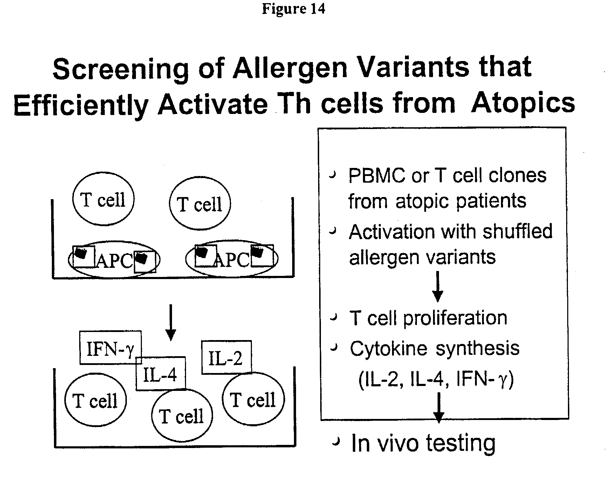

Figure 14 shows a strategy for screening of recombinant allergens to identify those that are effective in activating T_{H} cells. PBMC or T cell clones from atopic individuals are exposed to antigen-presenting cells that display the antigen variants obtained using the methods of the invention. To identify those allergen variants that are effective in activating T cells, the cultures are tested for induction of T cell proliferation or for a pattern of cytokine synthesis that is indicative of the particular type of T cell activation that is desired. If desired, the allergen variants that test positive in the *in vitro* assay can be subjected to *in vivo* testing.
Figure 15 shows a strategy for screening of recombinant cancer antigens to identify those that are effective in activating T cells of cancer patients.

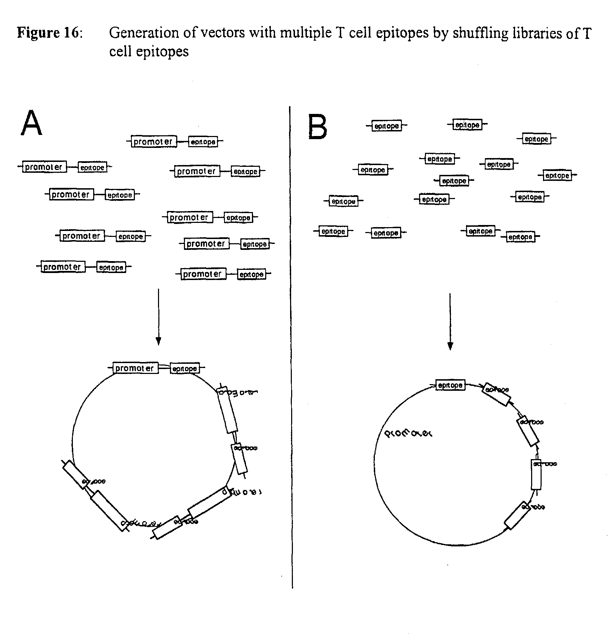

Figure 16A and Figure 16B show two different strategies for generating vectors that contain multiple T cell epitopes obtained, for example, by DNA shuffling. In Figure 16A, each individual shuffled epitope-encoding nucleic acid is linked to a single promoter, and multiple promoter-epitope gene constructs can be placed in a single vector. The scheme shown in Figure 16B involves linking multiple epitope-encoding nucleic acids to a single promoter.
Figure 17 shows the sequences of PreS2-S coding regions (SEQ ID NOS:9 and 11) and corresponding amino acid sequences (SEO ID NOS:10 and 12) of different hepatitis B surface antigen (HBsAg) or woodchuck hepatitis B (WHV) proteins (SEQ ID NOS:15 and 16). Primers suitable for amplification of this region are also shown (HBV, SEQ ID NOS:13 and 14; WHV, SEQ ID NOS:17 and 18).
Figure 18 shows primers (SEQ ID NOS:19-22) that are suitable for amplification of large fragments that contain the S2S coding sequences. The primers hybridize to regions that are approximately 200 bp outside the desired sequences.
Figure 19 shows an alignment of the amino acid sequences of surface antigens from different HVB subtypes (SEQ ID NOS:10 and 12).
Figure 20 shows a diagram of multimeric particles that assemble when an appropriate number of chimeric polypeptides and native HBsAg S monomers are mixed.

### DETAILED DESCRIPTION

### Definitions

The term "screening" describes, in general, a process that identifies optimal antigens. Several properties of the antigen can be used in selection and screening including antigen expression, folding, stability, immunogenicity and presence of epitopes from several related antigens. Selection is a form of screening in which identification and physical separation are achieved simultaneously by expression of a selection marker, which, in some genetic circumstances, allows cells expressing the marker to survive while other cells die (or vice versa). Screening markers include, for example, luciferase, beta-galactosidase and green fluorescent protein. Selection markers include drug and toxin resistance genes, and the like. Because of limitations in studying primary immune responses *in vitro, in vivo* studies are particularly useful screening methods. In these studies, the antigens are first introduced to test animals, and the immune responses are subsequently studied by analyzing protective immune responses or by studying the quality or strength of the induced immune response using lymphoid cells derived from the immunized animal. Although spontaneous selection can and does occur in the course of natural evolution, in the present methods selection is performed by man.

A "exogenous DNA segment", "heterologous sequence" or a "heterologous nucleic acid", as used herein, is one that originates from a source foreign to the particular host cell, or, if from the same source, is modified from its original form. Thus, a heterologous gene in a host cell includes a gene that is endogenous to the particular host cell, but has been modified. Modification of a heterologous sequence in the applications described herein typically occurs through the use of DNA shuffling. Thus, the terms refer to a DNA segment which is foreign or heterologous to the cell, or homologous to the cell but in a position within the host cell nucleic acid in which the element is not ordinarily found. Exogenous DNA segments are expressed to yield exogenous polypeptides.

The term "gene" is used broadly to refer to any segment of DNA associated with a biological function. Thus, genes include coding sequences and/or the regulatory sequences required for their expression. Genes also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

The term "isolated", when applied to a nucleic acid or protein, denotes that the nucleic acid or protein is essentially free of other cellular components with which it is associated in the natural state. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. In particular, an isolated gene is separated from open reading frames which flank the gene and encode a protein other than the gene of interest. The term "purified" denotes that a nucleic acid or protein gives rise to essentially one band in an electrophoretic gel. Particularly, it means that the nucleic acid or protein is at least about 50% pure, more preferably at least about 85% pure, and most preferably at least about 99% pure.

The term "naturally-occurring" is used to describe an object that can be found in nature as distinct from being artificially produced by man. For example, a polypeptide or polynucleotide sequence that is present in an organism (including viruses, bacteria, protozoa, insects, plants or mammalian tissue) that can be isolated from a source in nature and which has not been intentionally modified by man in the laboratory is naturally-occurring.

The term "nucleic acid" refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.* degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer *et al.* (1991) *Nucleic Acid Res. 19:* 5081; Ohtsuka *et al.* (1985) *J. Biol. Chem. 260:* 2605-2608; *Cassol et al.* (1992); Rossolini *et al.* (1994) *Mol. Cell. Probes 8:* 91-98). The term nucleic acid is used interchangeably with gene, cDNA, and mRNA encoded by a gene.

"Nucleic acid derived from a gene" refers to a nucleic acid for whose synthesis the gene, or a subsequence thereof, has ultimately served as a template. Thus, an mRNA, a cDNA reverse transcribed from an mRNA, an RNA transcribed from that cDNA, a DNA amplified from the cDNA, an RNA transcribed from the amplified DNA, *etc.,* are all derived from the gene and detection of such derived products is indicative of the presence and/or abundance of the original gene and/or gene transcript in a sample.

A nucleic acid is "operably linked" when it is placed into a functional relationship with another nucleic acid sequence. For instance, a promoter or enhancer is operably linked to a coding sequence if it increases the transcription of the coding sequence. Operably linked means that the DNA sequences being linked are typically contiguous and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

A specific binding affinity between two molecules, for example, a ligand and a receptor, means a preferential binding of one molecule for another in a mixture of molecules. The binding of the molecules can be considered specific if the binding affinity is about 1 x 10⁴ M⁻¹ to about 1 x 10⁶ M⁻¹ or greater.

The term "recombinant" when used with reference to a cell indicates that the cell replicates a heterologous nucleic acid, or expresses a peptide or protein encoded by a heterologous nucleic acid. Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques.

A "recombinant expression cassette" or simply an "expression cassette" is a nucleic acid construct, generated recombinantly or synthetically, with nucleic acid elements that are capable of effecting expression of a structural gene in hosts compatible with such sequences. Expression cassettes include at least promoters and optionally, transcription termination signals. Typically, the recombinant expression cassette includes a nucleic acid to be transcribed (*e.g.*, a nucleic acid encoding a desired polypeptide), and a promoter. Additional factors necessary or helpful in effecting expression may also be used as described herein. For example, an expression cassette can also include nucleotide sequences that encode a signal sequence that directs secretion of an expressed protein from the host cell. Transcription termination signals, enhancers, and other nucleic acid sequences that influence gene expression, can also be included in an expression cassette.

A "multivalent antigenic polypeptide" or a "recombinant multivalent antigenic polypeptide" is a non-naturally occurring polypeptide that includes amino acid sequences from more than one source polypeptide, which source polypeptide is typically a naturally occurring polypeptide. At least some of the regions of different amino acid sequences constitute epitopes that are recognized by antibodies found in a mammal that has been injected with the source polypeptide. The source polypeptides from which the different epitopes are derived are usually homologous *(i.e.,* have the same or a similar structure and/or function), and are often from different isolates, serotypes, strains, species, of organism or from different disease states, for example.

The terms "identical" or percent "identity," in the context of two or more nucleic acid or polypeptide sequences, refer to two or more sequences or subsequences that are the same or have a specified percentage of amino acid residues or nucleotides that are the same, when compared and aligned for maximum correspondence, as measured using one of the following sequence comparison algorithms or by visual inspection.

The phrase "substantially identical," in the context of two nucleic acids or polypeptides, refers to two or more sequences or subsequences that have at least 60%, preferably 80%, most preferably 90-95% nucleotide or amino acid residue identity, when compared and aligned for maximum correspondence, as measured using one of the following sequence comparison algorithms or by visual inspection. Preferably, the substantial identity exists over a region of the sequences that is at least about 50 residues in length, more preferably over a region of at least about 100 residues, and most preferably the sequences are substantially identical over at least about 150 residues. In some embodiments, the sequences are substantially identical over the entire length of the coding regions.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g.*, by the local homology algorithm of Smith & Waterman, *Adv. Appl. Math.* 2:482 (1981), by the homology alignment algorithm ofNeedleman & Wunsch, *J. Mol. Biol.* 48:443 (1970), by the search for similarity method of Pearson & Lipman, *Proc. Nat'l. Acad. Sci. USA* 85:2444 (1988), by computerized implementations of these algorithms (GAP, BESTFIT, PASTA, and TFASTA in the Wisconsin Genetics Software Package, Genetics Computer Group, 575 Science Dr., Madison, WI), or by visual inspection *(see generally* Ausubel *et al., infra).*

One example of an algorithm that is suitable for determining percent sequence identity and sequence similarity is the BLAST algorithm, which is described in Altschul *et al., J. Mol. Biol.* 215:403-410 (1990). Software for performing BLAST analyses is publicly available through the National Center for Biotechnology Information (http://www.ncbi.nlm.nih.gov/. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra).* These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix *(see* Henikoff & Henikoff (1989) *Proc. Natl. Acad. Sci. USA* 89:10915).

In addition to calculating percent sequence identity, the BLAST algorithm also performs a statistical analysis of the similarity between two sequences *(see, e.g.,* Karlin & Altschul (1993) *Proc. Nat'l. Acad. Sci. USA* 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

Another indication that two nucleic acid sequences are substantially identical is that the two molecules hybridize to each other under stringent conditions. The phrase "hybridizing specifically to", refers to the binding, duplexing, or hybridizing of a molecule only to a particular nucleotide sequence under stringent conditions when that sequence is present in a complex mixture (*e.g.*, total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

"Stringent hybridization conditions" and "stringent hybridization wash conditions" in the context of nucleic acid hybridization experiments such as Southern and northern hybridizations are sequence dependent, and are different under different environmental parameters. Longer sequences hybridize specifically at higher temperatures. An extensive guide to the hybridization of nucleic acids is found in Tijssen (1993) *Laboratory Techniques in Biochemistry and Molecular Biology--Hybridization with Nucleic Acid Probes* part I chapter 2 "Overview of principles of hybridization and the strategy of nucleic acid probe assays", Elsevier, New York. Generally, highly stringent hybridization and wash conditions are selected to be about 5° C lower than the thermal melting point (Tₘ) for the specific sequence at a defined ionic strength and pH. Typically, under "stringent conditions" a probe will hybridize to its target subsequence, but to no other sequences.

The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids which have more than 100 complementary residues on a filter in a Southern or northern blot is 50% formamide with 1 mg of heparin at 42°C, with the hybridization being carried out overnight. An example of highly stringent wash conditions is 0.15M NaCl at 72°C for about 15 minutes. An example of stringent wash conditions is a 0.2x SSC wash at 65°C for 15 minutes (*see,* Sambrook, *infra.,* for a description of SSC buffer). Often, a high stringency wash is preceded by a low stringency wash to remove background probe signal. An example medium stringency wash for a duplex of, *e.g.*, more than 100 nucleotides, is 1x SSC at 45°C for 15 minutes. An example low stringency wash for a duplex of, *e.g.*, more than 100 nucleotides, is 4-6x SSC at 40°C for 15 minutes. For short probes (*e.g.*, about 10 to 50 nucleotides), stringent conditions typically involve salt concentrations of less than about 1.0 M Na⁺ ion, typically about 0.01 to 1.0 M Na⁺ ion concentration (or other salts) at pH 7.0 to 8.3, and the temperature is typically at least about 30°C. Stringent conditions can also be achieved with the addition of destabilizing agents such as formamide. In general, a signal to noise ratio of 2x (or higher) than that observed for an unrelated probe in the particular hybridization assay indicates detection of a specific hybridization. Nucleic acids which do not hybridize to each other under stringent conditions are still substantially identical if the polypeptides which they encode are substantially identical. This occurs, *e.g.,* when a copy of a nucleic acid is created using the maximum codon degeneracy permitted by the genetic code.

A further indication that two nucleic acid sequences or polypeptides are substantially identical is that the polypeptide encoded by the first nucleic acid is immunologically cross reactive with, or specifically binds to, the polypeptide encoded by the second nucleic acid. Thus, a polypeptide is typically substantially identical to a second polypeptide, for example, where the two peptides differ only by conservative substitutions.

The phrase "specifically (or selectively) binds to an antibody" or "specifically (or selectively) immunoreactive with", when referring to a protein or peptide, refers to a binding reaction which is determinative of the presence of the protein, or an epitope from the protein, in the presence of a heterogeneous population of proteins and other biologies. Thus, under designated immunoassay conditions, the specified antibodies bind to a particular protein and do not bind in a significant amount to other proteins present in the sample. The antibodies raised against a multivalent antigenic polypeptide will generally bind to the proteins from which one or more of the epitopes were obtained. Specific binding to an antibody under such conditions may require an antibody that is selected for its specificity for a particular protein. A variety of immunoassay formats may be used to select antibodies specifically immunoreactive with a particular protein. For example, solid-phase ELISA immunoassays, Western blots, or immunohistochemistry are routinely used to select monoclonal antibodies specifically immunoreactive with a protein. *See* Harlow and Lane *(1988) Antibodies, A Laboratory Manual,* Cold Spring Harbor Publications, New York "Harlow and Lane"), for a description of immunoassay formats and conditions that can be used to determine specific immunoreactivity. Typically a specific or selective reaction will be at least twice background signal or noise and more typically more than 10 to 100 times background.

"Conservatively modified variations" of a particular polynucleotide sequence refers to those polynucleotides that encode identical or essentially identical amino acid sequences, or where the polynucleotide does not encode an amino acid sequence, to essentially identical sequences. Because of the degeneracy of the genetic code, a large number of functionally identical nucleic acids encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. Such nucleic acid variations are "silent variations," which are one species of "conservatively modified variations." Every polynucleotide sequence described herein which encodes a polypeptide also describes every possible silent variation, except where otherwise noted. One of skill will recognize that each codon in a nucleic acid (except AUG, which is ordinarily the only codon for methionine) can be modified to yield a functionally identical molecule by standard techniques. Accordingly, each "silent variation" of a nucleic acid which encodes a polypeptide is implicit in each described sequence.

Furthermore, one of skill will recognize that individual substitutions, deletions or additions which alter, add or delete a single amino acid or a small percentage of amino acids (typically less than 5%, more typically less than 1%) in an encoded sequence are "conservatively modified variations" where the alterations result in the substitution of an amino acid with a chemically similar amino acid. Conservative substitution tables providing functionally similar amino acids are well known in the art. The following five groups each contain amino acids that are conservative substitutions for one another:
Aliphatic: Glycine (G), Alanine (A), Valine (V), Leucine (L), Isoleucine (I);
Aromatic: Phenylalanine (F), Tyrosine (Y), Tryptophan (W);
Sulfur-containing: Methionine (M), Cysteine (C);
Basic: Arginine (R), Lysine (K), Histidine (H);
Acidic: Aspartic acid (D), Glutamic acid (E), Asparagine (N), Glutamine (Q).
*See also,* Creighton (1984) *Proteins,* W.H. Freeman and Company, for additional groupings of amino acids. In addition, individual substitutions, deletions or additions which alter, add or delete a single amino acid or a small percentage of amino acids in an encoded sequence are also "conservatively modified variations".

A "subsequence" refers to a sequence of nucleic acids or amino acids that comprise a part of a longer sequence of nucleic acids or amino acids (*e.g.*, polypeptide) respectively.

### Description of the Preferred Embodiments

The invention provides a new approach to vaccine development, which is termed "antigen library immunization." No other technologies are available for generating libraries of related antigens or optimizing known protective antigens. The most powerful previously existing methods for identification of vaccine antigens, such as high throughput sequencing or expression library immunization, only explore the sequence space provided by the pathogen genome. These approaches are likely to be insufficient, because they generally only target single pathogen strains, and because natural evolution has directed pathogens to downregulate their own immunogenicity. In contrast, the immunization protocols of the invention, which use shuffled antigen libraries, provide a means to identify novel antigen sequences. Those antigens that are most protective can be selected from these pools by *in vivo* challenge models. Antigen library immunization dramatically expands the diversity of available immunogen sequences, and therefore, these antigen chimera libraries can also provide means to defend against newly emerging pathogen variants of the future. The methods of the invention enable the identification of individual chimeric antigens that provide efficient protection against a variety of existing pathogens, providing improved vaccines for troops and civilian populations.

The methods of the invention provide an evolution-based approach, such as DNA shuffling in particular, that is an optimal approach to improve the immunogenicity of many types of antigens. For example, the methods provide means of obtaining optimized cancer antigens useful for preventing and treating malignant diseases. Furthermore, an increasing number of self-antigens, causing autoimmune diseases, and allergens, causing atopy, allergy and asthma, have been characterized. The immunogenicity and manufacturing of these antigens can likewise be improved with the methods of this invention.

The antigen library immunization methods of the invention provide a means by which one can obtain a recombinant antigen that has improved ability to induce an immune response to a pathogenic agent. A "pathogenic agent" refers to an organism or virus that is capable of infecting a host cell. Pathogenic agents typically include and/or encode a molecule, usually a polypeptide, that is immunogenic in that an immune response is raised against the immunogenic polypeptide. Often, the immune response raised against an immunogenic polypeptide from one serotype of the pathogenic agent is not capable of recognizing, and thus protecting against, a different serotype of the pathogenic agent, or other related pathogenic agents. In other situations, the polypeptide produced by a pathogenic agent is not produced in sufficient amounts, or is not sufficiently immunogenic, for the infected host to raise an effective immune response against the pathogenic agent.

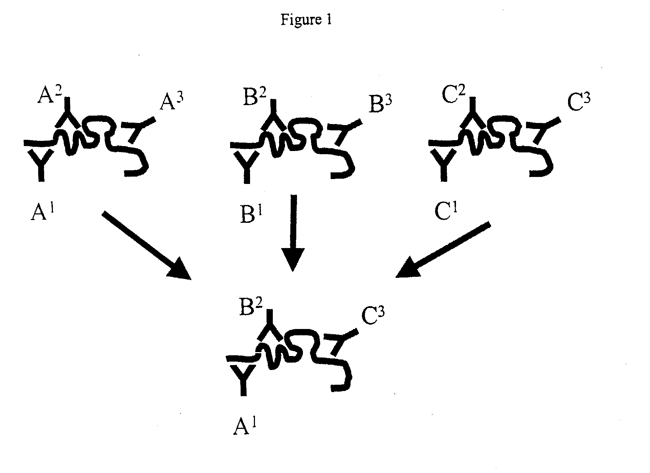

These problems are overcome by the methods of the invention, which typically involve recombining two or more forms of a nucleic acid that encode a polypeptide of the pathogenic agent, or antigen involved in another disease or condition. These recombination methods, referred to herein as "DNA shuffling", use as substrates forms of the nucleic acid that differ from each other in two or more nucleotides, so a library of recombinant nucleic acids results. The library is then screened to identify at least one optimized recombinant nucleic acid that encodes an optimized recombinant antigen that has improved ability to induce an immune response to the pathogenic agent or other condition. The resulting recombinant antigens often are chimeric in that they are recognized by antibodies (Abs) reacting against multiple pathogen strains, and generally can also elicit broad spectrum immune responses. Specific neutralizing antibodies are known to mediate protection against several pathogens of interest, although additional mechanisms, such as cytotoxic T lymphocytes, are likely to be involved. The concept of chimeric, multivalent antigens inducing broadly reacting antibody responses is further illustrated in Figure 1.

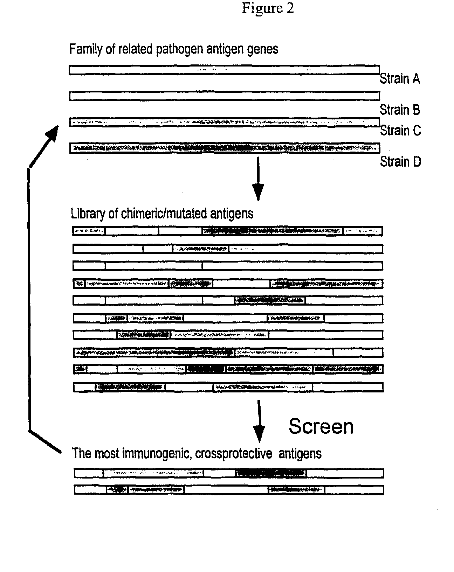

In preferred embodiments, the different forms of the nucleic acids that encode antigenic polypeptides are obtained from members of a family of related pathogenic agents. This scheme of performing DNA shuffling using nucleic acids from related organisms, known as "family shuffling," is described in Crameri *et al.* ((1998) *Nature* 391: 288-291) and is shown schematically in Figure 2. Polypeptides of different strains and serotypes of pathogens generally vary between 60-98%, which will allow for efficient family DNA shuffling. Therefore, family DNA shuffling provides an effective approach to generate multivalent, crossprotective antigens. The methods are useful for obtaining individual chimeras that effectively protect against most or all pathogen variants (Figure 3A). Moreover, immunizations using entire libraries or pools of shuffled antigen chimeras can also result in identification of chimeric antigens that protect against pathogen variants that were not included in the starting population of antigens (for example, protection against strain C by shuffled library of chimeras/mutants of strains A and B in Figure 3B). Accordingly, the antigen library immunization approach enables the development of immunogenic polypeptides that can induce immune responses against poorly characterized, newly emerging pathogen variants.

Sequence recombination can be achieved in many different formats and permutations of formats, as described in further detail below. These formats share some common principles. For example, the targets for modification vary in different applications, as does the property sought to be acquired or improved. Examples of candidate targets for acquisition of a property or improvement in a property include genes that encode proteins which have immunogenic and/or toxigenic activity when introduced into a host organism.

The methods use at least two variant forms of a starting target. The variant forms of candidate substrates can show substantial sequence or secondary structural similarity with each other, but they should also differ in at least one and preferably at least two positions. The initial diversity between forms can be the result of natural variation, *e.g.*, the different variant forms (homologs) are obtained from different individuals or strains of an organism, or constitute related sequences from the same organism (*e.g.*, allelic variations), or constitute homologs from different organisms (interspecific variants). Alternatively, initial diversity can be induced, *e.g.*, the variant forms can be generated by error-prone transcription, such as an error-prone PCR or use of a polymerase which lacks proof-reading activity (*see*, Liao (1990) *Gene* 88:107-111), of the first variant form, or, by replication of the first form in a mutator strain (mutator host cells are discussed in further detail below, and are generally well known). A mutator strain can include any mutants in any organism impaired in the functions of mismatch repair. These include mutant gene products of *mutS, mutT, mutH, mutL, ovrD, dcm, vsr, umuC, umuD, sbcB, recJ, etc.* The impairment is achieved by genetic mutation, allelic replacement, selective inhibition by an added reagent such as a small compound or an expressed antisense RNA, or other techniques. Impairment can be of the genes noted, or of homologous genes in any organism. Other methods of generating initial diversity include methods well known to those of skill in the art, including, for example, treatment of a nucleic acid with a chemical or other mutagen, through spontaneous mutation, and by inducing an error-prone repair system (*e.g.*, SOS) in a cell that contains the nucleic acid. The initial diversity between substrates is greatly augmented in subsequent steps of recombination for library generation.

### Properties involved in immunogenicity

The effectiveness of an antigen in inducing an immune response against a pathogen can depend upon several factors, many of which are not well understood. Most previously available methods for increasing the effectiveness of antigens are dependent upon understanding the molecular basis for these factors. However, DNA shuffling and antigen library immunization according to the methods of the invention are effective even where the molecular bases are unknown. The methods of the invention do not rely upon *a priori* assumptions.

Polynucleotide sequences that can positively or negatively affect the immunogenicity of an antigen encoded by the polynucleotide are often scattered throughout the entire antigen gene. Several of these factors are shown diagrammatically in Figure 4. By recombining different forms of polynucleotide that encode the antigen using DNA shuffling, followed by selection for those chimeric polynucleotides that encode an antigen that can induce an improved immune response, one can obtain primarily sequences that have a positive influence on antigen immunogenicity. Those sequences that negatively affect antigen immunogenicity are eliminated (Figure 4). One need not know the particular sequences involved.

### DNA Shuffling Methods

Generally, the methods of the invention entail performing DNA recombination ("shuffling") and screening or selection to "evolve" individual genes, whole plasmids or viruses, multigene clusters, or even whole genomes (Stemmer (1995) *Bio*/*Technology* 13:549-553). Reiterative cycles of recombination and screening/selection can be performed to further evolve the nucleic acids of interest. Such techniques do not require the extensive analysis and computation required by conventional methods for polypeptide engineering. Shuffling allows the recombination of large numbers of mutations in a minimum number of selection cycles, in contrast to natural pair-wise recombination events (e.g., as occur during sexual replication). Thus, the sequence recombination techniques described herein provide particular advantages in that they provide recombination between mutations in any or all of these, thereby providing a very fast way of exploring the manner in which different combinations of mutations can affect a desired result. In some instances, however, structural and/or functional information is available which, although not required for sequence recombination, provides opportunities for modification of the technique.

The DNA shuffling methods of the invention can involve at least one of at least four different approaches to improve immunogenic activity as well as to broaden specificity. First, DNA shuffling can be performed on a single gene. Secondly, several highly homologous genes can be identified by sequence comparison with known homologous genes. These genes can be synthesized and shuffled as a family of homologs, to select recombinants with the desired activity. The shuffled genes can be cloned into appropriate host cells, such as *E. coli,* yeast, plants, fungi, animal cells, and the like, and those that encode antigens having the desired properties can be identified by the methods described below. Third, whole genome shuffling can be performed to shuffle genes that encode antigenic polypeptides (along with other genomic nucleic acids). For whole genome shuffling approaches, it is not even necessary to identify which genes are being shuffled. Instead, *e.g.*, bacterial cell or viral genomes are combined and shuffled to acquire recombinant polypeptides that have enhanced ability to induce an immune response, as measured in any of the assays described below. Fourth, antigenic polypeptide-encoding genes can be codon modified to access mutational diversity not present in any naturally occurring gene. Details on each of these procedures can be found below.

Exemplary formats and examples for sequence recombination, sometimes referred to as DNA shuffling, evolution, or molecular breeding, have been described by the present inventors and co-workers in co-pending applications U.S. Patent Application Serial No. 08/198,431, filed February 17, 1994, Serial No. PCT/US95/02126, filed, February 17, 1995, Serial No. 08/425,684, filed April 18, 1995, Serial No. 08/537,874, filed October 30, 1995, Serial No. 08/564,955, filed November 30,1995, Serial No. 08/621,859, filed March 25, 1996, Serial No. 08/621,430, filed March 25, 1996, Serial No. PCT/US96/05480, filed April 18, 1996, Serial No. 08/650,400, filed May 20, 1996, Serial No. 08/675,502, filed July 3, 1996, Serial No. 08/721, 824, filed September 27, 1996, Serial No. PCT/US97/17300, filed September 26, 1997, and Serial No. PCT/US97/24239, filed December 17, 1997; Stemmer, *Science* 270:1510 (1995); Stemmer *et al., Gene* 164:49-53 (1995); Stemmer, *BiolTechnology* 13:549-553 (1995); Stemmer, *Proc. Natl. Acad. Sci. U.S.A.* 91:10747-10751 (1994); Stemmer, *Nature* 370:389-391 (1994); Crameri *et al., Nature Medicine* 2(1):1-3 (1996); Crameri *et al., Nature Biotechnology* 14:315-319 (1996), each of which is incorporated by reference in its entirety for all purposes.

Other methods for obtaining recombinant polynucleotides and/or for obtaining diversity in nucleic acids used as the substrates for shuffling include, for example, homologous recombination (PCT/US98/05223; Publ. No. WO98/42727); oligonucleotide-directed mutagenesis (for review *see,* Smith, *Ann. Rev. Genet.* 19: 423-462 (1985); Botstein and Shortle, *Science* 229: 1193-1201 (1985); Carter, *Biochem. J.* 237:1-7 (1986); Kunkel, "The efficiency of oligonucleotide directed mutagenesis" *in Nucleic acids & Molecular Biology,* Eckstein and Lilley, eds., Springer Verlag, Berlin (1987)). Included among these methods are oligonucleotide-directed mutagenesis (Zoller and Smith, *Nucl. Acids Res.* 10: 6487-6500 (1982), *Methods in Enzymol.* 100: 468-500 (1983), and *Methods in Enzymol.* 154: 329-350 (1987)) phosphothioate-modified DNA mutagenesis (Taylor *et al., Nucl. Acids Res.* 13: 8749-8764 (1985); Taylor *et al., Nucl. Acids Res.* 13: 8765-8787 (1985); Nakamaye and Eckstein, *Nucl. Acids Res.* 14: 9679-9698 (1986); Sayers *et al., Nucl. Acids Res. 16:* 791-802 (1988); Sayers *et al., Nucl. Acids Res.* 16: 803-814 (1988)), mutagenesis using uracil-containing templates (Kunkel, *Proc. Nat'l. Acad. Sci. USA* 82: 488-492 (1985) and Kunkel *et al., Methods in Enzymol.* 154: 367-382)); mutagenesis using gapped duplex DNA (Kramer *et al., Nucl. Acids Res.* 12: 9441-9456 (1984); Kramer and Fritz, *Methods in Enzymol.* 154: 350-367 (1987); Kramer *et al., Nucl. Acids Res.* 16: 7207 (1988)); and Fritz *et al., Nucl. Acids Res.* 16: 6987-6999 (1988)). Additional suitable methods include point mismatch repair (Kramer *et al., Cell* 38: 879-887 (1984)), mutagenesis using repair-deficient host strains (Carter *et al., Nucl. Acids Res.* 13: 4431-4443 (1985); Carter, *Methods in Enzymol.* 154: 382-403 (1987)), deletion mutagenesis (Eghtedarzadeh and Henikoff, *Nucl. Acids Res.* 14: 5115 (1986)), restriction-selection and restriction-purification (Wells *et al., Phil. Trans. R. Soc. Lond.* A 317: 415-423 (1986)), mutagenesis by total gene synthesis (Nambiar *et al., Science* 223: 1299-1301 (1984); Sakamar and Khorana, *Nucl. Acids Res. 14:* 6361-6372 (1988); Wells *et al., Gene* 34: 315-323 (1985); and Grundströrn *et al., Nucl. Acids Res.* 13: 3305-3316 (1985). Kits for mutagenesis are commercially available (*e.g.*, Bio-Rad, Amersham International, Anglian Biotechnology).

The breeding procedure starts with at least two substrates that generally show some degree of sequence identity to each other (*i.e.*, at least about 30%, 50%, 70%, 80% or 90% sequence identity), but differ from each other at certain positions. The difference can be any type of mutation, for example, substitutions, insertions and deletions. Often, different segments differ from each other in about 5-20 positions. For recombination to generate increased diversity relative to the starting materials, the starting materials must differ from each other in at least two nucleotide positions. That is, if there are only two substrates, there should be at least two divergent positions. If there are three substrates, for example, one substrate can differ from the second at a single position, and the second can differ from the third at a different single position. The starting DNA segments can be natural variants of each other, for example, allelic or species variants. The segments can also be from nonallelic genes showing some degree of structural and usually functional relatedness (*e.g.*, different genes within a superfamily, such as the family of *Yersinia* V-antigens, for example). The starting DNA segments can also be induced variants of each other. For example, one DNA segment can be produced by error-prone PCR replication of the other, the nucleic acid can be treated with a chemical or other mutagen, or by substitution of a mutagenic cassette. Induced mutants can also be prepared by propagating one (or both) of the segments in a mutagenic strain, or by inducing an error-prone repair system in the cells. In these situations, strictly speaking, the second DNA segment is not a single segment but a large family of related segments. The different segments forming the starting materials are often the same length or substantially the same length. However, this need not be the case; for example; one segment can be a subsequence of another. The segments can be present as part of larger molecules, such as vectors, or can be in isolated form.

The starting DNA segments are recombined by any of the sequence recombination formats provided herein to generate a diverse library of recombinant DNA segments. Such a library can vary widely in size from having fewer than 10 to more than 10⁵, 10⁹, 10¹² or more members. In some embodiments, the starting segments and the recombinant libraries generated will include full-length coding sequences and any essential regulatory sequences, such as a promoter and polyadenylation sequence, required for expression. In other embodiments, the recombinant DNA segments in the library can be inserted into a common vector providing sequences necessary for expression before performing screening/selection.

### Substrates for Evolution of Optimized Recombinant Antigens

The invention provides methods of obtaining recombinant polynucleotides that encode antigens that exhibit improved ability to induce an immune response to a pathogenic agent. The methods are applicable to a wide range of pathogenic agents, including potential biological warfare agents and other organisms and polypeptides that can cause disease and toxicity in humans and other animals. The following examples are merely illustrative, and not limiting.

### 1. Bacterial Pathogens and Toxins

In some embodiments, the methods of the invention are applied to bacterial pathogens, as well as to toxins produced by bacteria and other organisms. One can use the methods to obtain recombinant polypeptides that can induce an immune response against the pathogen, as well as recombinant toxins that are less toxic than native toxin polypeptides. Often, the polynucleotides of interest encode polypeptides that are present on the surface of the pathogenic organism.

Among the pathogens for which the methods of the invention are useful for producing protective immunogenic recombinant polypeptides are the *Yersinia* species. *Yersinia pestis,* the causative agent of plague, is one of the most virulent bacteria known with LD₅₀ values in mouse of less than 10 bacteria. The pneumonic form of the disease is readily spread between humans by aerosol or infectious droplets and can be lethal within days. A particularly preferred target for obtaining a recombinant polypeptide that can protect against *Yersinia* infection is the V antigen, which is a 37 kDa virulence factor, induces protective immune responses and is currently being evaluated as a subunit vaccine (Brubaker (1991) *Current Investigations of the Microbiology of Yersinae,* 12: 127). The V-antigen alone is not toxic, but *Y. pestis* isolates that lack the V-antigen are avirulent. The *Yersinia* V-antigen has been successfully produced in *E. coli* by several groups *(Leary et al.* (1995) *Infect. Immun.* 3: 2854). Antibodies that recognize the V-antigen can provide passive protection against homologous strains, but not against heterologous strains. Similarly, immunization with purified V antigen protects against only homologous strains. To obtain cross-protective recombinant V antigen, in a preferred embodiment, V antigen genes from various *Yersinia* species are subjected to family shuffling. The genes encoding the V antigen from *Y. pestis, Y. enterocolitica,* and *Y. pseudotuberculosis,* for example, are 92-99% identical at the DNA level, making them ideal for optimization using family shuffling according to the methods of the invention. After shuffling, the library of recombinant nucleic acids is screened and/or selected for those that encode recombinant V antigen polypeptides that can induce an improved immune response and/or have greater cross-protectivity.

*Bacillus anthracis,* the causative agent of anthrax, is another example of a bacterial target against which the methods of the invention are useful. The anthrax protective antigen (PA) provides protective immune responses in test animals, and antibodies against PA also provide some protection. However, the immunogenicity of PA is relatively poor, so multiple injections are typically required when wild-type PA is used. Co-vaccination with lethal factor (LF) can improve the efficacy of wild-type PA vaccines, but toxicity is a limiting factor. Accordingly the DNA shuffling and antigen library immunization methods of the invention can be used to obtain nontoxic LF. Polynucleotides that encode LF from various *B. anthracis* strains are subjected to family shuffling. The resulting library of recombinant LF nucleic acids can then be screened to identify those that encode recombinant LF polypeptides that exhibit reduced toxicity. For example, one can inoculate tissue culture cells with the recombinant LF polypeptides in the presence of PA and select those clones for which the cells survive. If desired, one can then backcross the nontoxic LF polypeptides to retain the immunogenic epitopes of LF. Those that are selected through the first screen can then be subjected to a secondary screen. For example, one can test for the ability of the recombinant nontoxic LF polypeptides to induce an immune response (*e.g.*, CTL or antibody response) in a test animal such as mice. In preferred embodiments, the recombinant nontoxic LF polypeptides are then tested for ability to induce protective immunity in test animals against challenge by different strains of *B. anthracis.*

The protective antigen (PA) of *B. anthracis* is also a suitable target for the methods of the invention. PA-encoding nucleic acids from various strains of *B. anthracis* are subjected to DNA shuffling. One can then screen for proper folding in, for example, *E. coli,* using polyclonal antibodies. Screening for ability to induce broad-spectrum antibodies in a test animal is also typically used, either alone or in addition to a preliminary screening method. In presently preferred embodiments, those recombinant polynucleotides that exhibit the desired properties can be backcrossed so that the immunogenic epitopes are maintained. Finally, the selected recombinants are tested for ability to induce protective immunity against different strains of *B. anthracis* in a test animal.

The *Staphylococcus aureus* and *Streptococcus* toxins are another example of a target polypeptide that can be altered using the methods of the invention. Strains of *Staphylococcus aureus* and group A Streptococci are involved in a range of diseases, including food poisoning, toxic shock syndrome, scarlet fever and various autoimmune disorders. They secrete a variety of toxins, which include at least five cytolytic toxins, a coagulase, and a variety of enterotoxins. The enterotoxins are classified as superantigens in that they crosslink MHC class II molecules with T cell receptors to cause a constitutive T cell activation (Fields *et al.* (1996) *Nature* 384: 188). This results in the accumulation of pathogenic levels of cytokines that can lead to multiple organ failure and death. At least thirty related, yet distinct enterotoxins have been sequenced and can be phylogenetically grouped into families. Crystal structures have been obtained for several members alone and in complex with MHC class II molecules. Certain mutations in the MHC class II-binding site of the toxins strongly reduce their toxicity and can form the basis of attenuated vaccines (Woody *et al.* (1997) *Vaccine* 15: 133). However, a successful immune response to one type of toxin may provide protection against closely related family members, whereas little protection against toxins from the other families is observed. Family shuffling of enterotoxin genes from various family members can be used to obtain recombinant toxin molecules that have reduced toxicity and can induce a cross-protective immune response. Shuffled antigens can also be screened to identify antigens that elicit neutralizing antibodies in an appropriate animal model such as mouse or monkey. Examples of such assays can include ELISA formats in which the elicited antibodies prevent binding of the enterotoxin to the MHC complex and/or T cell receptors on cells or purified forms. These assays can also include formats where the added antibodies would prevent T cells from being cross-linked to appropriate antigen presenting cells.

Cholera is an ancient, potentially lethal disease caused by the bacterium *Vibrio cholerae* and an effective vaccine for its prevention is still unavailable. Much of the pathogenesis of this disease is caused by the cholera enterotoxin. Ingestion of microgram quantities of cholera toxin can induce severe diarrhea causing loss of tens of liters of fluid. Cholera toxin is a complex of a single catalytic A subunit with a pentameric ring of identical B subunits. Each subunit is inactive on its own. The B subunits bind to specific ganglioside receptors on the surface of intestinal epithelial cells and trigger the entry of the A subunit into the cell. The A subunit ADP-ribosylates a regulatory G protein initiating a cascade of events causing a massive, sustained flow of electrolytes and water into the intestinal lumen resulting in extreme diarrhea.

The B subunit of cholera toxin is an attractive vaccine target for a number of reasons. It is a major target of protective antibodies generated during cholera infection and contains the epitopes for antitoxin neutralizing antibodies. It is nontoxic without the A subunit, is orally effective, and stimulates production of a strong IgA-dominated gut mucosal immune response, which is essential in protection against cholera and cholera toxin. The B subunit is also being investigated for use as an adjuvant in other vaccine preparations, and therefore, evolved toxins may provide general improvements for a variety of different vaccines. The heat-labile enterotoxins (LT) from enterotoxigenic *E. coli* strains are structurally related to cholera toxin and are 75% identical at the DNA sequence level. To obtain optimized recombinant toxin molecules that exhibit reduced toxicity and increased ability to induce an immune response that is protective against *V. cholerae* and *E. coli,* the genes that encode the related toxins are subjected to DNA shuffling.

The recombinant toxins are then tested for one or more of a several desirable traits. For example, one can screen for improved cross-reactivity of antibodies raised against the recombinant toxin polypeptides, for lack of toxicity in a cell culture assay, and for ability to induce a protective immune response against the pathogens and/or against the toxins themselves. The shuffled clones can be selected by phage display and/or screened by phage ELISA and ELISA assays for the presence of epitopes from the different serotypes. Variant proteins with multiple epitopes can then be purified and used to immunize mice or other test animal. The animal serum is then assayed for antibodies to the different B chain subtypes and variants that elicit a broad cross-reactive response will be evaluated further in a virulent challenge model. The *E. coli* and *V. cholerae* toxins can also act as adjuvants that are capable of enhancing mucosal immunity and oral delivery of vaccines and proteins. Accordingly, one can test the library of recombinant toxins for enhancement of the adjuvant activity.

Shuffled antigens can also be screened for improved expression levels and stability of the B chain pentamer, which may be less stable than when in the presence of the A chain in the hexameric complex. Addition of a heat treatment step or denaturing agents such as salts, urea, and/or guanidine hydrochloride can be included prior to ELISA assays to measure yields of correctly folded molecules by appropriate antibodies. It is sometimes desirable to screen for stable monomeric B chain molecules, in an ELISA format, for example, using antibodies that bind monomeric, but not pentameric B chains. Additionally, the ability of shuffled antigens to elicit neutralizing antibodies in an appropriate animal model such as mouse or monkey can be screened. For example, antibodies that bind to the B chain and prevent its binding to its specific ganglioside receptors on the surface of intestinal epithelial cells may prevent disease. Similarly antibodies that bind to the B chain and prevent its pentamerization or block A chain binding may be useful in preventing disease.

The bacterial antigens that can be improved by DNA shuffling for use as vaccines also include, but are not limited to, *Helicobacter pylori* antigens CagA and VacA (Blaser (1996) *Aliment. Pharmacol.* Ther. 1: 73-7; Blaser and Crabtree (1996) *Am. J. Clin. Pathol.* 106: 565-7; Censini *et al.* (1996) *Proc. Nat'l. Acad. Sci. USA* 93: 14648-14643). Other suitable *H. pylori* antigens include, for example, four immunoreactive proteins of 45-65 kDa as reported by *Chatha et al.* (1997) *Indian J. Med. Res.* 105: 170-175 and the H. *pylori* GroES homologue (HspA) *(Kansau et al.* (1996) *Mol. Microbiol.* 22:1013-1023. Other suitable bacterial antigens include, but are not limited to, the 43-kDa and the fimbrilin (41 kDa) proteins of *P*. *gingivalis* (Boutsl *et al.* (1996) *Oral Microbiol, Immunol.* 11: 236-241); pneumococcal surface protein A (Briles *et al.* (1996) *Ann. NY Acad. Sci.* 797: 118-126); *Chlamydia psittaci* antigens, 80-90 kDa protein and 110 kDa protein (Buendia *et al.* (1997) *FEMS Microbiol, Lett.* 150: 113-9); the chlamydial exoglycolipid antigen (GLXA) (Whittum-Hudson *et al.* (1996) *Nature Med.* 2: 1116-1121 ); *Chlamydia pneumoniae* species-specific antigens in the molecular weight ranges 92-98, 51-55,43-46 and 31.5-33 kDa and genus-specific antigens in the ranges 12, 26 and 65-70 kDa (Halme *et al.* (1997) *Scand. J. Immunol.* 45: 378-84); *Neisseria gonorrhoeae* (GC) or *Escherichia coli* phase-variable opacity (Opa) proteins (Chen and Gotschlich (1996) *Proc. Nat'l. Acad. Sci. USA* 93: 14851-14856), any of the twelve immunodominant proteins of *Schistosoma mansoni* (ranging in molecular weight from 14 to 208 kDa) as described by Cutts and Wilson (1997) *Parasitology* 114: 245-55; the 17-kDa protein antigen *of Brucella abortus* (De Mot *et al.* (1996) *Curr. Microbiol.* 33: 26-30); a gene homolog of the 17-kDa protein antigen of the Gram-negative pathogen *Brucella abortus* identified in the nocardioform actinomycete *Rhodococcus* sp. NI86/21 (De Mot *et al. (1996) Curr. Microbiol.* 33: 26-30); the staphylococcal enterotoxins (SEs) (Wood *et al.* (1997) *FEMS Immunol. Med. Microbiol. 17:* 1-10), a 42-kDa *M. hyopneumoniae* NrdF ribonucleotide reductase R2 protein or 15-kDa subunit protein of *M. hyopneumoniae* (Fagan *et al.* (1997) *Infect. Immun.* 65: 2502-2507), the meningococcal antigen PorA protein (Feavers *et al.* (1997) *Clin. Diagn. Lab. Immunol.* 3: 444-50); pneumococcal surface protein A (PspA) (McDaniel *et al.* (1997) *Gene Ther.* 4: 375-377); *F. tularensis* outer membrane protein FopA (Fulop *et al.* (1996) *FEMS Immunol. Med. Microbiol.* 13: 245-247); the major outer membrane protein within strains of the genus Actinobacillus (Hartmann *et al.* (1996) *Zentralbl. Bakteriol.* 284: 255-262); p60 or listeriolysin (Hly) antigen of *Listeria monocytogenes* (Hess *et al.* (1996) *Proc. Nat'l. Acad. Sci. USA* 93: 1458-1463); flagellar (G) antigens observed on *Salmonella enteritidis* and *S. pullorum* (Holt and Chaubal (1997) *J. Clin. Microbiol.* 35:1016-1020); *Bacillus anthracis* protective antigen (PA) (Ivins *et al.* (1995) *Vaccine* 13: 1779-1784); *Echinococcus granulosus* antigen 5 (Jones *et al.* (1996) *Parasitology* 113: 213-222); the *rol* genes of *Shigella dysenteriae* 1 and *Escherichia coli* K-12 (Klee *et al.* (1997) *J. Bacteriol.* 179: 2421-2425); cell surface proteins Rib and alpha of group B streptococcus (Larsson *et al.* (1996) *Infect. Immun.* 64: 3518-3523); the 37 kDa secreted polypeptide encoded on the 70 kb virulence plasmid of pathogenic *Yersinia* spp. (Leary *et al.* (1995) *Contrib. Microbiol. Immunol.* 13: 216-217 and Roggenkamp *et al.* (1997) *Infect. Immun.* 65:446-51); the OspA (outer surface protein A) of the Lyme disease spirochete *Borrelia burgdorferi* (Li *et al.* (1997) *Proc. Nat'l. Acad. Sci. USA* 94: 3584-3589, Padilla *et al.* (1996) *J. Infect. Dis.* 174: 739-746, and Wallich *et al.* (1996) *Infection* 24: 396-397); the *Brucella melitensis* group 3 antigen gene encoding Omp28 (Lindler *et al.* (1996) *Infect. Immun.* 64: 2490-2499); the PAc antigen of *Streptococcus mutans* (Murakami *et al.* (1997) *Infect. Immun.* 65: 794-797); pneumolysin, Pneumococcal neuraminidases, autolysin, hyaluronidase, and the 37 kDa pneumococcal surface adhesin A (Paton *et al.* (1997) *Microb. Drug Resist. 3:* 1-10); 29-32, 41-45, 63-71 x 10(3) MW antigens of *Salmonella typhi* (Perez *et al.* (1996) *Immunology* 89: 262-267); K-antigen as a marker of *Klebsiella pneumoniae* (Priamukhina and Morozova (1996) *Klin. Lab. Diagn.* 47-9); nocardial antigens of molecular mass approximately 60, 40, 20 and 15-10 kDa (Prokesova *et al.* (1996) *Int. J. Immunopharmacol.* 18: 661-668); *Staphylococcus aureus* antigen ORF-2 (Rieneck *et al.* (1997) *Biochim Biophys Acta* 1350: 128-132); GlpQ antigen of Borrelia hermsii (Schwan *et al.* (1996) *J. Clin. Microbiol.* 34: 2483-2492); cholera protective antigen (CPA) (Sciortino (I996) *J. Diarrhoeal Dis. Res. 14:* 16-26); a 190-kDa protein antigen of Streptococcus mutans (Senpuku *et al.* (1996) Oral *Microbiol. Immunol.* 11: 121-128); Anthrax toxin protective antigen (PA) (Sharma *et al.* (1996) *Protein Expr. Purif.* 7: 33-38); *Clostridium perfringens* antigens and toxoid (Strom *et al.* (1995) *Br. J. Rheumatol.* 34: 1095-1096); the SEF14 fimbrial antigen of *Salmonella enteritidis* (Thorns *et al.* (1996) *Microb. Pathog.* 20: 235-246); the *Yersinia pestis* capsular antigen (F1 antigen) (Titball *et al.* (1997) *Infect. Immun.* 65: 1926-1930); a 35-kilodalton protein of *Mycobacterium leprae* (Triccas *et al.* (1996) *Infect. Immun.* 64: 5171-5177); the major outer membrane protein, CD, extracted from *Moraxella* (Branhamella) catarrhalis (Yang *et al.* (1997) *FEMS Immunol. Med. Microbiol.* 17: 187-199); pH6 antigen (PsaA protein) of *Yersinia pestis* (Zav'yalov *et al.* (1996) *FEMS Immunol. Med. Microbiol.* 14: 53-57); a major surface glycoprotein, gp63, of *Leishmania major* (Xu and Liew (1994) *Vaccine* 12: 1534-1536; Xu and Liew (1995) *Immunology* 84: 173-176); mycobacterial heat shock protein 65, mycobacterial antigen *(Mycobacterium leprae* hsp65) (Lowrie *et al.* (1994) *Vaccine* 12: 1537-1540; Ragno *et al.* (1997) *Arthritis Rheum.* 40: 277-283; Silva (1995) *Braz. J. Med. Biol. Res.* 28: 843-851); *Mycobacterium tuberculosis* antigen 85 (Ag85) (Huygen *et al.* (1996) *Nat. Med.* 2: 893-898); the 45/47 kDa antigen complex (APA) of *Mycobacterium tuberculosis, M. bovis* and BCG (Horn *et al.* (1996) *J. Immunol. Methods* 197: 151-159); the mycobacterial antigen, 65-kDa heat shock protein, hsp65 (Tascon *et al.* (1996) *Nat. Med.* 2: 888-892); the mycobacterial antigens MPB64, MPB70, MPB57 and alpha antigen (Yamada *et al.* (1995) *Kekkaku* 70: 639-644); the *M. tuberculosis* 38 kDa protein (Vordermeier *et al.* (1995) *Vaccine* 13: 1576-1582); the MPT63, MPT64 and MPT-59 antigens from *Mycobacterium tuberculosis* (Manca *et al.* (1997) *Infect. Immun.* 65: 16-23; Oettinger *et al.* (1997) *Scand. J. Immunol.* 45: 499-503; Wilcke *et al.* (1996) *Tuber. Lung Dis.* 77: 250-256); the 35-kilodalton protein of *Mycobacterium leprae* (Triccas *et al.* (1996) *Infect. Immun.* 64: 5171-5177); the ESAT-6 antigen of virulent mycobacteria *(Brandt et al.* (1996) *J. Imrnunol.* 157: 3527-3533; Pollock and Andersen (1997) *J. Infect. Dis. 175:* 1251-1254); *Mycobacterium tuberculosis* 16-kDa antigen (Hsp16.3) (Chang *et al.* (1996) *J. Biol. Chem.* 271: 7218-7223); and the 18-kilodalton protein of *Mycobacterium leprae* (Baumgart *et al.* (1996) *Infect. Immun.* 64: 2274-2281).

### 2. Viral Pathogens

The methods of the invention are also useful for obtaining recombinant nucleic acids and polypeptides that have enhanced ability to induce an immune response against viral pathogens. While the bacterial recombinants described above are typically administered in polypeptide form, recombinants that confer viral protection are preferably administered in nucleic acid form, as genetic vaccines.

One illustrative example is the Hantaan virus. Glycoproteins of this virus typically accumulate at the membranes of the Golgi apparatus of infected cells. This poor expression of the glycoprotein prevents the development of efficient genetic vaccines against these viruses. The methods of the invention solve this problem by performing DNA shuffling on nucleic acids that encode the glycoproteins and identifying those recombinants that exhibit enhanced expression in a host cell, and/or for improved immunogenicity when administered as a genetic vaccine. A convenient screening method for these methods is to express the recombinant polynucleotides as fusion proteins to PIG, which results in display of the polypeptides on the surface of the host cell (Whitehorn *et al.* (1995) *Biotechnology* (N Y) 13:1215-9). Fluorescence-activated cell sorting is then used to sort and recover those cells that express an increased amount of the antigenic polypeptide on the cell surface. This preliminary screen can be followed by immunogenicity tests in mammals, such as mice. Finally, in preferred embodiments, those recombinant nucleic acids are tested as genetic vaccines for their ability to protect a test animal against challenge by the virus.

The flaviviruses are another example of a viral pathogen for which the methods of the invention are useful for obtaining a recombinant polypeptide or genetic vaccine that is effective against a viral pathogen. The flaviviruses consist of three clusters of antigenically related viruses: Dengue 1-4 (62-77% identity), Japanese, St. Louis and Murray Valley encephalitis viruses (75-82% identity), and the tick-borne encephalitis viruses (77-96% identity). Dengue virus can induce protective antibodies against SLE and Yellow fever (40-50% identity), but few efficient vaccines are available. To obtain genetic vaccines and recombinant polypeptides that exhibit enhanced cross-reactivity and immunogenicity, the polynucleotides that encode envelope proteins of related viruses are subjected to DNA shuffling. The resulting recombinant polynucleotides can be tested, either as genetic vaccines or by using the expressed polypeptides, for ability to induce a broadly reacting neutralizing antibody response. Finally, those clones that are favorable in the preliminary screens can be tested for ability to protect a test animal against viral challenge.

Viral antigens that can be evolved by DNA shuffling for improved activity as vaccines include, but are not limited to, influenza A virus N2 neuraminidase (Kilbourne *et al.* (1995) Vaccine 13: 1799-1803); Dengue virus envelope (E) and premembrane (prM) antigens (Feighny *et al.* (1994) *Am. J. Trop. Med. Hyg.* 50: 322-328; Putnak *et al.* (1996) *Am. J. Trop. Med. Hyg.* 55: 504-10); HIV antigens Gag, Pol, Vif and Nef (Vogt *et al.* (1995) *Vaccine* 13: 202-208); HIV antigens gp120 and gp160 (Achour *et al.* (1995) *Cell. Mol. Biol.* 41: 395-400; Hone *et al.* (1994) *Dev. Biol. Stand.* 82: 159-162); gp41 epitope of human immunodeficiency virus (Eckhart *et al.* (1996) *J. Gen. Virol.* 77: 2001-2008); rotavirus antigen VP4 (Mattion *et al.* (1995) *J. Virol.* 69: 5132-5137); the rotavirus protein VP7 or VP7sc (Emslie *et al.* (1995) J. *Virol.* 69: 1747-1754; Xu *et al.* (1995) *J. Gen. Virol. 76:* 1971-1980); herpes simplex virus (HSV) glycoproteins gB, gC, gD, gE, gG, gH, and gI (Fleck *et al.* (1994) *Med. Microbiol. Immunol.* (Berl) 183: 87-94 [Mattion, 1995]; Ghiasi *et al.* (1995) *Invest. Ophthalmol. Vis. Sci.* 36: 1352-1360; McLean *et al.* (1994) *J. Infect. Dis.* 170: 1100-1109); immediate-early protein ICP47 of herpes simplex virus-type 1 (HSV-1) (Banks *et al.* (1994) *Virology* 200: 236-245); immediate-early (IE) proteins ICP27, ICP0, and ICP4 of herpes simplex virus (Manickan *et al.* (1995) *J*. *Virol.* 69: 4711-4716); influenza virus nucleoprotein and hemagglutinin (Deck *et al.* (1997) *Vaccine* 15: 71-78; Fu *et al.* (1997) *J. Virol.* 71: 2715-2721 ); B 19 parvovirus capsid proteins VP1 (Kawase *et al.* (1995) *Virology* 211: 359-366) or VP2 (Brown *et al.* (1994) *Virology* 198: 477-488); Hepatitis B virus core and e antigen (Schodel *et al.* (1996) *Intervirology* 39: 104-106); hepatitis B surface antigen (Shiau and Murray (1997) *J. Med. Virol.* 51: 159-166); hepatitis B surface antigen fused to the core antigen of the virus (Id.); Hepatitis B virus core-preS2 particles (Nemeckova *et al.* (1996) *Acta Virol.* 40: 273-279); HBV preS2-S protein (Kutinova *et al.* (1996) *Vaccine* 14: 1045-1052); VZV glycoprotein I (Kutinova *et al.* (1996) *Vaccine* 14: 1045-1052); rabies virus glycoproteins (Xiang *et al.* (1994) *Virology* 199: 132-140; Xuan *et al.* (1995) *Virus Res.* 36: 151-161) or ribonucleocapsid (Hooper *et al.* (1994) *Proc. Nat'l*. *Acad. Sci. USA* 91:10908-10912); human cytomegalovirus (HCMV) glycoprotein B (UL55) (Britt *et al.* (1995) *J. Infect. Dis.* 171: 18-25); the hepatitis C virus (HCV) nucleocapsid protein in a secreted or a nonsecreted form, or as a fusion protein with the middle (pre-S2 and S) or major (S) surface antigens of hepatitis B virus (HBV) (Inchauspe *et al.* (1997) *DNA Cell Biol.* 16: 185-195; Major *et al.* (1995) *J. Virol.* 69: 5798-5805); the hepatitis C virus antigens: the core protein (pC); E1 (pE1) and E2 (pE2) alone or as fusion proteins (Saito *et al.* (1997) *Gastroenterology* 112: 1321-1330); the gene encoding respiratory syncytial virus fusion protein (PFP-2) (Falsey and Walsh (1996) *Vaccine* 14:1214-1218; Piedra *et al.* (1996) *Pediatr. Infect. Dis. J.* 15: 23-31); the VP6 and VP7 genes of rotaviruses (Choi *et al.* (1997) *Virology* 232: 129-138; Jin *et al.* (1996) *Arch. Virol.* 141: 2057-2076); the E1, E2, E3, E4, E5, E6 and E7 proteins of human papillomavirus (Brown *et al.* (1994) *Virology* 201: 46-54; Dillner *et al.* (1995) *Cancer Detect. Prev.* 19: 381-393; Krul *et al.* (1996) *Cancer Immunol. Immunother.* 43: 44-48; Nakagawa *et al.* (1997) *J. Infect. Dis.* 175: 927-931); a human T-lymphotropic virus type I gag protein (Porter *et al.* (1995) *J. Med. Virol.* 45: 469-474); Epstein-Barr virus (EBV) gp340 (Mackett *et al.* (1996) *J. Med. Virol.* 50: 263-271); the Epstein-Barr virus (EBV) latent membrane protein LMP2 (Lee *et al.* (1996) *Eur. J. Immunol.* 26: 1875-1883); Epstein-Barr virus nuclear antigens 1 and 2 (Chen and Cooper (1996) *J. Virol.* 70: 4849-4853; Khanna *et al.* (1995) *Virology* 214: 633-637); the measles virus nucleoprotein (N) (Fooks *et al.* (1995) *Virology* 210: 456-465); and cytomegalovirus glycoprotein gB (Marshall *et al.* (1994) *J. Med. Virol.* 43: 77-83) or glycoprotein gH (Rasmussen *et al.* (1994) *J. Infect. Dis.* 170: 673-677).

### 3. Parasites

Antigens from parasites can also be optimized by the methods of the invention. These include, but are not limited to, the schistosome gut-associated antigens CAA (circulating anodic antigen) and CCA (circulating cathodic antigen) in *Schistosoma mansoni, S. haematobium* or *S. japonicum* (Deelder *et al.* (1996) *Parasitology* 112: 21-3 5); a multiple antigen peptide (MAP) composed of two distinct protective antigens derived from the parasite *Schistosoma mansoni* (Ferru *et al.* (1997) *Parasite Immunol.* 19: 1-11); *Leishmania* parasite surface molecules (Lezama-Davila (1997) *Arch. Med. Res.* 28: 47-53); third-stage larval (L3) antigens of L. loa (Akue *et al.* (1997) *.I. Infecr. Dis.* 175: 158-63); the genes. Tarns 1-1 and Tarns 1-2, encoding the 30-and 32-kDa major merozoite surface antigens of Theileria annulata (Ta) (d'Oliveira *et al.* (1996) *Gene* 172: 33-39); *Plasmodium falciparum* merozoite surface antigen 1 or 2 (al-Yaman *et al.* (1995) *Trans. R. Soc. Trop. Med. Hyg.* 89: 555-559; Beck *et al.* (1997) *J. Infect. Dis.* 175: 921-926; Rzepczyk *et al.* (1997) *Infect. Immun.* 65: 1098-1100)-. circumsporozoite (CS) protein-based B-epitopes from *Plasmodium berghei,* (PPPPNPND)₂ (SEQ ID NO:23) and *Plasmodium yoelii,* (QGPGAP)₃QG (SEQ ID NO:24), along with a *P. berghei* T-helper epitope KQIRDSITEEWS (SEQ ID NO:25)(Reed *et al.* (1997) *Vaccine* 15: 482-488); NYVAC-Pf7 encoded *Plasmodium falciparum* antigens derived from the sporozoite (circumsporozoite protein and sporozoite surface protein 2), liver (liver stage antigen 1), blood (merozoite surface protein 1, serine repeat antigen, and apical membrane antigen 1), and sexual (25-kDa sexual-stage antigen) stages of the parasite life cycle were inserted into a single NYVAC genome to generate NYVAC-Pf7 (Tine *et al.* (1996) *Infect. Immun.* 64: 3833-3844); *Plasmodium falciparum* antigen Pfs230 (Williamson *et al.* (1996) *Mol. Biochem. Parasitol.* 78: 161-169); *Plasmodium falciparum* apical membrane antigen (AMA-1) (Lal *et al.* (1996) *Infect. Immun.* 64: 1054-1059); *Plasmodium falciparum* proteins Pfs28 and Pfs25 (Duffy and Kaslow (1997) *Infect. Immun.* 65: 1109-1113); *Plasmodium falciparum* merozoite surface protein, MSP1 (Hui *et al.* (1996) *Infect. Immun.* 64: 1502-1509); the malaria antigen Pf332 (Ahlborg *et al.* (1996) *Immunology* 88: 630-635); *Plasmodium falciparum* erythrocyte membrane protein 1 (Baruch *et al.* (1995) *Proc. Nat'l. Acad. Sci. USA* 93: 3497-3502; Baruch *et al.* (1995) *Cell* 82: 77-87); *Plasmodium falciparum* merozoite surface antigen, PfMSP-1 (Egan *et al.* (1996) *J. Infect. Dis.* 173: 765-769); *Plasmodium falciparum* antigens SERA, EBA-175, RAP1 and RAP2 (Riley (1997) *J. Pharm. Pharmacol.* 49: 21-27); *Schistosoma japonicum* paramyosin (Sj97) or fragments thereof (Yang *et al.* (1995) *Biochem. Biophys. Res. Commun.* 212: 1029-1039); and Hsp70 in parasites (Maresca and Kobayashi (1994) *Experientia* 50: 1067-1074).

### 4. Allergy

The invention also provides methods of obtaining reagents that are useful for treating allergy. In one embodiment, the methods involve making a library of recombinant polynucleotides that encode an allergen, and screening the library to identify those recombinant polynucleotides that exhibit improved properties when used as immunotherapeutic reagents for treating allergy. For example, specific immunotherapy of allergy using natural antigens carries a risk of inducing anaphylaxis, which can be initiated by cross-linking of high-affinity IgE receptors on mast cells. Therefore, allergens that are not recognized by pre-existing IgE are desirable. The methods of the invention provide methods by which one can obtain such allergen variants. Another improved property of interest is induction of broader immune responses, increased safety and efficacy.

Synthesis of polyclonal and allergen-specific IgE requires multiple interactions between B cells, T cells and professional antigen-presenting cells (APC). Activation of naive, unprimed B cells is initiated when specific B cells recognize the allergen by cell surface immunoglobulin (sIg). However, costimulatory molecules expressed by activated T cells in both soluble and membrane-bound forms are necessary for differentiation of B cells into IgE-secreting plasma cells. Activation ofT helper cells requires recognition of an antigenic peptide in the context of MHC class II molecules on the plasma membrane of APC, such as monocytes, dendritic cells, Langerhans cells or primed B cells. Professional APC can efficiently capture the antigen and the peptide-MHC class II complexes are formed in a post-Golgi, proteolytic intracellular compartment and subsequently exported to the plasma membrane, where they are recognized by T cell receptor (TCR) (Whitton (1998) *Curr. Top. Microbiol. Immunol.* 232: 1-13). In addition, activated B cells express CD80 (B7-1) and CD86 (B7-2, B70), which are the counter receptors for CD28 and which provide a costimulatory signal for T cell activation resulting in T cell proliferation and cytokine synthesis. Since allergen-specific T cells from atopic individuals generally belong to the T_{H}2 cell subset, activation of these cells also leads to production of IL-4 and IL-13, which, together with membrane-bound costimulatory molecules expressed by activated T helper cells, direct B cell differentiation into IgE-secreting plasma cells.

Mast cells and eosinophils are key cells in inducing allergic symptoms in target organs. Recognition of specific antigen by IgE bound to high-affinity IgE receptors on mast cells, basophils or eosinophils results in crosslinking of the receptors leading to degranulation of the cells and rapid release of mediator molecules, such as histamine, prostaglandins and leukotrienes, causing allergic symptoms.

Immunotherapy of allergic diseases currently includes hyposensibilization treatments using increasing doses of allergen injected to the patient. These treatments result skewing of immune responses towards T_{H}1 phenotype and increase the ratio of IgG/IgE antibodies specific for allergens. Because these patients have circulating IgE antibodies specific for the allergens, these treatments include significant risk of anaphylactic reactions. In these reactions, free circulating allergen is recognized by IgE molecules bound to high-affinity IgE receptors on mast cells and eosinophils. Recognition of the allergen results in crosslinking of the receptors leading to release of mediators, such as histamine, prostaglandins, and leukotrienes, which cause the allergic symptoms, and occasionally anaphylactic reactions. Other problems associated with hyposensibilization include low efficacy and difficulties in producing allergen extracts reproducibly.

The methods of the invention provide a means to obtain allergens that, when used in genetic vaccines, provide a means of circumventing the problems that have limited the usefulness of previously known hyposensibilization treatments. For example, by expressing antigens on the surface of cells, such as muscle cells, the risk of anaphylactic reactions is significantly reduced. This can be conveniently achieved by using genetic vaccine vectors that encode transmembrane forms of allergens. The allergens can also be modified in such a way that they are efficiently expressed in transmembrane forms, further reducing the risk of anaphylactic reactions. Another advantage provided by the use of genetic vaccines for hyposensibilization is that the genetic vaccines can include cytokines and accessory molecules which further direct the immune responses towards the T_{H}1 phenotype, thus reducing the amount of IgE antibodies produced and increasing the efficacy of the treatments. To further reduce IgE production, one can administer the shuffled allergens using vectors that have been evolved to induce primarily IgG and IgM responses, with little or no IgE response (*see, e.g.,* US Patent Application Ser. No. 09/021,769, filed February 11, 1998).

In these methods, polynucleotides encoding known allergens, or homologs or fragments thereof (*e.g.*, immunogenic peptides) are inserted into DNA vaccine vectors and used to immunize allergic and asthmatic individuals. Alternatively, the shuffled allergens are expressed in manufacturing cells, such as *E. coli* or yeast cells, and subsequently purified and used to treat the patients or prevent allergic disease. DNA shuffling or other recombination method can be used to obtain allergens that activate T cells but cannot induce anaphylactic reactions. For example, a library of recombinant polynucleotides that encode allergen variants can be expressed in cells, such as antigen presenting cells, which are than contacted with PBMC or T cell clones from atopic patients. Those library members that efficiently activate T_{H} cells from the atopic patients can be identified by assaying for T cell proliferation, or by cytokine synthesis (*e.g.*, synthesis of IL-2, IL-4, IFN-γ. Those recombinant allergen variants that are positive in the *in vitro* tests can then be subjected to *in vivo* testing.

Examples of allergies that can be treated include, but are not limited to, allergies against house dust mite, grass pollen, birch pollen, ragweed pollen, hazel pollen, cockroach, rice, olive tree pollen, fungi, mustard, bee venom. Antigens of interest include those of animals, including the mite (*e.g., Dermatophagoides pteronyssinus, Dermatophagoides farinae, Blomia tropicalis*), such as the allergens derp1 (Scobie *et al.* (1994) *Biochem. Soc. Trans.* 22: 448S; Yssel *et al.* (1992) *J. Immunol.* 148: 738-745), der p2 (Chua *et al.* (1996) *Clin. Exp. Allergy* 26: 829-837), der p3 (Smith and Thomas (1996) *Clin. Exp. Allergy* 26: 571-579), der p5, der p V (Lin *et al.* (1994) *J. Allergy Clin. Immunol. 94:* 989-996), der p6 (Bennett and Thomas (1996) *Clin. Exp. Allergy* 26: 1150-1154), der p 7 (Shen *et al.* (1995) *Clin. Exp. Allergy* 25: 416-422), der f2 (Yuuki *et al.* (1997) *Int. Arch. Allergy Immunol.* 112: 44-48), der f3 (Nishiyama *et al.* (1995) *FEBS Lett.* 377: 62-66), der f7 (Shen *et al.* (1995) *Clin. Exp. Allergy* 25: 1000-1006); Mag 3 (Fujikawa *et al.* (1996) *Mol. Immunol.* 33: 311-319). Also of interest as antigens are the house dust mite allergens Tyr p2 (Eriksson *et al.* (1998) *Eur. J. Biochem.* 251: 443-447), Lep d1 (Schmidt *et al.* (1995) *FEBS Lett.* 370:11-14), and glutathione S-transferase (O'Neill *et al.* (1995) *Immunol Lett.* 48: 103-107); the 25,589 Da, 219 amino acid polypeptide with homology with glutathione S-transferases (O'Neill *et al.* (1994) *Biochim. Biophys. Acta.* 1219: 521-528); Blo t 5 (Arruda *et al.* (1995) *Int. Arch. Allergy Immunol.* 107: 456-457); bee venom phospholipase A2 (Carballido *et al.* (1994) *J. Allergy Clin. Immunol.* 93: 758-767; Jutel *et al.* (1995) *J. Immunol.* 154: 4187-4194); bovine dermal/dander antigens BDA 11 (Rautiainen *et al.* (1995) *J. Invest. Dermatol.* 105: 660-663) and BDA20 (Mantyjarvi *et al.* (1996) *J. Allergy Clin. Immunol.* 97: 1297-1303); the major horse allergen Equ c1 (Gregoire *et al.* (1996) *J. Biol. Chem.* 271: 32951-32959); Jumper ant *M. pilosula* allergen Myr p I and its homologous allergenic polypeptides Myr p2 (Donovan *et al.* (1996) *Biochem. Mol. Biol. Int.* 39: 877-885); 1-13, 14, 16 kD allergens of the mite *Blomia tropicalis* (Caraballo *et al.* (1996) *J. Allergy Clin. Immunol.* 98: 573-579); the cockroach allergens Bla g Bd90K (Helm *et al.* (1996) *J. Allergy Clin. Immunol.* 98: 172-80) and Bla g 2 (Arruda *et al.* (1995) *J. Biol. Chem.* 270: 19563-19568); the cockroach Cr-PI allergens (Wu *et al.* (1996) *J. Biol. Chem.* 271: 17937-17943); fire ant venom allergen, Sol i 2 (Schmidt *et al.* (1996) *J. Allergy Clin. Immunol.* 98: 82-88); the insect *Chironomus thummi* major allergen Chi t 1-9 (Kipp *et al.* (1996) *Int. Arch. Allergy Immunol.* 110: 348-353); dog allergen Can f 1 or cat allergen Fel d 1 (Ingram *et al.* (1995) *J. Allergy Clin. Immunol.* 96: 449-456); albumin, derived, for example, from horse, dog or cat (Goubran Botros *et al.* (1996) *Immunology* 88: 340-347); deer allergens with the molecular mass of 22 kD, 25 kD or 60 kD (Spitzauer *et al.* (1997) *Clin. Exp. Allergy* 27: 196-200); and the 20 kd major allergen of cow (Ylonen *et al.* (1994) *J. Allergy Clin. Immunol.* 93: 851-858).

Pollen and grass allergens are also useful in vaccines, particularly after optimization of the antigen by the methods of the invention. Such allergens include, for example, Hor v9 (Astwood and Hill (1996) *Gene* 182: 53-62, Lig v1 (Batanero *et al.* (1996) *Clin. Exp. Allergy* 26: 1401-1410); Lol p 1 (Muller *et al.* (1996) *Int. Arch. Allergy Immunol.* 109: 352-355), Lol p II (Tamborini *et al.* (1995) *Mol. Immunol.* 32: 505-513), Lol pVA, Lol pVB (Ong *et al.* (1995) *Mol. Immunol.* 32: 295-302), Lol p 9 (Blaher *et al.* (1996) *J. Allergy Clin. Immunol.* 98: 124-132); Par J I (Costa *et al.* (1994) *FEBS Lett.* 341: 182-186; Sallusto *et al.* (1996) *J. Allergy Clin. Immunol.* 97: 627-637), Par j 2.0101 (Duro *et al.* (1996) *FEBS Lett.* 399: 295-298); Bet v1 (Faber *et al.* (1996) *J. Biol. Chem.* 271: 19243-19250), Bet v2 (Rihs *et al.* (1994) *Int. Arch. Allergy Immunol.* 105: 190-194); Dac g3 (Guerin-Marchand *et al.* (1996) *Mol. Immunol.* 33: 797-806); Phl p 1 (Petersen *et al.* (1995) *J. Allergy Clin. Immunol.* 95: 987-994), Phl p 5 (Muller *et al.* (1996) *Int. Arch. Allergy Immunol.* 109: 352-355), Phl p 6 (Petersen *et al.* (1995) *Int. Arch. Allergy Immunol.* 108: 55-59); Cry j I (Sone *et al.* (1994) *Biochem. Biophys. Res. Commun.* 199: 619-625), Cry j II (Namba *et al.* (1994) *FEBS Lett.* 353: 124-128); Cor a 1 (Schenk *et al.* (1994) *Eur. J. Biochem.* 224: 717-722); cyn d1 (Smith *et al.* (1996) *J. Allergy Clin. Immunol.* 98: 331-343), cyn d7 (Suphioglu *et al.* (1997) *FEBS Lett.* 402: 167-172); Pha a 1 and isoforms of Pha a 5 (Suphioglu and Singh (1995) *Clin. Exp. Allergy* 25: 853-865); Cha o 1 (Suzuki *et al.* (1996) *Mol. Immunol.* 33: 451-460); profilin derived, *e.g,* from timothy grass or birch pollen (Valenta *et al.* (1994) *Biochem. Biophys. Res. Commun.* 199: 106-118); P0149 (Wu *et al.* (1996) *Plant Mol. Biol.* 32:1037-1042); Ory s1 (Xu *et al.* (1995) *Gene* 164: 255-259); and Amb a V and Amb t 5 (Kim *et al.* (1996) *Mol. Immunol.* 33: 873-880; Zhu *et al.* (1995) *J. Immunol.* 155: 5064-5073).

Vaccines against food allergens can also be developed using the methods of the invention. Suitable antigens for shuffling include, for example, profilin (Rihs *et al.* (1994) *Int. Arch. Allergy Immunol.* 105: 190-194); rice allergenic cDNAs belonging to the alpha-amylase/trypsin inhibitor gene family (Alvarez *et al.* (1995) *Biochim Biophys Acta* 1251: 201-204); the main olive allergen, Ole e I (Lombardero *et al.* (1994) *Clin Exp Allergy* 24: 765-770); Sin a 1, the major allergen from mustard (Gonzalez De La Pena *et al.* (1996) *Eur J Biochem.* 237: 827-832); parvalbumin, the major allergen of salmon (Lindstrom *et al.* (1996) *Scand. J. Immunol.* 44: 335-344); apple allergens, such as the major allergen Mal d 1 (Vanek-Krebitz *et al.* (1995) *Biochem. Biophys. Res. Commun.* 214: 538-551); and peanut allergens, such as Ara h I *(Burks et al.* (1995) *J. Clin. Invest.* 96: 1715-1721).

The methods of the invention can also be used to develop recombinant antigens that are effective against allergies to fungi. Fungal allergens useful in these vaccines include, but are not limited to, the allergen, Cla h III, of *Cladosporium herbarum* (Zhang *et al.* (1995) *J. Immunol.* 154: 710-717); the allergen Psi c 2, a fungal cyclophilin, from the basidiomycete *Psilocybe cubensis* (Horner *et al.* (1995) *Int. Arch. Allergy Immunol.* 107: 298-300); hsp 70 cloned from a cDNA library of *Cladosporium herbarum* (Zhang *et al.* (1996) *Clin Exp Allergy* 26: 88-95); the 68 kD allergen *of Penicillium notatum* (Shen *et al.* (1995) *Clin. Exp. Allergy* 26: 350-356); aldehyde dehydrogenase (ALDH) (Achatz *et al.* (1995) *Mol Immunol.* 32: 213-227); enolase (Achatz *et al.* (1995) *Mol. Immunol.* 32: 213-227); YCP4 (Id.); acidic ribosomal protein P2 (Id.).

Other allergens that can be used in the methods of the invention include latex allergens, such as a major allergen (Hev b 5) from natural rubber latex (Akasawa *et al.* (1996) *J. Biol. Chem.* 271: 25389-25393; *Slater et al.* (1996) *J. Biol. Chem.* 271: 25394-25399).

The invention also provides a solution to another shortcoming of vaccination as a treatment for allergy and asthma. While genetic vaccination primarily induces CD8⁺ T cell responses, induction of allergen-specific IgE responses is dependent on CD4⁺ T cells and their help to B cells. T_{H}2-type cells are particularly efficient in inducing IgE synthesis because they secrete high levels of IL-4, IL-5 and IL-13, which direct Ig isotype switching to IgE synthesis. IL-5 also induces eosinophilia. The methods of the invention can be used to develop recombinant antigens that efficiently induce CD4⁺ T cell responses, and direct differentiation of these cells towards the T_{H}1 phenotype.

### 5. Inflammatory and Autoimmune Diseases

Autoimmune diseases are characterized by immune response that attacks tissues or cells of ones own body, or pathogen-specific immune responses that also are harmful for ones own tissues or cells, or non-specific immune activation which is harmful for ones own tissues or cells. Examples of autoimmune diseases include, but are not limited to, rheumatoid arthritis, SLE, diabetes mellitus, myasthenia gravis, reactive arthritis, ankylosing spondylitis, and multiple sclerosis. These and other inflammatory conditions, including IBD, psoriasis, pancreatitis, and various immunodeficiencies, can be treated using antigens that are optimized using the methods of the invention.

These conditions are often characterized by an accumulation of inflammatory cells, such as lymphocytes, macrophages, and neutrophils, at the sites of inflammation. Altered cytokine production levels are often observed, with increased levels of cytokine production. Several autoimmune diseases, including diabetes and rheumatoid arthritis, are linked to certain MHC haplotypes. Other autoimmune-type disorders, such as reactive arthritis, have been shown to be triggered by bacteria such as *Yersinia* and *Shigella,* and evidence suggests that several other autoimmune diseases, such as diabetes, multiple sclerosis, rheumatoid arthritis, may also be initiated by viral or bacterial infections in genetically susceptible individuals.

Current strategies of treatment generally include anti-inflammatory drugs, such as NSAID or cyclosporin, and antiproliferative drugs, such as methotrexate. These therapies are non-specific, so a need exists for therapies having greater specificity, and for means to direct the immune responses towards the direction that inhibits the autoimmune process.

The present invention provides several strategies by which these needs can be fulfilled. First, the invention provides methods of obtaining antigens having greater tolerogenicity and/or have improved antigenicity. In a preferred embodiment, the antigens prepared according to the invention exhibit improved induction of tolerance by oral delivery. Oral tolerance is characterized by induction of immunological tolerance after oral administration of large quantities of antigen. In animal models, this approach has proven to be a very promising approach to treat autoimmune diseases, and clinical trials are in progress to address the efficacy of this approach in the treatment of human autoimmune diseases, such as rheumatoid arthritis and multiple sclerosis. It has also been suggested that induction of oral tolerance against viruses used in gene therapy might reduce the immunogenicity of gene therapy vectors. However, the amounts of antigen required for induction of oral tolerance are very high and the methods of the invention provide a means for obtaining antigens that exhibit a significant improvement in induction of oral tolerance.

Expression library immunization (Barry *et al.* (1995) *Nature* 377: 632) is a particularly useful method of screening for optimal antigens for use in genetic vaccines. For example, to identify autoantigens present in *Yersinia, Shigella,* and the like, one can screen for induction of T cell responses in HLA-B27 positive individuals. Complexes that include epitopes of bacterial antigens and MHC molecules associated with autoimmune diseases, *e.g.*, HLA-B27 in association with *Yersinia* antigens can be used in the prevention of reactive arthritis and ankylosing spondylitis in HLA-B27 positive individuals.

Screening of optimized antigens can be done in animal models which are known to those of skill in the art. Examples of suitable models for various conditions include collagen induced arthritis, the NFS/sld mouse model of human Sjogren's syndrome; a 120 kD organ-specific autoantigen recently identified as an analog of human cytoskeletal protein (α-fodrin (Haneji *et al.* (1997) *Science* 276: 604), the New Zealand Black/White F1 hybrid mouse model of human SLE, NOD mice, a mouse model of human diabetes mellitus, fas/fas ligand mutant mice, which spontaneously develop autoimmune and lymphoproliferative disorders (Watanabe-Fukunaga *et al.* (1992) *Nature* 356: 314), and experimental autoimmune encephalomyelitis (EAE), in which myelin basic protein induces a disease that resembles human multiple sclerosis.

Autoantigens that can be shuffled according to the methods of the invention and used in vaccines for treating multiple sclerosis include, but are not limited to, myelin basic protein (Stinissen *et al.* (1996) *J. Neurosci. Res.* 45: 500-511) or a fusion protein of myelin basic protein and proteolipid protein (Elliott *et al.* (1996) *J. Clin. Invest.* 98: 1602-1612), proteolipid protein (PLP) (Rosener *et al.* (1997) *J. Neuroimmunol.* 75: 28-34), 2',3'-cyclic nucleotide 3'-phosphodiesterase (CNPase) *(Rosener et al.* (1997) *J. Neuroimmunol.* 75: 28-34), the Epstein Barr virus nuclear antigen-1 (EBNA-1) (Vaughan *et al.* (1996) *J. Neuroimmunol.* 69: 95-102), HSP70 (Salvetti *et al.* (1996) *J. Neuroimmunol.* 65: 143-53; Feldmann *et al.* (1996) *Cell* 85: 307).

Target antigens that, after shuffling according to the methods of the invention, can be used to treat scleroderma, systemic sclerosis, and systemic lupus erythematosus include, for example, (-2-GPI, 50 kDa glycoprotein (Blank *et al.* (1994) *J. Autoimmun.* 7: 441-455), Ku (p70/p80) autoantigen, or its 80-kd subunit protein (Hong *et al.* (1994) *Invest. Ophthalmol. Vis. Sci.* 35: 4023-4030; Wang *et al.* (1994) *J. Cell Sci.* 107: 3223-3233), the nuclear autoantigens La (SS-B) and Ro (SS-A) (Huang *et al.* (1997) *J. Clin. Immunol. 17:* 212-219; Igarashi *et al.* (1995) *Autoimmunity* 22: 33-42; Keech *et al.* (1996) *Clin. Exp. Immunol,* 104: 255-263; Manoussakis *et al. (1995) J. Autoimmun.* 8: 959-969; Topfer *et al.* (1995) *Proc. Nat 'l. Acad. Sci. USA* 92: 875-879), proteasome (-type subunit C9 (Feist *et al.* (1996) *J. Exp. Med.* 184: 1313-1318), Scleroderma antigens Rpp 30, Rpp 38 or Scl-70 (Eder *et al.* (1997) *Proc. Nat'l. Acad. Sci. USA* 94: 1101-1106; Hietarinta *et al.* (1994) *Br. J. Rheumatol.* 33: 323-326), the centrosome autoantigen PCM-1 (Bao *et al.* (1995) *Autoimmunity* 22: 219-228), polymyositis-scleroderma autoantigen (PM-Scl) (Kho *et al.* (1997) *J. Biol. Chem.* 272: 13426-13431), scleroderma (and other systemic autoimmune disease) autoantigen CENP-A (Muro *et al.* (1996) *Clin. Immunol. Immunopathol.* 78: 86-89), U5, a small nuclear ribonucleoprotein (snRNP) (Okano *et al.* (1996) *Clin. Immunol. Immunopathol.* 81: 41-47), the 100-kd protein of PM-Scl autoantigen (Ge *et al.* (1996) *Arthritis Rheum.* 39: 1588-1595), the nucleolar U3- and Th(7-2) ribonucleoproteins (Verheijen *et al.* (1994) *J. Immunol. Methods* 169: 173-182), the ribosomal protein L7 (Neu *et al.* (1995) *Clin. Exp. Immunol.* 100: 198-204), hPop1 (Lygerou *et al.* (1996) *EMBO J.* 15: 5936-5948), and a 36-kd protein from nuclear matrix antigen (Deng *et al.* (1996) *Arthritis Rheum.* 39: 1300-1307).

Hepatic autoimmune disorders can also be treated using improved recombinant antigens that are prepared according to the methods described herein. Among the antigens that are useful in such treatments are the cytochromes P450 and UDP-glucuronosyl-transferases (Obermayer-Straub and Manns (1996) *Baillieres Clin. Gastroenterol.* 10: 501-532), the cytochromes P450 2C9 and P450 1A2 (Bourdi *et al.* (1996) *Chem. Res. Toxicol.* 9: 1159-1166; Clemente *et al.* (1997) *J. Clin. Endocrinol. Metab.* 82: 1353-1361), LC-1 antigen (Klein *et al.* (1996) *J. Pediatr. Gastroenterol. Nutr.* 23: 461-465), and a 230-kDa Golgi-associated protein *(Funaki et al.* (1996) *Cell Struct. Funct.* 21: 63-72).

For treatment of autoimmune disorders of the skin, useful antigens include, but are not limited to, the 450 kD human epidermal autoantigen (Fujiwara *et al.* (1996) *J. Invest. Dermatol.* 106: 1125-1130), the 230 kD and 180 kD bullous pemphigoid antigens (Hashimoto (1995) *Keio J. Med.* 44: 115-123; Murakami *et al.* (1996) *J. Dermatol. Sci.* 13: 112-117), pemphigus foliaceus antigen (desmoglein 1), pemphigus vulgaris antigen (desmoglein 3), BPAg2, BPAg1, and type VII collagen (Batteux *et al.* (1997) *J. Clin. Immunol.* 17: 228-233; Hashimoto *et al.* (1996) *J. Dermatol. Sci.* 12:10-17), a 168-kDa mucosal antigen in a subset of patients with cicatricial pemphigoid (Ghohestani *et al.* (1996) *J. Invest. Dermatol.* 107: 136-139), and a 218-kd nuclear protein (218-kd Mi-2) (Seelig *et al.* (1995) *Arthritis Rheum.* 38: 1389-1399).

The methods of the invention are also useful for obtaining improved antigens for treating insulin dependent diabetes mellitus, using one or more of antigens which include, but are not limited to, insulin, proinsulin, GAD65 and GAD67, heat-shock protein 65 (hsp65), and islet-cell antigen 69 (ICA69) (French *et al.* (1997) *Diabetes* 46: 34-39; Roep (1996) *Diabetes* 45: 1147-1156; Schloot *et al.* (1997) *Diabetologia* 40: 332-338), viral proteins homologous to GAD65 (Jones and Crosby (1996) *Diabetologia* 39: 1318-1324), islet cell antigen-related protein-tyrosine phosphatase (PTP) (Cui *et al.* (1996) *J. Biol. Chem.* 271: 24817-24823), GM2-1 ganglioside (Cavallo *et al.* (1996) *J. Endocrinol.* 150: 113-120; Dotta *et al.* (1996) *Diabetes* 45: 1193-1196), glutamic acid decarboxylase (GAD) (Nepom (1995) *Curr. Opin. Immunol.* 7: 825-830; Panina-Bordignon *et al.* (1995) *J. Exp. Med.* 181: 1923-1927), an islet cell antigen (ICA69) (Karges *et al.* (1997) *Biochim. Biophys. Acta* 1360: 97-101; Roep *et al.* (1996) *Eur. J. Immunol.* 26: 1285-1289), Tep69, the single T cell epitope recognized by T cells from diabetes patients (Karges *et al.* (1997) *Biochim. Biophys. Acta* 1360: 97-101), ICA 512, an autoantigen of type I diabetes (Solimena *et al.* (1996) *EMBO J.* 15: 2102-2114), an islet-cell protein tyrosine phosphatase and the 37-kDa autoantigen derived from it in type 1 diabetes (including IA-2, IA-2) (La Gasse *et al.* (1997) *Mol. Med.* 3: 163-173), the 64 kDa protein from In-111 cells or human thyroid follicular cells that is immunoprecipitated with sera from patients with islet cell surface antibodies (ICSA) (Igawa *et al.* (1996) *Endocr. J.* 43: 299-306), phogrin, a homologue of the human transmembrane protein tyrosine phosphatase, an autoantigen of type 1 diabetes (Kawasaki *et al.* (1996) *Biochem. Biophys. Res. Commun.* 227: 440-447), the 40 kDa and 37 kDa tryptic fragments and their precursors IA-2 and IA-2 in IDDM (Lampasona *et al.* (1996) *J. Immunol.* 157: 2707-2711; Notkins *et al.* (1996) *J. Autoimmun.* 9: 677-682), insulin or a cholera toxoid-insulin conjugate (Bergerot *et al.* (1997) *Proc. Nat'l. Acad. Sci. USA* 94: 4610-4614), carboxypeptidase H, the human homologue of gp330, which is a renal epithelial glycoprotein involved in inducing Heymann nephritis in rats, and the 38-kD islet mitochondrial autoantigen (Arden *et al.* (1996) *J. Clin. Invest.* 97: 551-561.

Rheumatoid arthritis is another condition that is treatable using optimized antigens prepared according to the present invention. Useful antigens for rheumatoid arthritis treatment include, but are not limited to, the 45 kDa DEK nuclear antigen, in particular onset juvenile rheumatoid arthritis and iridocyclitis (Murray *et al.* (1997) *J. Rheumatol.* 24: 560-567), human cartilage glycoprotein-39, an autoantigen in rheumatoid arthritis *(Verheijden et al.* (1997) *Arthritis Rheum.* 40: 1115-1125), a 68k autoantigen in rheumatoid arthritis (Blass *et al.* (1997) *Ann. Rheum. Dis.* 56: 317-322), collagen (Rosloniec *et al.* (1995) *J. Immunol.* 155: 4504-4511), collagen type II *(Cook et al.* (1996) *Arthritis Rheum.* 39: 1720-1727; Trentham (1996) *Ann. N. Y. Acad. Sci.* 778: 306-314), cartilage link protein (Guerassimov *et al.* (1997) *J. Rheumatol.* 24: 959-964), ezrin, radixin and moesin, which are auto-immune antigens in rheumatoid arthritis (Wagatsuma *et al.* (1996) *Mol. Immunol.* 33: 1171-1176), and mycobacterial heat shock protein 65 (Ragno *et al,* (1997) *Arthritis Rheum.* 40: 277-283).

Also among the conditions for which one can obtain an improved antigen suitable for treatment are autoimmune thyroid disorders. Antigens that are useful for these applications include, for example, thyroid peroxidase and the thyroid stimulating hormone receptor (Tandon and Weetman (1994) *J. R. Coll. Physicians Lond.* 28: 10-18), thyroid peroxidase from human Graves' thyroid tissue (Gardas *et al.* (1997) *Biochem. Biophys. Res. Commun.* 234: 366-370; Zimmer *et al.* (1997) *Histochem. Cell. Biol.* 107: 115-120), a 64-kDa antigen associated with thyroid-associated ophthalmopathy (Zhang *et al.* (1996) *Clin. Immunol. Immunopathol.* 80: 236-244), the human TSH receptor (Nicholson *et al.* (1996) *J. Mol. Endocrinol.* 16: 159-170), and the 64 kDa protein from In-111 cells or human thyroid follicular cells that is immunoprecipitated with sera from patients with islet cell surface antibodies (ICSA) (Igawa *et al.* (1996) *Endocr. J.* 43: 299-306).

Other conditions and associated antigens include, but are not limited to, Sjogren's syndrome (-fodrin; Haneji *et al.* (1997) *Science* 276: 604-607), myastenia gravis (the human M2 acetylcholine receptor or fragments thereof, specifically the second extracellular loop of the human M2 acetylcholine receptor; Fu *et al.* (1996) *Clin. Immunol. Immunopathol.* 78: 203-207), vitiligo (tyrosinase; Fishman *et al.* (1997) *Cancer* 79: 1461-1464), a 450 kD human epidermal autoantigen recognized by serum from individual with blistering skin disease, and ulcerative colitis (chromosomal proteins HMG1 and HMG2; *Sobajima et al.* (1997) *Clin. Exp. Immunol.* 107: 135-140).

### 6. Cancer

Immunotherapy has great promise for the treatment of cancer and prevention of metastasis. By inducing an immune response against cancerous cells, the body's immune system can be enlisted to reduce or eliminate cancer. Improved antigens obtained using the methods of the invention provide cancer immunotherapies of increased effectiveness compared to those that are presently available.

One approach to cancer immunotherapy is vaccination using vaccines that include or encode antigens that are specific for tumor cells or by injecting the patients with purified recombinant cancer antigens. The methods of the invention can be used for obtaining antigens that exhibit an enhancement of immune responses against known tumor-specific antigens, and also to search for novel protective antigenic sequences. Antigens having optimized expression, processing, and presentation can be obtained as described herein. The approach used for each particular cancer can vary. For treatment of hormone-sensitive cancers (for example, breast cancer and prostate cancer), methods of the invention can be used to obtain optimized hormone antagonists. For highly immunogenic tumors, including melanoma, one can screen for recombinant antigens that optimally boost the immune response against the tumor. Breast cancer, in contrast, is of relatively low immunogenicity and exhibits slow progression, so individual treatments can be designed for each patient. Prevention of metastasis is also a goal in design of cancer vaccines.

Among the tumor-specific antigens that can be used in the antigen shuffling methods of the invention are: bullous pemphigoid antigen 2, prostate mucin antigen (PMA) (Beckett and Wright (1995) *Int. J Cancer* 62: 703-710), tumor associated Thomsen-Friedenreich antigen (Dahlenborg *et al.* (1997) *Int. J. Cancer* 70: 63-71), prostate-specific antigen (PSA) (Dannull and Belldegrun (1997) *Br. J Urol.* 1: 97-103), luminal epithelial antigen (LEA. 135) of breast carcinoma and bladder transitional cell carcinoma (TCC) (Jones *et al.* (1997) *Anticancer Res.* 17: 685-687), cancer-associated serum antigen (CASA) and cancer antigen 125 (CA 125) (Kierkegaard *et al.* (1995) *Gynecol. Oncol.* 59: 251-254), the epithelial glycoprotein 40 (EGP40) (Kievit *et al.* (1997) *Int. J. Cancer* 71: 237-245), squamous cell carcinoma antigen (SCC) (Lozza *et al.* (1997) *Anticancer Res.* 17: 525-529), cathepsin E (Mota *et al.* (1997) *Am. J. Pathol.* 150: 1223-1229), tyrosinase in melanoma (Fishman *et al.* (1997) *Cancer* 79: 1461-1464), cell nuclear antigen (PCNA) of cerebral cavernomas (Natelet *et al.* (1997) *Surg. Neurol.* 47: 364-370), DF3/MUC1 breast cancer antigen (Apostolopoulos *et al.* (1996) *Immunol. Cell. Biol.* 74: 457-464; Pandey *et al.* (1995) *Cancer Res.* 55: 4000-4003), carcinoembryonic antigen (Paone *et al.* (1996) *J. Cancer Res. Clin. Oncol.* 122: 499-503; Schlom *et al.* (1996) *Breast Cancer Res. Treat.* 38: 27-39), tumor-associated antigen CA 19-9 (Tolliver and O'Brien (1997) *South Med. J.* 90: 89-90; Tsuruta *et al.* (1997) *Urol. Int.* 58: 20-24), human melanoma antigens MART-l/Melan-A27-35 and gp 100 (Kawakami and Rosenberg (1997) *Int. Rev. Immunol.* 14: 173-192; *Zajac et al.* (1997) *Int. J. Cancer* 71: 491-496), the T and Tn pancarcinoma (CA) glycopeptide epitopes (Springer (1995) *Crit. Rev. Oncog.* 6: 57-85), a 35 kD tumor-associated autoantigen in papillary thyroid carcinoma (Lucas *et al.* (1996) *Anticancer Res.* 16: 2493-2496), KH-1 adenocarcinoma antigen (Deshpande and Danishefsky (1997) *Nature* 387: 164-166), the A60 mycobacterial antigen (Maes *et al.* (1996) *J. Cancer Res. Clin. Oncol.* 122: 296-300), heat shock proteins (HSPs) (Blachere and Srivastava (1995) *Semin. Cancer Biol.* 6: 349-355), and MAGE, tyrosinase, melan-A and gp75 and mutant oncogene products (*e.g.*, p53, ras, and HER-2/neu (Bueler and Mulligan (1996) *Mol. Med.* 2: 545-555; Lewis and Houghton (1995) *Semin. Cancer Biol.* 6: 321-327; Theobald *et al.* (1995) *Proc. Nat'l. Acad. Sci. USA* 92: 11993-11997).

### 7. Contraception

Genetic vaccines that contain optimized antigens obtained by the methods of the invention are also useful for contraception. For example, genetic vaccines can be obtained that encode sperm cell specific antigens, and thus induce anti-sperm immune responses. Vaccination can be achieved by, for example, administration of recombinant bacterial strains, *e.g.* Salmonella and the like, which express sperm antigen, as well as by induction of neutralizing anti-hCG antibodies by vaccination by DNA vaccines encoding human chorionic gonadotropin (hCG), or a fragment thereof.

Sperm antigens which can be used in the genetic vaccines include, for example, lactate dehydrogenase (LDH-C4), galactosyltransferase (GT), SP-10, rabbit sperm autoantigen (RSA), guinea pig (g)PH-20, cleavage signal protein (CS-1), HSA-63, human (h)PH-20, and AgX-1 (Zhu and Naz (1994) *Arch. Androl.* 33: 141-144), the synthetic sperm peptide, P10G (O'R and *et al.* (1993) *J. Reprod. Immunol.* 25: 89-102), the 135kD, 95kD, 65kD, 47kD, 41kD and 23kD proteins of sperm, and the FA-1 antigen (Naz *et al.* (1995) *Arch. Androl.* 35: 225-231), and the 35 kD fragment of cytokeratin 1 (Lucas *et al.* (1996) *Anticancer Res.* 16: 2493-2496).

The methods of the invention can also be used to obtain genetic vaccines that are expressed specifically in testis. For example, polynucleotide sequences that direct expression of genes that are specific to testis can be used (*e.g.*, fertilization antigen-1 and the like). In addition to sperm antigens, antigens expressed on oocytes or hormones regulating reproduction may be useful targets of contraceptive vaccines. For example, genetic vaccines can be used to generate antibodies against gonadotropin releasing hormone (GnRH) or zona pellucida proteins (Miller *et al.* (1997) *Vaccine* 15:1858-1862). Vaccinations using these molecules have been shown to be efficacious in animal models (Miller *et al.* (1997) *Vaccine* 15:1858-1862). Another example of a useful component of a genetic contraceptive vaccine is the ovarian zona pellucida glycoprotein ZP3 (Tung *et al.* (1994) *Reprod. Fertil. Dev.* 6:349-355).

### Methods of Selecting and Identifying Optimized Recombinant Antigens

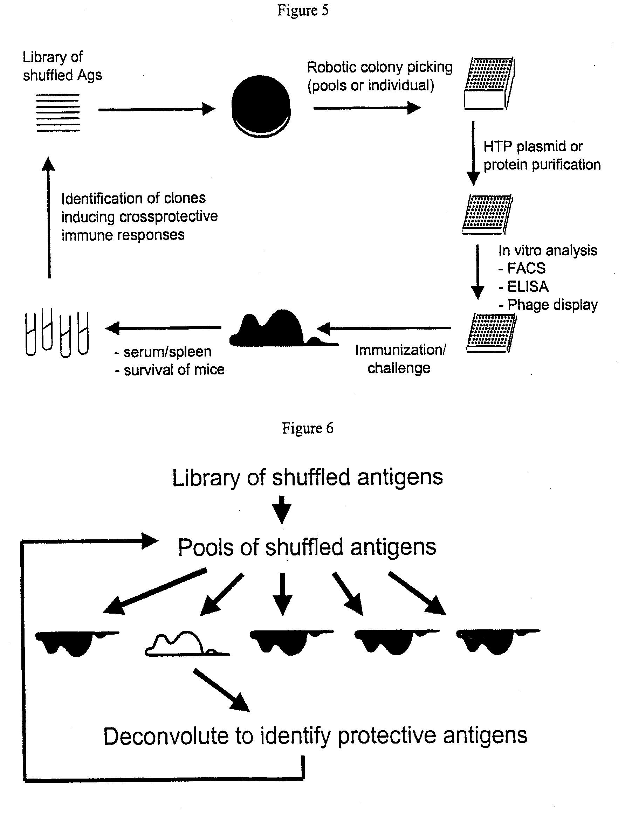

Once one has performed DNA shuffling to obtain a library of polynucleotides that encode recombinant antigens, the library is subjected to selection and/or screening to identify those library members that encode antigenic peptides that have improved ability to induce an immune response to the pathogenic agent. Selection and screening of recombinant polynucleotides that encode polypeptides having an improved ability to induce an immune response can involve either *in vivo* and *in vitro* methods, but most often involves a combination of these methods. For example, in a typical embodiment the members of a library of recombinant nucleic acids are picked, either individually or as pools. The clones can be subjected to analysis directly, or can be expressed to produce the corresponding polypeptides. In a presently preferred embodiment, an *in vitro* screen is performed to identify the best candidate sequences for the *in vivo* studies. Alternatively, the library can be subjected to *in vivo* challenge studies directly. The analyses can employ either the nucleic acids themselves (e.g., as genetic vaccines), or the polypeptides encoded by the nucleic acids. A schematic diagram of a typical strategy is shown in Figure 5. Both *in vitro* and in vivo methods are described in more detail below.

If a recombination cycle is performed *in vitro,* the products of recombination, *i.e.*, recombinant segments, are sometimes introduced into cells before the screening step. Recombinant segments can also be linked to an appropriate vector or other regulatory sequences before screening. Alternatively, products of recombination generated *in vitro* are sometimes packaged in viruses (*e.g.*, bacteriophage) before screening. If recombination is performed *in vivo,* recombination products can sometimes be screened in the cells in which recombination occurred. In other applications, recombinant segments are extracted from the cells, and optionally packaged as viruses, before screening.

Often, improvements are achieved after one round of recombination and selection. However, recursive sequence recombination can also be employed to achieve still further improvements in a desired property, or to bring about new (or "distinct") properties. Recursive sequence recombination entails successive cycles of recombination to generate molecular diversity. That is, one creates a family of nucleic acid molecules showing some sequence identity to each other but differing in the presence of mutations. In any given cycle, recombination can occur *in vivo* or *in vitro,* intracellularly or extracellularly. Furthermore, diversity resulting from recombination can be augmented in any cycle by applying prior methods of mutagenesis (*e.g.*, error-prone PCR or cassette mutagenesis) to either the substrates or products for recombination.

In a presently preferred embodiment, polynucleotides that encode optimized recombinant antigens are subjected to molecular backcrossing, which provides a means to breed the shuffled chimeras/mutants back to a parental or wild-type sequence, while retaining the mutations that are critical to the phenotype that provides the optimized immune responses. In addition to removing the neutral mutations, molecular backcrossing can also be used to characterize which of the many mutations in an improved variant contribute most to the improved phenotype. This cannot be accomplished in an efficient library fashion by any other method. Backcrossing is performed by shuffling the improved sequence with a large molar excess of the parental sequences.

The nature of screening or selection depends on what property or characteristic is to be acquired or the property or characteristic for which improvement is sought, and many examples are discussed below. It is not usually necessary to understand the molecular basis by which particular products of recombination (recombinant segments) have acquired new or improved properties or characteristics relative to the starting substrates. For example, a gene that encodes an antigenic polypeptide can have many component sequences each having a different intended role *(see, e.g.,* Figure 4). Each of these component sequences can be varied and recombined simultaneously. Screening/selection can then be performed, for example, for recombinant segments that have increased ability to induce an immune response to a pathogenic agent without the need to attribute such improvement to any of the individual component sequences of the recombinant polynucleotide.

Depending on the particular screening protocol used for a desired property, initial round(s) of screening can sometimes be performed using bacterial cells due to high transfection efficiencies and ease of culture. However, especially for testing of immunogenic activity, test animals are used for library expression and screening. Similarly other types of screening which are not amenable to screening in bacterial or simple eukaryotic library cells, are performed in cells selected for use in an environment close to that of their intended use. Final rounds of screening can be performed in cells or organisms that are as close as possible to the precise cell type or organism of intended use.

If further improvement in a property is desired, at least one, and usually a collection, of recombinant segments surviving a first round of screening/selection are subject to a further round of recombination. These recombinant segments can be recombined with each other or with exogenous segments representing the original substrates or further variants thereof. Again, recombination can proceed *in vitro or in vivo.* If the previous screening step identifies desired recombinant segments as components of cells, the components can be subjected to further recombination *in vivo,* or can be subjected to further recombination *in vitro,* or can be isolated before performing a round *of in vitro* recombination. Conversely, if the previous screening step identifies desired recombinant segments in naked form or as components of viruses, these segments can be introduced into cells to perform a round of *in vivo* recombination. The second round of recombination, irrespective how performed, generates further recombinant segments which encompass additional diversity than is present in recombinant segments resulting from previous rounds.

The second round of recombination can be followed by a further round of screening/selection according to the principles discussed above for the first round. The stringency of screening/selection can be increased between rounds. Also, the nature of the screen and the property being screened for can vary between rounds if improvement in more than one property is desired or if acquiring more than one new property is desired. Additional rounds of recombination and screening can then be performed until the recombinant segments have sufficiently evolved to acquire the desired new or improved property or function.

The practice of this invention involves the construction of recombinant nucleic acids and the expression of genes in transfected host cells. Molecular cloning techniques to achieve these ends are known in the art. A wide variety of cloning and *in vitro* amplification methods suitable for the construction of recombinant nucleic acids such as expression vectors are well-known to persons of skill. General texts which describe molecular biological techniques useful herein, including mutagenesis, include Berger and Kimmel, *Guide to Molecular Cloning Techniques, Methods in Enzymology* volume 152 Academic Press, Inc., San Diego, CA (Berger); Sambrook *et al., Molecular Cloning - A Laboratory Manual* (2nd Ed.), Vol. 1-3, Cold Spring Harbor Laboratory, Cold Spring Harbor, New York, 1989 ("Sambrook") and *Current Protocols in Molecular Biology,* F.M. Ausubel *et al.,* eds., Current Protocols, a joint venture between Greene Publishing Associates, Inc. and John Wiley & Sons, Inc., (supplemented through 1998) ("Ausubel")). Examples of techniques sufficient to direct persons of skill through *in vitro* amplification methods, including the polymerase chain reaction (PCR) the ligase chain reaction (LCR), Q - replicase amplification and other RNA polymerase mediated techniques (*e.g.*, NASBA) are found in Berger, Sambrook, and Ausubel, as well as Mullis *et al.* (1987) U.S. Patent No. 4,683,202; *PCR Protocols A Guide to Methods and Applications* (Innis *et al.* eds) Academic Press Inc. San Diego, CA (1990) (Innis); Amheim & Levinson (October 1, 1990) *C&EN* 36-47; *The Journal Of NIH Research* (1991) 3, 81-94; (Kwoh *et al.* (1989) *Proc. Natl. Acad. Sci. USA* 86, 1173; Guatelli *et al.* (1990) *Proc. Natl. Acad. Sci. USA* 87, 1874; Lomell *et al. (1989) J. Clin. Chem* 35, 1826; Landegren *et al.* (1988) *Science* 241, 1077-1080; Van Brunt (1990) *Biotechnology* 8, 291-294; Wu and Wallace (1989) *Gene* 4, 560; Barringer *et al.* (1990) *Gene* 89, 117, and Sooknanan and Malek (1995) *Biotechnology* 13: 563-564. Improved methods of *cloning in vitro* amplified nucleic acids are described in Wallace *et al.,* U.S. Pat. No. 5,426,039. Improved methods of amplifying large nucleic acids by PCR are summarized in Cheng *et al.* (1994) *Nature* 369: 684-685 and the references therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See,* Ausubel, Sambrook and Berger, *all supra.*

Oligonucleotides for use as probes, *e.g.,* in *in vitro* amplification methods, for use as gene probes, or as shuffling targets (*e.g.*, synthetic genes or gene segments) are typically synthesized chemically according to the solid phase phosphoramidite triester method described by Beaucage and Caruthers (1981) *Tetrahedron Letts.,* 22(20):1859-1862, *e.g.*, using an automated synthesizer, as described in Needham-VanDevanter *et al.* (1984) *Nucleic Acids Res.,* 12:6159-6168. Oligonucleotides can also be custom made and ordered from a variety of commercial sources known to persons of skill.

Indeed, essentially any nucleic acid with a known sequence can be custom ordered from any of a variety of commercial sources, such as The Midland Certified Reagent Company (mcrc@oligos.com), The Great American Gene Company (http://www.genco.com), ExpressGen Inc. (www.expressgen.com), Operon Technoloigies Inc. (Alameda, CA) and many others. Similarly, peptides and antibodies can be custom ordered from any of a variety of sources, such as PeptidoGenic (pkim@ccnet.com), HTI Bio-products, Inc. (http://www.htibio.com), BMA Biomedicals Ltd (U.K.), Bio Synthesis, Inc., and many others.

### 1. Purification and in vitro analysis of recombinant nucleic acids and polypeptides

Once DNA shuffling has been performed, the resulting library of recombinant polynucleotides can be subjected to purification and preliminary analysis *in vitro,* in order to identify the most promising candidate recombinant nucleic acids. Advantageously, the assays can be practiced in a high-throughput format. For example, to purify individual shuffled recombinant antigens, clones can robotically picked into 96-well formats, grown, and, if desired, frozen for storage.

Whole cell lysates (V-antigen), periplasmic extracts, or culture supernatants (toxins) can be assayed directly by ELISA as described below, but high throughput purification is sometimes also needed. Affinity chromatography using immobilized antibodies or incorporation of a small nonimmunogenic affinity tag such as a hexahistidine peptide with immobilized metal affinity chromatography will allow rapid protein purification. High binding-capacity reagents with 96-well filter bottom plates provide a high throughput purification process. The scale of culture and purification will depend on protein yield, but initial studies will require less than 50 micrograms of protein. Antigens showing improved properties can be purified in larger scale by FPLC for re-assay and animal challenge studies.

In some embodiments, the shuffled antigen-encoding polynucleotides are assayed as genetic vaccines. Genetic vaccine vectors containing the shuffled antigen sequences can be prepared using robotic colony picking and subsequent robotic plasmid purification. Robotic plasmid purification protocols are available that allow purification of 600-800 plasmids per day. The quantity and purity of the DNA can also be analyzed in 96-well plates, for example. In a presently preferred embodiment, the amount of DNA in each sample is robotically normalized, which can significantly reduce the variation between different batches of vectors.

Once the proteins and/or nucleic acids are picked and purified as desired, they can be subjected to any of a number of *in vitro* analysis methods. Such screenings include, for example, phage display, flow cytometry, and ELISA assays to identify antigens that are efficiently expressed and have multiple epitopes and a proper folding pattern. In the case of bacterial toxins, the libraries may also be screened for reduced toxicity in mammalian cells.

As one example, to identify recombinant antigens that are cross-reactive, one can use a panel of monoclonal antibodies for screening. A humoral immune response generally targets multiple regions of antigenic proteins. Accordingly, monoclonal antibodies can be raised against various regions of immunogenic proteins (Alving *et al. (1995) Immunol. Rev.* 145: 5). In addition, there are several examples of monoclonal antibodies that only recognize one strain of a given pathogen, and by definition, different serotypes of pathogens are recognized by different sets of antibodies. For example, a panel of monoclonal antibodies have been raised against VEE envelope proteins, thus providing a means to recognize different subtypes of the virus (Roehrig and Bolin (1997) *J. Clin. Microbiol.* 35: 1887). Such antibodies, combined with phage display and ELISA screening, can be used to enrich recombinant antigens that have epitopes from multiple pathogen strains. Flow cytometry based cell sorting will further allow for the selection of variants that are most efficiently expressed.

Phage display provides a powerful method for selecting proteins of interest from large libraries (Bass *et al.* (1990) *Proteins: Struct. Funct. Genet.* 8: 309; Lowman and Wells (1991) *Methods: A Companion to Methods Enz.* 3(3);205-216. Lowman and Wells (1993) *J. Mol. Biol.* 234;564-578). Some recent reviews on the phage display technique include, for example, McGregor (1996) *Mol Biotechnol.* 6(2):155-62; Dunn (1996) *Curr. Opin. Biotechnol.* 7(5):547-53; Hill *et al.* (1996) *Mol Microbiol* 20(4):685-92; *Phage Display of Peptides and Proteins: A Laboratory Manual.* BK. Kay, J. Winter, J, McCafferty eds., Academic Press 1996; O'Neil *et al.* (1995) *Curr. Opin. Struct. Biol.* 5(4):443-9; Phizicky *et al.* (1995) *Microbiol Rev.* 59(1):94-123; Clackson *et al.* (1994) *Trends Biotechnol.* 12(5):173-84; Felici *et al.* (1995) *Biotechnol. Annu. Rev.* 1:149-83; Burton (1995) *Immunotechnology* 1(2):87-94.) *See,also,* Cwirla *et al., Proc. Natl. Acad. Sci.* USA 87: 6378-6382 (1990); Devlin *et al., Science* 249: 404-406 (1990), Scott & Smith, *Science* 249: 386-388 (1990); Ladner *et al.,* US 5,571,698. Each phage particle displays a unique variant protein on its surface and packages the gene encoding that particular variant. The shuffled genes for the antigens are fused to a protein that is expressed on the phage surface, *e.g.*, gene III of phage M13, and cloned into phagemid vectors. In a presently preferred embodiment, a suppressible stop codon (*e.g.*, an amber stop codon) separates the genes so that in a suppressing strain of *E*. *coli*, the antigen-gIIIp fusion is produced and becomes incorporated into phage particles upon infection with M13 helper phage. The same vector can direct production of the unfused antigen alone in a nonsuppressing *E. coli* for protein purification.

The genetic packages most frequently used for display libraries are bacteriophage, particularly filamentous phage, and especially phage M13, Fd and Fl. Most work has involved inserting libraries encoding polypeptides to be displayed into either gIII or gVIII of these phage forming a fusion protein. *See, e.g.,* Dower, WO 91/19818; Devlin, WO 91/18989; MacCafferty, WO 92/01047 (gene III); Huse, WO 92/06204; Kang, WO 92/18619 (gene VIII). Such a fusion protein comprises a signal sequence, usually but not necessarily, from the phage coat protein, a polypeptide to be displayed and either the gene III or gene VIII protein or a fragment thereof. Exogenous coding sequences are often inserted at or near the N-terminus of gene III or gene VIII although other insertion sites are possible.

Eukaryotic viruses can be used to display polypeptides in an analogous manner. For example, display of human heregulin fused to gp70 of Moloney murine leukemia virus has been reported by Han *et al., Proc. Natl. Acad. Sci. USA* 92: 9747-9751 (1995). Spores can also be used as replicable genetic packages. In this case, polypeptides are displayed from the outer surface of the spore. For example, spores from *B. subtilis* have been reported to be suitable. Sequences of coat proteins of these spores are provided by *Donovan et al., J. Mol. Biol.* 196, 1-10 (1987). Cells can also be used as replicable genetic packages. Polypeptides to be displayed are inserted into a gene encoding a cell protein that is expressed on the cells surface. Bacterial cells including *Salmonella typhimurium, Bacillus subtilis, Pseudomonas aeruginosa, Vibrio cholerae, Klebsiella pneumonia, Neisseria gonorrhoeae, Neisseria meningitidis, Bacteroides nodosus, Moraxella bovis,* and especially *Escherichia coli* are preferred. Details of outer surface proteins are discussed by Ladner et al., US 5,571,698 and references cited therein. For example, the *lam*B protein of *E. coli* is suitable.

A basic concept of display methods that use phage or other replicable genetic package is the establishment of a physical association between DNA encoding a polypeptide to be screened and the polypeptide. This physical association is provided by the replicable genetic package, which displays a polypeptide as part of a capsid enclosing the genome of the phage or other package, wherein the polypeptide is encoded by the genome. The stablishment of a physical association between polypeptides and their genetic material allows simultaneous mass screening of very large numbers of phage bearing different polypeptides. Phage displaying a polypeptide with affinity to a target, *e.g.,* a receptor, bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means, or the polynucleotide that encodes the peptide or polypeptide can be used as part of a genetic vaccine.

Variants with specific binding properties, in this case binding to family-specific antibodies, are easily enriched by panning with immobilized antibodies. Antibodies specific for a single family are used in each round of panning to rapidly select variants that have multiple epitopes from the antigen families. For example, A-family specific antibodies can be used to select those shuffled clones that display A-specific epitopes in the first round of panning. A second round of panning with B-specific antibodies will select from the "A" clones those that display both A- and B-specific epitopes. A third round of panning with C-specific antibodies will select for variants with A, B, and C epitopes. A continual selection exists during this process for clones that express well in *E. coli* and that are stable throughout the selection. Improvements in factors such as transcription, translation, secretion, folding and stability are often observed and will enhance the utility of selected clones for use in vaccine production.

Phage ELISA methods can be used to rapidly characterize individual variants. These assays provide a rapid method for quantitation of variants without requiring purification of each protein. Individual clones are arrayed into 96-well plates, grown, and frozen for storage. Cells in duplicate plates are infected with helper phage, grown overnight and pelleted by centrifugation. The supernatants containing phage displaying particular variants are incubated with immobilized antibodies and bound clones are detected by anti-M13 antibody conjugates. Titration series of phage particles, immobilized antigen, and/or soluble antigen competition binding studies are all highly effective means to quantitate protein binding. Variant antigens displaying multiple epitopes will be further studied in appropriate animal challenge models.

Several groups have reported an *in vitro* ribosome display system for the screening and selection of mutant proteins with desired properties from large libraries. This technique can be used similarly to phage display to select or enrich for variant antigens with improved properties such as broad cross reactivity to antibodies and improved folding *(see,* e.g., Hanes *et al.* (1997) *Proc. Nat 'l. Acad. Sci. USA* 94(10):4937-42; Mattheakis *et al.* (1994) *Proc. Nat*^{*l*} *Acad. Sci. USA* 91(19):9022-6; He *et al.* (1997) *Nucl. Acids Res.* 25(24):5132-4; Nemoto *et al.* (1997) *FEBS Lett.* 414(2):405-8).

Other display methods exist to screen antigens for improved properties such as increased expression levels, broad cross reactivity, enhanced folding and stability. These include, but are not limited to display of proteins on intact *E. coli* or other cells *(e.g.,* Francisco *et al.* (1993) *Proc. Nat'l. Acad. Sci. USA* 90: 1044-10448; Lu *et al.* (1995) *Bio*/*Technology* 13: 366-372). Fusions of shuffled antigens to DNA-binding proteins can link the antigen protein to its gene in an expression vector (Schatz *et al.* (1996) *Methods Enzymol.* 267: 171-91; Gates *et al.* (1996) *J. Mol. Biol.* 255: 373-86.)

The various display methods and ELISA assays can be used to screen for shuffled antigens with improved properties such as presentation of multiple epitopes, improved immunogenicity, increased expression levels, increased folding rates and efficiency, increased stability to factors such as temperature, buffers, solvents, improved purification properties, etc. Selection of shuffled antigens with improved expression, folding, stability and purification profile under a variety of chromatographic conditions can be very important improvements to incorporate for the vaccine manufacturing process.

To identify recombinant antigenic polypeptides that exhibit improved expression in a host cell, flow cytometry is a useful technique. Flow cytometry provides a method to efficiently analyze the functional properties of millions of individual cells. One can analyze the expression levels of several genes simultaneously, and flow cytometry-based cell sorting allows for the selection of cells that display properly expressed antigen variants on the cell surface or in the cytoplasm. Very large numbers (>10⁷) of cells can be evaluated in a single vial experiment, and the pool of the best individual sequences can be recovered from the sorted cells. These methods are particularly useful in the case of, for example, Hantaan virus glycoproteins, which are generally very poorly expressed in mammalian cells. This approach provides a general solution to improve expression levels of pathogen antigens in mammalian cells, a phenomenon that is critical for the function of genetic vaccines.

To use flow cytometry to analyze polypeptides that are not expressed on the cell surface, one can engineer the recombinant polynucleotides in the library such that the polynucleotide is expressed as a fusion protein that has a region of amino acids which is targeted to the cell membrane. For example, the region can encode a hydrophobic stretch of C-terminal amino acids which signals the attachment of a phosphoinositol-glycan (PIG) terminus on the expressed protein and directs the protein to be expressed on the surface of the transfected cell (Whitehorn *et al.* (1995) *Biotechnology* (N Y) 13:1215-9). With an antigen that is naturally a soluble protein, this method will likely not affect the three dimensional folding of the protein in this engineered fusion with a new C-terminus. With an antigen that is naturally a transmembrane protein (*e.g.*, a surface membrane protein on pathogenic viruses, bacteria, protozoa or tumor cells) there are at least two possibilities. First, the extracellular domain can be engineered to be in fusion with the C-terminal sequence for signaling PIG-linkage. Second, the protein can be expressed *in toto* relying on the signalling of the host cell to direct it efficiently to the cell surface. In a minority of cases, the antigen for expression will have an endogenous PIG terminal linkage (*e.g.*, some antigens of pathogenic protozoa).

Those cells expressing the antigen can be identified with a fluorescent monoclonal antibody specific for the C-terminal sequence on PIG-linked forms of the surface antigen. FACS analysis allows quantitative assessment of the level of expression of the correct form of the antigen on the cell population. Cells expressing the maximal level of antigen are sorted and standard molecular biology methods are used to recover the plasmid DNA vaccine vector that conferred this reactivity. An alternative procedure that allows purification of all those cells expressing the antigen (and that may be useful prior to loading onto a cell sorter since antigen expressing cells may be a very small minority population), is to rosette or pan-purify the cells expressing surface antigen. Rosettes can be formed between antigen expressing cells and erythrocytes bearing covalently coupled antibody to the relevant antigen. These are readily purified by unit gravity sedimentation. Panning of the cell population over petri dishes bearing immobilized monoclonal antibody specific for the relevant antigen can also be used to remove unwanted cells.

In the high throughput assays of the invention, it is possible to screen up to several thousand different shuffled variants in a single day. For example, each well of a microtiter plate can be used to run a separate assay, or, if concentration or incubation time effects are to be observed, every 5-10 wells can test a single variant. Thus, a single standard microtiter plate can assay about 100 (*e.g.*, 96) reactions. If 1536 well plates are used, then a single plate can easily assay from about 100 to about 1500 different reactions. It is possible to assay several different plates per day; assay screens for up to about 6,000-20,000 different assays (*i*.*e*., involving different nucleic acids, encoded proteins, concentrations, *etc.*) is possible using the integrated systems of the invention. More recently, microfluidic approaches to reagent manipulation have been developed, *e.g.*, by Caliper Technologies (Palo Alto, CA).

In one aspect, library members, *e.g.*, cells, viral plaques, or the like, are separated on solid media to produce individual colonies (or plaques). Using an automated colony picker (*e.g.,* the Q-bot, Genetix, U.K.), colonies or plaques are identified, picked, and up to 10,000 different mutants inoculated into 96 well microtiter dishes, optionally containing glass balls in the wells to prevent aggregation. The Q-bot does not pick an entire colony but rather inserts a pin through the center of the colony and exits with a small sampling of cells (or viruses in plaque applications). The time the pin is in the colony, the number of dips to inoculate the culture medium, and the time the pin is in that medium each effect inoculum size, and each can be controlled and optimized. The uniform process of the Q-bot decreases human handling error and increases the rate of establishing cultures (roughly 10,000/4 hours). These cultures are then shaken in a temperature and humidity controlled incubator. The glass balls in the microtiter plates act to promote uniform aeration of cells dispersal of cells, or the like, similar to the blades of a fermentor. Clones from cultures of interest can be cloned by limiting dilution. Plaques or cells constituting libraries can also be screened directly for production of proteins, either by detecting hybridization, protein activity, protein binding to antibodies, or the like.

The ability to detect a subtle increase in the performance of a shuffled library member over that of a parent strain relies on the sensitivity of the assay. The chance of finding the organisms having an improvement in ability to induce an immune response is increased by the number of individual mutants that can be screened by the assay. To increase the chances of identifying a pool of sufficient size, a prescreen that increases the number of mutants processed by 10-fold can be used. The goal of the prescreen will be to quickly identify mutants having equal or better product titers than the parent strain(s) and to move only these mutants forward to liquid cell culture for subsequent analysis.

A number of well known robotic systems have also been developed for solution phase chemistries useful in assay systems. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, Hewlett-Packard, Palo Alto, Calif.) which mimic the manual synthetic operations performed by a scientist. Any of the above devices are suitable for use with the present invention, *e.g.*, for high-throughput screening of molecules encoded by codon-altered nucleic acids. The nature and implementation of modifications to these devices (if any) so that they can operate as discussed herein with reference to the integrated system will be apparent to persons skilled in the relevant art.

High throughput screening systems are commercially available (*see, e.g.,* Zymark Corp., Hopkinton, MA; Air Technical Industries, Mentor, OH; Beckman Instruments, Inc. Fullerton, CA; Precision Systems, Inc., Natick, MA, *etc.*). These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization.

The manufacturers of such systems provide detailed protocols the various high throughput. Thus, for example, Zymark Corp. provides technical bulletins describing screening systems for detecting the modulation of gene transcription, ligand binding, and the like. Microfluidic approaches to reagent manipulation have also been developed, *e.g.*, by Caliper Technologies (Palo Alto, CA).

Optical images viewed (and, optionally, recorded) by a camera or other recording device (*e.g.*, a photodiode and data storage device) are optionally further processed in any of the embodiments herein, *e.g.*, by digitizing the image and/or storing and analyzing the image on a computer. As noted above, in some applications, the signals resulting from assays are florescent, making optical detection approaches appropriate in these instances. A variety of commercially available peripheral equipment and software is available for digitizing, storing and analyzing a digitized video or digitized optical image, *e*.*g*., using PC (Intel x86 or Pentium chip- compatible DOS , OS2 WINDOWS , WINDOWS NT or WINDOWS95 based machines), MACINTOSH , or UNIX based (*e.g.*, SUN work station) computers.

One conventional system carries light from the assay device to a cooled charge-coupled device (CCD) camera, in common use in the art. A CCD camera includes an array of picture elements (pixels). The light from the specimen is imaged on the CCD. Particular pixels corresponding to regions of the specimen (*e.g.*, individual hybridization sites on an array of biological polymers) are sampled to obtain light intensity readings for each position. Multiple pixels are processed in parallel to increase speed. The apparatus and methods of the invention are easily used for viewing any sample, e.g., by fluorescent or dark field microscopic techniques.

Integrated systems for analysis in the present invention typically include a digital computer with high-throughput liquid control software, image analysis software, data interpretation software, a robotic liquid control armature for transferring solutions from a source to a destination operably linked to the digital computer, an input device (*e.g.*, a computer keyboard) for entering data to the digital computer to control high throughput liquid transfer by the robotic liquid control armature and, optionally, an image scanner for digitizing label signals from labeled assay component. The image scanner interfaces with the image analysis software to provide a measurement of optical intensity. Typically, the intensity measurement is interpreted by the data interpretation software to show whether the optimized recombinant antigenic polypeptide products are produced.

### 2. Antigen Library Immunization

In a presently preferred embodiment, antigen library immunization (ALI) is used to identify optimized recombinant antigens that have improved immunogenicity. ALI involves introduction of the library of recombinant antigen-encoding nucleic acids, or the recombinant antigens encoded by the shuffled nucleic acids, into a test animal. The animals are then subjected to *in vivo* challenge using live pathogens. Neutralizing antibodies and cross-protective immune responses are studied after immunization with the entire libraries, pools and/or individual antigen variants.

Methods of immunizing test animals are well known to those of skill in the art. In presently preferred embodiments, test animals are immunized twice or three times at two week intervals. One week after the last immunization, the animals are challenged with live pathogens (or mixtures of pathogens), and the survival and symptoms of the animals is followed. Immunizations using test animal challenge are described in, for example, Roggenkamp *et al.* (1997) *Infect. Immun.* 65: 446; Woody *et al.* (1997) *Vaccine* 2: 133; Agren *et al.* (1997) *J. Immunol.* 158: 3936; Konishi *et al.* (1992) *Virology* 190: 454; Kinney *et al.* (1988) *J. Virol.* 62: 4697; Iacono-Connors *et al.* (1996) *Virus Res.* 43: 125; Kochel *et al.* (1997) *Vaccine* 15: 547; and Chu *et al.* (1995) *J. Virol.* 69: 6417.

The immunizations can be performed by injecting either the recombinant polynucleotides themselves, *i.e.*, as a genetic vaccine, or by immunizing the animals with polypeptides encoded by the recombinant polynucleotides. Bacterial antigens are typically screened primarily as recombinant proteins, whereas viral antigens are preferably analyzed using genetic vaccinations.

To dramatically reduce the number of experiments required to identify individual antigens having improved immunogenic properties, one can use pooling and deconvolution, as diagrammed in Figure 6. Pools of recombinant nucleic acids, or polypeptides encoded by the recombinant nucleic acids, are used to immunize test animals. Those pools that result in protection against pathogen challenge are then subdivided and subjected to additional analysis. The high throughput *in vitro* approaches described above can be used to identify the best candidate sequences for the *in vivo* studies.

The challenge models that can be used to screen for protective antigens include pathogen and toxin models, such as *Yersinia* bacteria, bacterial toxins (such as *Staphylococcal* and *Streptococcal* enterotoxins, *E.coli*/*V. cholerae* enterotoxins), Venezuelan equine encephalitis virus (VEE), Flaviviruses (Japanese encephalitis virus, Tick-borne encephalitis virus, Dengue virus), Hantaan virus, *Herpes simplex,* influenza virus (*e.g.*, Influenza A virus), Vesicular Stomatitis Virus, *Pseudomonas aeruginosa, Salmonella typhimurium, Escherichia coli, Klebsiella pneumoniae, Toxoplasma gondii, Plasmodium yoelii, Herpes simplex,* influenza virus *(e.g.,* Influenza A virus), and Vesicular Stomatitis Virus. However, the test animals can also be challenged with tumor cells to enable screening of antigens that efficiently protect against malignancies. Individual shuffled antigens or pools of antigens are introduced into the animals intradermally, intramuscularly, intravenously, intratracheally, anally, vaginally, orally, or intraperitoneally and antigens that can prevent the disease are chosen, when desired, for further rounds of shuffling and selection. Eventually, the most potent antigens, based on *in vivo* data in test animals and comparative *in vitro* studies in animals and man, are chosen for human trials, and their capacity to prevent and treat human diseases is investigated.

In some embodiments, antigen library immunization and pooling of individual clones is used to immunize against a pathogen strain that was not included in the sequences that were used to generate the library. The level of crossprotection provided by different strains of a given pathogen can significantly. However, homologous titer is always higher than heterologous titer. Pooling and deconvolution is especially efficient in models where minimal protection is provided by the wild-type antigens used as starting material for shuffling (for example minimal protection by antigens A and B against strain C in Figure 3B). This approach can be taken, for example, when evolving the V-antigen of *Yersinae* or Hantaan virus glycoproteins.

In some embodiments, the desired screening involves analysis of the immune response based on immunological assays known to those skilled in the art. Typically, the test animals are first immunized and blood or tissue samples are collected for example one to two weeks after the last immunization. These studies enable one to one can measure immune parameters that correlate to protective immunity, such as induction of specific antibodies (particularly IgG) and induction of specific T lymphocyte responses, in addition to determining whether an antigen or pools of antigens provides protective immunity. Spleen cells or peripheral blood mononuclear cells can be isolated from immunized test animals and measured for the presence of antigen-specific T cells and induction of cytokine synthesis. ELISA, ELISPOT and cytoplasmic cytokine staining, combined with flow cytometry, can provide such information on a single-cell level.

Common immunological tests that can be used to identify the efficacy of immunization include antibody measurements, neutralization assays and analysis of activation levels or frequencies of antigen presenting cells or lymphocytes that are specific for the antigen or pathogen. The test animals that can be used in such studies include, but are not limited to, mice, rats, guinea pigs, hamsters, rabbits, cats, dogs, pigs and monkeys. Monkey is a particularly useful test animal because the MHC molecules of monkeys and humans are very similar.

Virus neutralization assays are useful for detection of antibodies that not only specifically bind to the pathogen, but also neutralize the function of the virus. These assays are typically based on detection of antibodies in the sera of immunized animal and analysis of these antibodies for their capacity to inhibit viral growth in tissue culture cells. Such assays are known to those skilled in the art. One example of a virus neutralization assay is described by Dolin R (*J*. *Infect. Dis.* 1995, 172:1175-83). Virus neutralization assays provide means to screen for antigens that also provide protective immunity.

In some embodiments, shuffled antigens are screened for their capacity to induce T cell activation *in vivo.* More specifically, peripheral blood mononuclear cells or spleen cells from injected mice can be isolated and the capacity of cytotoxic T lymphocytes to lyse infected, autologous target cells is studied. The spleen cells can be reactivated with the specific antigen *in vitro.* In addition, T helper cell activation and differentiation is analyzed by measuring cell proliferation or production of T_{H}1 (IL-2 and IFN-γ) and T_{H}2 (IL-4 and IL-5) cytokines by ELISA and directly in CD4⁺ T cells by cytoplasmic cytokine staining and flow cytometry. Based on the cytokine production profile, one can also screen for alterations in the capacity of the antigens to direct T_{H}1/'T_{H}2 differentiation (as evidenced, for example, by changes in ratios of IL-4/IFN-γ, IL-4/IL-2, IL-5/IFN-γ, IL-5/IL-2, IL-13/IFN-γ, IL-13/IL-2). The analysis of the T cell activation induced by the antigen variants is a very useful screening method, because potent activation of specific T cells in vivo correlates to induction of protective immunity.

The frequency of antigen-specific CD8⁺ T cells *in vivo* can also be directly analyzed using tetramers of MHC class I molecules expressing specific peptides derived from the corresponding pathogen antigens (Ogg and McMichael, *Curr. Opin. Immunol.* 1998, 10:393-6; Altman *et al., Science* 1996, 274:94-6). The binding of the tetramers can be detected using flow cytometry, and will provide information about the efficacy of the shuffled antigens to induce activation of specific T cells. For example, flow cytometry and tetramer stainings provide an efficient method of identifying T cells that are specific to a given antigen or peptide. Another method involves panning using plates coated with tetramers with the specific peptides. This method allows large numbers of cells to be handled in a short time, but the method only selects for highest expression levels. The higher the frequency of antigen-specific T cells *in vivo* is, the more efficient the immunization has been, enabling identification of the antigen variants that have the most potent capacity to induce protective immune responses. These studies are particularly useful when conducted in monkeys, or other primates, because the MHC class I molecules of humans mimic those of other primates more closely than those of mice.

Measurement of the activation of antigen presenting cells (APC) in response to immunization by antigen variants is another useful screening method. Induction of APC activation can be detected based on changes in surface expression levels of activation antigens, such as B7-1 (CD80), B7-2 (CD86), MHC class I and II, CD14, CD23, and Fc receptors, and the like.

Shuffled cancer antigens that induce cytotoxic T cells that have the capacity to kill cancer cells can be identified by measuring the capacity of T cells derived from immunized animals to kill cancer cells *in vitro.* Typically the cancer cells are first labeled with radioactive isotopes and the release of radioactivity is an indication of tumor cell killing after incubation in the presence of T cells from immunized animals. Such cytotoxicity assays are known in the art.

An indication of the efficacy of an antigen to activate T cells specific for, for example, cancer antigens, allergens or autoantigens, is also the degree of skin inflammation when the antigen is injected into the skin of a patient or test animal. Strong inflammation is correlated with strong activation of antigen-specific T cells. Improved activation of tumor-specific T cells may lead to enhanced killing of the tumors. In case of autoantigens, one can add immunomodulators that skew the responses towards T_{H}2, whereas in the case of allergens a T_{H}1 response is desired. Skin biopsies can be taken, enabling detailed studies of the type of immune response that occurs at the sites of each injection (in mice and monkeys large numbers of injections/antigens can be analyzed). Such studies include detection of changes in expression of cytokines, chemokines, accessory molecules, and the like, by cells upon injection of the antigen into the skin.

To screen for antigens that have optimal capacity to activate antigen-specific T cells, peripheral blood mononuclear cells from previously infected or immunized humans individuals can be used. This is a particularly useful method, because the MHC molecules that will present the antigenic peptides are human MHC molecules. Peripheral blood mononuclear cells or purified professional antigen-presenting cells (APCs) can be isolated from previously vaccinated or infected individuals or from patients with acute infection with the pathogen of interest. Because these individuals have increased frequencies of pathogen-specific T cells in circulation, antigens expressed in PBMCs or purified APCs of these individuals will induce proliferation and cytokine production by antigen-specific CD4⁺ and CD8⁺ T cells. Thus, antigens that simultaneously harbor epitopes from several antigens can be recognized by their capacity to stimulate T cells from various patients infected or immunized with different pathogen antigens, cancer antigens, autoantigens or allergens. One buffy coat derived from a blood donor contains lymphocytes from 0.5 liters of blood, and up to 10⁴ PBMC can be obtained, enabling very large screening experiments using T cells from one donor.

When healthy vaccinated individuals (lab volunteers) are studied, one can make EBV-transformed B cell lines from these individuals. These cell lines can be used as antigen presenting cells in subsequent experiments using blood from the same donor; this reduces interassay and donor-to-donor variation. In addition, one can make antigen-specific T cell clones, after which antigen variants are introduced to EBV transformed B cells. The efficiency with which the transformed B cells induce proliferation of the specific T cell clones is then studied. When working with specific T cell clones, the proliferation and cytokine synthesis responses are significantly higher than when using total PBMCs, because the frequency of antigen-specific T cells among PBMC is very low.

CTL epitopes can be presented by most cells types since the class I major histocompatibility complex (MHC) surface glycoproteins are widely expressed. Therefore, transfection of cells in culture by libraries of shuffled antigen sequences in appropriate expression vectors can lead to class I epitope presentation. If specific CTLs directed to a given epitope have been isolated from an individual, then the co-culture of the transfected presenting cells and the CTLs can lead to release by the CTLs of cytokines, such as IL-2, IFN-γ, or TNF, if the epitope is presented. Higher amounts of released TNF will correspond to more efficient processing and presentation of the class I epitope from the shuffled, evolved sequence. Shuffled antigens that induce cytotoxic T cells that have the capacity to kill infected cells can also be identified by measuring the capacity of T cells derived from immunized animals to kill infected cells in vitro. Typically the target cells are first labeled with radioactive isotopes and the release of radioactivity is an indication of target cell killing after incubation in the presence of T cells from immunized animals. Such cytotoxicity assays are known in the art.

A second method for identifying optimized CTL epitopes does not require the isolation of CTLs reacting with the epitope. In this approach, cells expressing class I MHC surface glycoproteins are transfected with the library of evolved sequences as above. After suitable incubation to allow for processing and presentation, a detergent soluble extract is prepared from each cell culture and after a partial purification of the MHC-epitope complex (perhaps optional) the products are submitted to mass spectrometry (Henderson *et al.* (1993) *Proc. Nat'l. Acad. Sci. USA* 90: 10275-10279). Since the sequence is known of the epitope whose presentation to be increased, one can calibrate the mass spectrogram to identify this peptide. In addition, a cellular protein can be used for internal calibration to obtain a quantitative result; the cellular protein used for internal calibration could be the MHC molecule itself. Thus one can measure the amount of peptide epitope bound as a proportion of the MHC molecules.

### Use of Recombinant Multivalent Antigens

The multivalent antigens of the invention are useful for treating and/or preventing the various diseases and conditions with which the respective antigens are associated. For example, the multivalent antigens can be expressed in a suitable host cell and are administered in polypeptide form. Suitable formulations and dosage regimes for vaccine delivery are well known to those of skill in the art.

In presently preferred embodiments, the optimized recombinant polynucleotides that encode improved allergens are used in conjunction with a genetic vaccine vector. The choice of vector and components can also be optimized for the particular purpose of treating allergy. For example, the polynucleotide that encodes the recombinant antigenic polypeptide can be placed under the control of a promoter, e.g., a high activity or tissue-specific promoter. The promoter used to express the antigenic polypeptide can itself be optimized using recombination and selection methods analogous to those described herein. The vector can contain immunostimulatory sequences such as are described in copending, commonly assigned US Patent Application Serial No. , entitled "Optimization of Immunomodulatory Molecules," filed as TTC Attorney Docket No. 18097-030300US on February 10, 1999. A vector engineered to direct a T_{H}1 response is preferred for many of the immune responses mediated by the antigens described herein (*see, e.g.,* copending, commonly assigned US Patent Application Serial No. , entitled "Genetic Vaccine Vector Engineering," filed on February 10, 1999 as TTC Attorney Docket No. 18097-030100US). It is sometimes advantageous to employ a genetic vaccine that is targeted for a particular target cell type (*e.g.*, an antigen presenting cell or an antigen processing cell); suitable targeting methods are described in copending, commonly assigned US patent application Serial No. , entitled "Targeting of Genetic Vaccine Vectors," filed on February 10, 1999 as TTC Attorney Docket No. 18097-030200US.

Genetic vaccines that encode the multivalent antigens described herein can be delivered to a mammal (including humans) to induce a therapeutic or prophylactic immune response. Vaccine delivery vehicles can be delivered *in vivo* by administration to an individual patient, typically by systemic administration (*e.g.*, intravenous, intraperitoneal, intramuscular, subdermal, intracranial, anal, vaginal, oral, buccal route or they can be inhaled) or they can be administered by topical application. Alternatively, vectors can be delivered to cells *ex vivo,* such as cells explanted from an individual patient *(e.g.,* lymphocytes, bone marrow aspirates, tissue biopsy) or universal donor hematopoietic stem cells, followed by reimplantation of the cells into a patient, usually after selection for cells which have incorporated the vector.

A large number of delivery methods are well known to those of skill in the art. Such methods include, for example liposome-based gene delivery (Debs and Zhu (1993) WO 93/24640; Mannino and Gould-Fogerite (1988) *BioTechniques* 6(7): 682-691; Rose U.S. Pat No. 5,279,833; Brigham (1991) WO 91/06309; and Felgner *et al.* (1987) *Proc. Natl. Acad. Sci. USA* 84: 7413-7414), as well as use of viral vectors (*e.g.*, adenoviral *(see, e.g.,* Berns *et al.* (1995) *Ann. NY Acad Sci.* 772: 95-104; Ali *et al.* (1994) *Gene Ther.* 1: 367-384; and *Haddada et al.* (1995) *Curr. Top. Microbiol. Immunol.* 199 ( Pt 3): 297-306 for review), papillomaviral, retroviral *(see, e.g.,* Buchscher *et al.* (1992) *J. Virol.* 66(5) 2731-2739; Johann *et al.* (1992) *J. Virol.* 66 (5):1635-1640 (1992); Sommerfelt *et al.,* (1990) *Virol.* 176:58-59; Wilson *et al.* (1989) *J. Virol.* 63:2374-2378; Miller et al., *J. Virol.* 65:2220-2224 (1991); Wong-Staal *et al.,* PCT/US94/05700, and Rosenburg and Fauci (1993) in *Fundamental Immunology, Third Edition* Paul (ed) Raven Press, Ltd., New York and the references therein, and Yu *et al., Gene Therapy* (1994) *supra.*)*,* and adeno-associated viral vectors *(see,* West *et al.* (1987) *Virology* 160:38-47; Carter *et al.* (1989) U.S. Patent No. 4,797,368; Carter *et al.* WO 93/24641 (1993); Kotin (1994) *Human Gene Therapy* 5:793-801; Muzyczka (1994) *J. Clin. Invst.* 94:1351 and Samulski (*supra*) for an overview of AAV vectors; *see also,* Lebkowski, U.S. Pat. No. 5,173,414; Tratschin *et al.* (1985) *Mol. Cell. Biol.* 5(11):3251-3260; Tratschin, *et al.* (1984) *Mol. Cell. Biol.,* 4:2072-2081; Hermonat and Muzyczka (1984) *Proc. Natl. Acad. Sci. USA,* 81:6466-6470; McLaughlin *et al. (198*8) and *Samulski et al.* (1989) *J. Virol.,* 63:03822-3828), and the like.

"Naked" DNA and/or RNA that comprises a genetic vaccine can be introduced directly into a tissue, such as muscle. *See, e.g.,* USPN 5,580,859. Other methods such as "biolistic" or particle-mediated transformation *(see, e.g.,* Sanford *et al.,* USPN 4,945,050; USPN 5,036,006) are also suitable for introduction of genetic vaccines into cells of a mammal according to the invention. These methods are useful not only for *in vivo* introduction of DNA into a mammal, but also for ex *vivo* modification of cells for reintroduction into a mammal. As for other methods of delivering genetic vaccines, if necessary, vaccine administration is repeated in order to maintain the desired level of immunomodulation.

Genetic vaccine vectors (*e.g.*, adenoviruses, liposomes, papillomaviruses, retroviruses, *etc.)* can be administered directly to the mammal for transduction of cells *in vivo.* The genetic vaccines obtained using the methods of the invention can be formulated as pharmaceutical compositions for administration in any suitable manner, including parenteral (*e.g.*, subcutaneous, intramuscular, intradermal, or intravenous), topical, oral, rectal, intrathecal, buccal (*e.g.*, sublingual), or local administration, such as by aerosol or transdermally, for prophylactic and/or therapeutic treatment. Pretreatment of skin, for example, by use of hair-removing agents, may be useful in transdermal delivery. Suitable methods of administering such packaged nucleic acids are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions of the present invention. A variety of aqueous carriers can be used, *e*.*g*., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of genetic vaccine vector in these formulations can vary widely, and will be selected primarily based on fluid volumes, viscosities, body weight and the like in accordance with the particular mode of administration selected and the patient's needs.

Formulations suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, tragacanth, microcrystalline cellulose, acacia, gelatin, colloidal silicon dioxide, croscarmellose sodium, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, usually sucrose and acacia or tragacanth, as well as pastilles comprising the active ingredient in an inert base, such as gelatin and glycerin or sucrose and acacia emulsions, gels, and the like containing, in addition to the active ingredient, carriers known in the art. It is recognized that the genetic vaccines, when administered orally, must be protected from digestion. This is typically accomplished either by complexing the vaccine vector with a composition to render it resistant to acidic and enzymatic hydrolysis or by packaging the vector in an appropriately resistant carrier such as a liposome. Means of protecting vectors from digestion are well known in the art. The pharmaceutical compositions can be encapsulated, *e.g.*, in liposomes, or in a formulation that provides for slow release of the active ingredient.

The packaged nucleic acids, alone or in combination with other suitable components, can be made into aerosol formulations (*e.g.*, they can be "nebulized") to be administered via inhalation. Aerosol formulations can be placed into pressurized acceptable propellants, such as dichlorodifluoromethane, propane, nitrogen, and the like.

Suitable formulations for rectal administration include, for example, suppositories, which consist of the packaged nucleic acid with a suppository base. Suitable suppository bases include natural or synthetic triglycerides or paraffin hydrocarbons. In addition, it is also possible to use gelatin rectal capsules which consist of a combination of the packaged nucleic acid with a base, including, for example, liquid triglycerides, polyethylene glycols, and paraffin hydrocarbons.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. Parenteral administration and intravenous administration are the preferred methods of administration. The formulations of packaged nucleic acid can be presented in unit-dose or multi-dose sealed containers, such as ampoules and vials.

Injection solutions and suspensions can be prepared from sterile powders, granules, and tablets of the kind previously described. Cells transduced by the packaged nucleic acid can also be administered intravenously or parenterally.

The dose administered to a patient, in the context of the present invention should be sufficient to effect a beneficial therapeutic response in the patient over time. The dose will be determined by the efficacy of the particular vector employed and the condition of the patient, as well as the body weight or vascular surface area of the patient to be treated. The size of the dose also will be determined by the existence, nature, and extent of any adverse side-effects that accompany the administration of a particular vector, or transduced cell type in a particular patient.

In determining the effective amount of the vector to be administered in the treatment or prophylaxis of an infection or other condition, the physician evaluates vector toxicities, progression of the disease, and the production of anti-vector antibodies, if any. In general, the dose equivalent of a naked nucleic acid from a vector is from about 1 µg to 1 mg for a typical 70 kilogram patient, and doses of vectors used to deliver the nucleic acid are calculated to yield an equivalent amount of therapeutic nucleic acid. Administration can be accomplished via single or divided doses.

In therapeutic applications, compositions are administered to a patient suffering from a disease (*e*.*g*., an infectious disease or autoimmune disorder) in an amount sufficient to cure or at least partially arrest the disease and its complications. An amount adequate to accomplish this is defined as a "therapeutically effective dose." Amounts effective for this use will depend upon the severity of the disease and the general state of the patient's health. Single or multiple administrations of the compositions may be administered depending on the dosage and frequency as required and tolerated by the patient. In any event, the composition should provide a sufficient quantity of the proteins of this invention to effectively treat the patient.

In prophylactic applications, compositions are administered to a human or other mammal to induce an immune response that can help protect against the establishment of an infectious disease or other condition.

The toxicity and therapeutic efficacy of the genetic vaccine vectors provided by the invention are determined using standard pharmaceutical procedures in cell cultures or experimental animals. One can determine the LD₅₀ (the dose lethal to 50% of the population) and the ED₅₀ (the dose therapeutically effective in 50% of the population) using procedures presented herein and those otherwise known to those of skill in the art.

A typical pharmaceutical composition for intravenous administration would be about 0.1 to 10 mg per patient per day. Dosages from 0.1 up to about 100 mg per patient per day may be used, particularly when the drug is administered to a secluded site and not into the blood stream, such as into a body cavity or into a lumen of an organ. Substantially higher dosages are possible in topical administration. Actual methods for preparing parenterally administrable compositions will be known or apparent to those skilled in the art and are described in more detail in such publications as *Remington's Pharmaceutical Science,* 15th ed., Mack Publishing Company, Easton, Pennsylvania (1980).

The multivalent antigenic polypeptides of the invention, and genetic vaccines that express the polypeptides, can be packaged in packs, dispenser devices, and kits for administering genetic vaccines to a mammal. For example, packs or dispenser devices that contain one or more unit dosage forms are provided. Typically, instructions for administration of the compounds will be provided with the packaging, along with a suitable indication on the label that the compound is suitable for treatment of an indicated condition. For example, the label may state that the active compound within the packaging is useful for treating a particular infectious disease, autoimmune disorder, tumor, or for preventing or treating other diseases or conditions that are mediated by, or potentially susceptible to, a mammalian immune response.

### EXAMPLES

The following examples are offered to illustrate, but not to limit the present invention.

### Example 1

### Development Of Broad-Spectrum Vaccines Against Bacterial Pathogens And Toxins

### A. Evolution of Yersinia V-antigens

This Example describes the use of DNA shuffling to develop immunogens that produce strong cross-protective immune responses against a variety of *Yersinia* strains. Passive immunization with anti-V-antigen antibodies or active immunization with purified V-antigen can provide protection from challenge with a virulent autologous *Yersinia* species. However, protection against heterologous species is limited (Motin *et al.* (1994) *Infect. Immun.* 62: 4192).

V-antigen genes from a variety of *Yersinia* strains, including serotypes of *Y. pestis, Y. enterocolitica,* and *Y. pseudotuberculosis* are subjected to DNA shuffling as described herein. The *Yersinia pestis* V antigen coding sequence, for example, is used as a query in a database search to identify homologous genes that can be used in a family shuffling format to obtain improved antigens. Results for a BLAST search of GenBank and EMBL databases are shown in Table 1, in which each line represents a unique sequence entry listing the database, accession number, locus name, bit score and E value. *See*, Altschul *et al.* (1997) *Nucleic Acids Res.* 25:3389-3402, for a description of the search algorithm). Homologous antigens have been cloned and sequenced from a number of related yet distinct *Yersinia* strains and additional natural diversity is obtained by cloning antigen genes from other strains. These genes and others or fragments thereof are cloned by methods such as PCR, shuffled and screened for improved antigens.

**Table 1**

| **Sequences producing significant alignments** | | | |
|---|---|---|---|
| **Database/Accession No.** | **Gene** | **Score (bits)** | **E Value** |
| gb\|M26405\|YEPLCR | *Yersinia pestis* 1crG, 1crV, and 1crH genes, co | 1945 | 0.0 |
| gb\|AF053946\|AF053946 | *Yersinia pestis* plasmid pCD1, complete pla | 1945 | 0.0 |
| emb\|X96802\|YPTPIVANT | *Y. pseudotuberculosis* V antigen gene | 1834 | 0.0 |
| gb\|M57893\|YEPLCRGVHP | *Yersinia pseudotuberculosis* V-antigen | 1818 | 0.0 |
| gb\|AF080155\|AF080155 | *Yersinia enterocolitica* pYV LcrV (lcrV) antigen | 1723 | 0.0 |
| emb\|X96801\|YE96PVANT | *Y. enterocolitica* V antigen gene, strain Y-... | 1667 | 0.0 |
| emb\|X96799\|YE108VANT | *Y. enterocolitica* V antigen gene, strain Y-... | 1659 | 0.0 |
| emb\|X96800\|YE527VANT | *Y. enterocolitica* V antigen gene, serotype ... | 1651 | 0.0 |
| emb\|X96798\|YE808VANT | *Y. enterocolitica* V antigen gene, strain 8081 | 1643 | 0.0 |
| emb\|X96796\|YE314VANT | Y. *enterocolitica* V antigen gene, strain WA. | 1237 | 0.0 |
| emb\|X96797\|YENCTVANT | *Y. enterocolitica* V antigen gene, strain NCTC | 1221 | 0.0 |
| gb\|S38727\|S38727 | lcrGVH operon: lcrV=V-antigen [*Yersinia pseudo.*] | 365 | 9e-99 |

Shuffled clones are selected by phage display and/or screened by ELISA to identify those recombinant nucleic acids that encode polypeptides that have multiple epitopes corresponding to the different serotypes. The shuffled antigen genes are cloned into a filamentous phage genome for polyvalent phage display or a suitable phagemid vector for monovalent phage display. A typical protocol for panning antigens by phage display is as follows.
- Coat an appropriate surface (e.g., Nunc Maxisorp tube or multiwell plate) at 4°C overnight with the target antibody, usually at a concentration of 1-10 µg/ml in PBS or other suitable buffer
- Rinse and Block with PBSM (PBS + 3% nonfat dry milk) at 37°C for 1-2 hr
- Pre-block phage if needed (PBSM, RT 1 hr)
- Rinse tube and allow phage to bind (usually 1 hr @ 37°C)
- Can vary time, temp, buffer, add a competitive inhibitor, etc.
- Wash extensively (15x) with PBST (PBS + 0.1% TWEEN20), then PBS
- Elute bound phage with low pH (*e.g.*, 10 mM glycine), 100 mM triethylamine, competitive ligand, protease, etc. and then neutralize pH if needed.
- Infect *E. coli* with eluted phage to transduce expression phagemid into new host. Titer and plate for colonies on drug plates
- Pool colonies into media, grow cells and infect with helper phage to produce phage for next round

Phage ELISA assays are a useful method to rapidly evaluate single clones after panning of libraries. Single colonies are picked in individual wells of a multiwell plate containing 2YT media and grown as a master plate. A replicate plate is infected with helper phage and grown so that phage from a single well will display a single antigen variant. A suitable protocol for phage ELISA assays is as follows.
- Coat microtiter plate with 50 µl of 1 µg/ml target antibody 4 °C overnight
- Rinse and block with PBSM for 2 hrs @ 37 °C
- Rinse, add preblocked phage and allow to bind 1 hr @ 37 °C
- Wash plates with PBST 3x, then PBS 3x with 2 min soaks
- Add HRP(or AP)-conjugated anti-M13 antibodies for 1 hr @ 37 °C
- Add substrate and measure absorbance
- Identify positive clones for further evaluation

ELISA assays can also be used to screen for individual antigens with multiple epitopes or increased expression levels. Single colonies are picked in individual wells of a multiwell plate containing appropriate media and grown as a master plate so that antigens produced from a single well are a single antigen variant. A replicate plate is grown and induced for protein production, *e.g*., by addition of 0.5 mM IPTG for Lac repressor-based systems and grown for an appropriate time for the antigen to be produced. At this point a crude antigen preparation is made which depends on the antigen and where it is produced. Secreted proteins can be evaluated by assaying the cell supernatants after centrifugation. Periplasmic proteins are often readily released from cells by simple extraction into hyper- or hypo-tonic buffers. Intracellularly produced proteins will require some form of cell lysis such as detergent treatment to release them. A suitable protocol for ELISA assays is as follows.
- Coat microtiter plate with 50 µl of 1 µg/ml target antibody 4 °C overnight
- Rinse and block with PBSM for 2 hrs @ 37 °C
- Rinse, add antigen prep and allow to bind 1 hr @ 37 °C
- Wash plates with PBST 3x
- Add HRP(or AP)-conjugated secondary antibody and incubate for 1 hr @ 37°C
- Add appropriate substrate and measure absorbance
- Identify positive clones for further evaluation

Antibodies specific for many of the various antigens are commercially available (*e.g.*, Toxin Technology, Inc, Sarasota, FL) or can be generated by immunizing suitable animals with purified antigens. Protein A or Protein G Sepharose (Pharmacia) can be used to purify immunoglobulins from the serum. Various affinity purification schemes can be used to further purify family-specific antibodies if needed such as immobilization of specific antigens to NHS-, CNBr-, or epoxy-activated sepharose beads. Other related antigens may be included soluble form to prevent binding and immobilization of cross-reactive antibodies.

The multivalent polypeptides that are identified by the initial screening protocol are purified and subjected to *in vivo* screening. For example, the shuffled antigens selected by a combination of any or none of these methods are purified and used to immunize animals, initially mice, which are then evaluated for improved immune responses. Typically 10 micrograms of protein is injected to a suitable location with or without appropriate adjuvant, *e.g.*, Alhydrogel (EM Seargent Pulp and Chemical, Inc.) and the animals are boosted with an additional dose after 2-4 weeks. At this point serum samples is drawn and evaluated by ELISA assay for the presence of antibodies that cross-react against multiple parental antigens. In this ELISA assay format the antigens are coated onto multiwell plates, then serial dilutions of each sera is allowed to bind. After washing unbound antibodies, a secondary HRP- or AP- conjugated antibody directed against the appropriate test antibody constant region, *e.g.*, goat anti-mouse IgG Fc (Sigma) is bound. After another washing, the appropriate substrate is added, *e.g.*, O-phenylenediamine (Sigma). The absorbance of each well is read by a plate reader at the appropriate wavelength (*e.g.*, 490 nm for OPD) and those producing high antibody titers to multiple antigens are selected for further evaluation.

Additionally, the ability of antigens to generate neutralizing antibodies can be evaluated in an appropriate system. Antigen variants that elicit a broad cross-reactive response are evaluated further in a virulent challenge model with the appropriate pathogenic organism. For example, the multivalent polypeptides are used to immunize mice, which are then challenged with live *Yersinia* bacteria. Those multivalent polypeptides that protect against the challenge are identified and purified.

### B. Evolution of broad-spectrum vaccines against bacterial toxins

This Example describes the use of DNA shuffling to obtain multivalent polypeptides that are effective in inducing an immune response against a broad spectrum of bacterial toxins.

### 1. Staphylococcus

The Group A *Streptococci,* which can cause diseases such as food poisoning, toxic shock syndrome, and autoimmune disorders, are highly toxic by inhalation. The family of Group A *Streptococcus* toxins numbers about 30 related members, making this group a suitable target for family shuffling. Accordingly, this Example describes the use of family DNA shuffling to create chimeric proteins that are capable of eliciting broad spectrum protection.

Nucleic acids that encode many diverse attenuated toxins are subjected to DNA shuffling as described herein. Table 2 shows the output of a BLAST search of GenBank, PDL, EMBL, and Swissprot using the *S. aureus* enterotoxin B protein to identify homologous genes that may be used in a family shuffling format to obtain improved antigens.

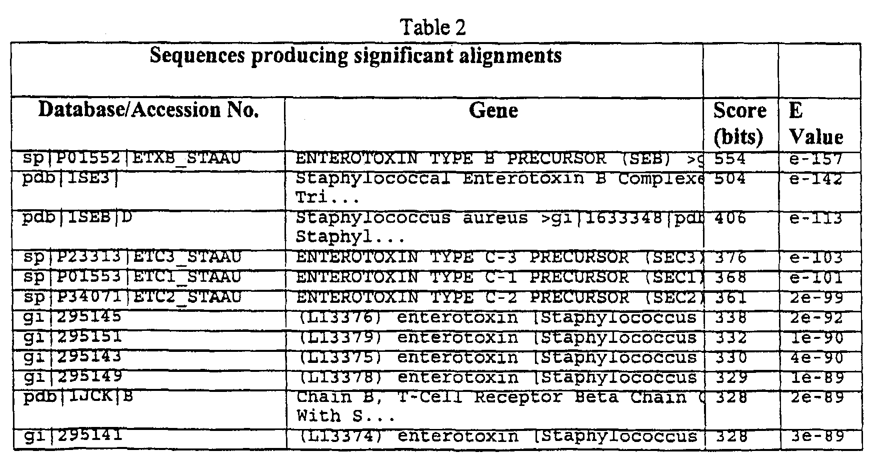

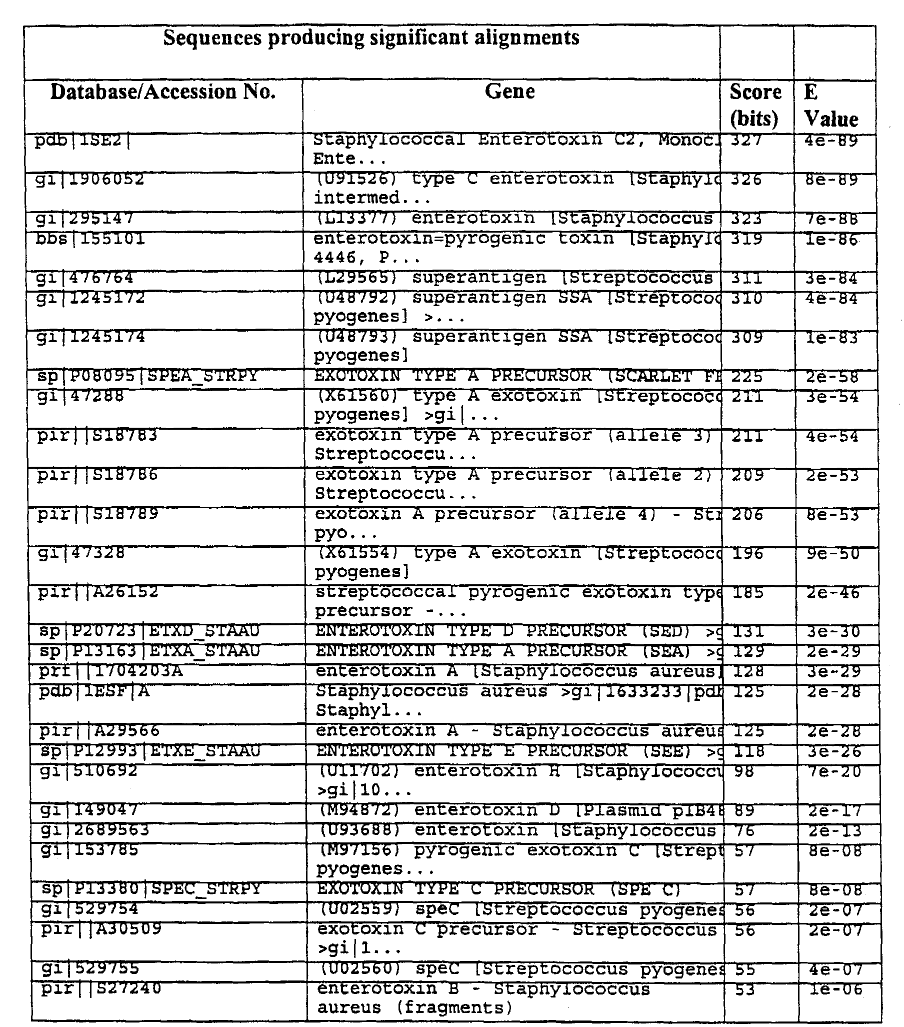

Shuffled recombinant clones are initially selected by phage display and/or screened by ELISA for the presence of multiple epitopes from the different families. Variant proteins with multiple epitopes are purified and used to for *in vivo* screening as described above. The mouse sera are analyzed for antibodies specific for different toxin subtypes and variants that elicit broadly cross-reactive responses will be evaluated further in challenge models.

### 2. Escherichia coli and Vibrio cholerae

This Example describes the use of DNA shuffling to obtain cross-reactive multivalent polypeptides that induce an immune response against the *E. coli* heat-labile toxin (LT), cholera toxin (CT), and verotoxin (VT). Nucleic acids that encode cholera and LT toxin B-chains are subjected to DNA shuffling. Table 3 shows the results of a BLAST search using the *V. cholerae* toxin B-chain to identify homologous genes that can be used in a family shuffling format to obtain improved antigens. Homologous antigens have been cloned and sequenced from a number of related yet distinct *Vibrio* and *E. coli* strains, and additional natural diversity can be obtained by cloning antigen genes from other strains. These genes and others or fragments thereof can be cloned by methods such as PCR, shuffled and screened for improved antigens.

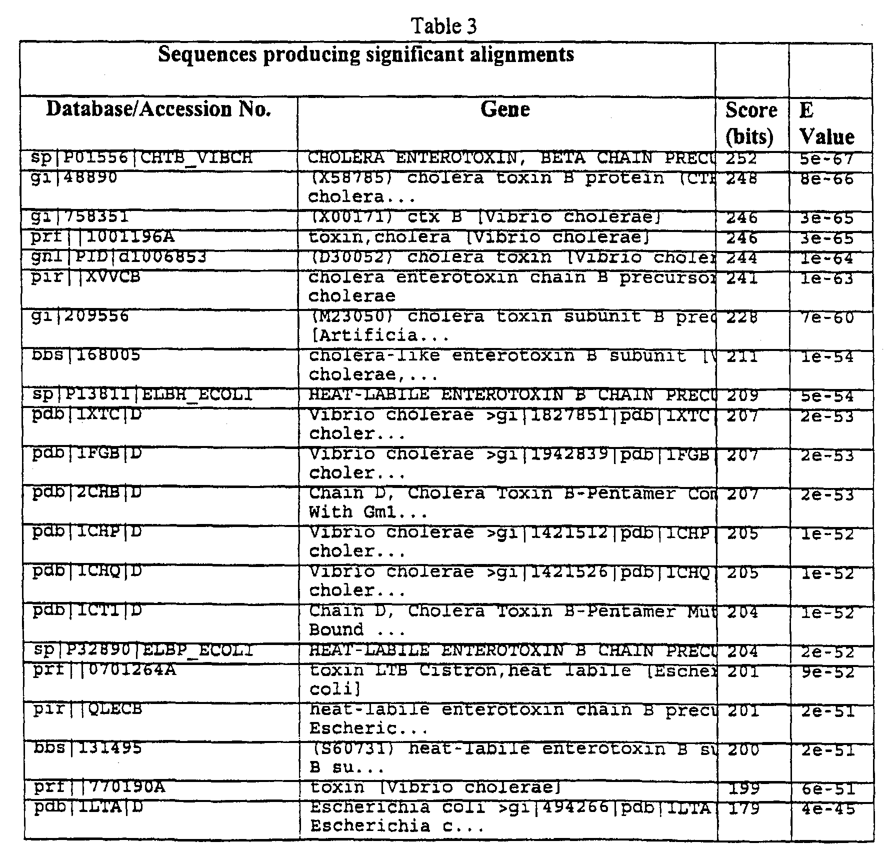

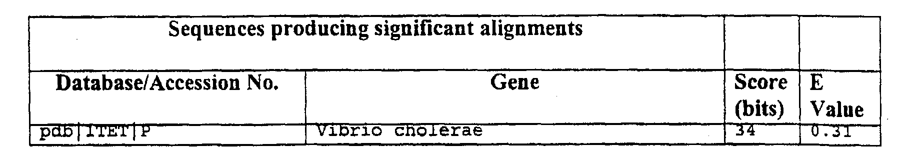

Those chimeric toxins that elicit high levels of neutralizing antibodies against both toxins and have improved adjuvant properties are identified. For example, shuffled clones are selected by phage display and/or screened by ELISA assays for the presence of epitopes from the different parental B-chains. Variants with multiple epitopes are purified and further studied for their capacity to act as adjuvants and to elicit cross-protective immune responses in challenge models.

### Example 2

### Evolution of Broad-spectrum Vaccines against Borrelia burgdorferi

Lyme disease is currently one of the fastest-growing infectious diseases in the United States. It is caused by infection of the spirochete bacterium *Borrelia burgdorferi,* which is carried and spread by the bite of infected ticks. Early signs of infection include skin rash and flu-like symptoms. If left untreated Lyme disease can cause arthritis, heart abnormalities, and facial paralysis. Treatment of early Lyme disease with antibiotics can stop the infection, but a lasting immunity may not develop making reinfection possible. A current vaccine requires three immunizations over a 1-year period to acquire immunity.

Both passive and active immunization with the purified *B. burgdorferi* outer surface protein A (OspA) protein has been successful in protecting against infection with *B. burgdorferi,* but has no effect against ongoing infections, since this antigen is not expressed in vertebrate hosts. OspA is normally anchored on the outside of the cell by a covalently attached lipid moiety through an amino terminal cysteine residue. In contrast, the outer surface protein C (OspC) is highly expressed by the spirochete in vertebrate hosts and vaccination of infected individuals with OspC may be an effective therapeutic in curing the infection (Zhong *et al.* (1997) *Proc. Nat'l. Acad. Sci. USA* 94 12533-12538.

A recent BLAST search (Altschul, *et al.,* (1997) Nucleic Acids Res. 25:3389-3402) of the non-redundant GenBank, PDB, SwissProt, Spupdate, and PIR databases was used to identify homologues of the OspA outer surface protein gene. This resulted in the identification of over 200 entries related to OspA. One hundred entries are shown in Table 4 below from different strains of *B. burgdorferi. B. garinii. B. afzelii, B. tanukii,* and *B. turdi* that share at least 83% DNA sequence identity to the *Borrelia burgdorferi* OspA protein. The *ospA* genes from these and other strains provide a source of diversity for family shuffling to obtain improved antigens for the prevention of Lyme disease. These genes are cloned by methods such as PCR, shuffled and screened for improved antigens.

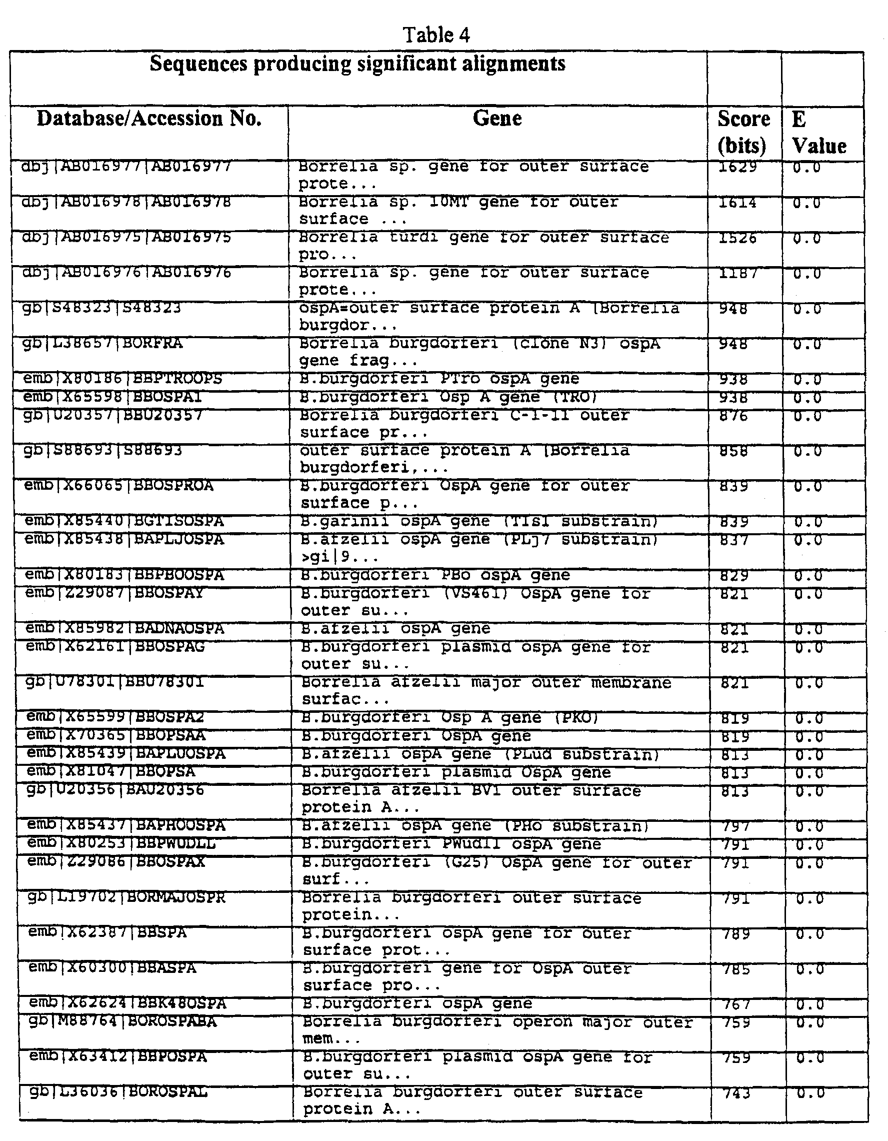

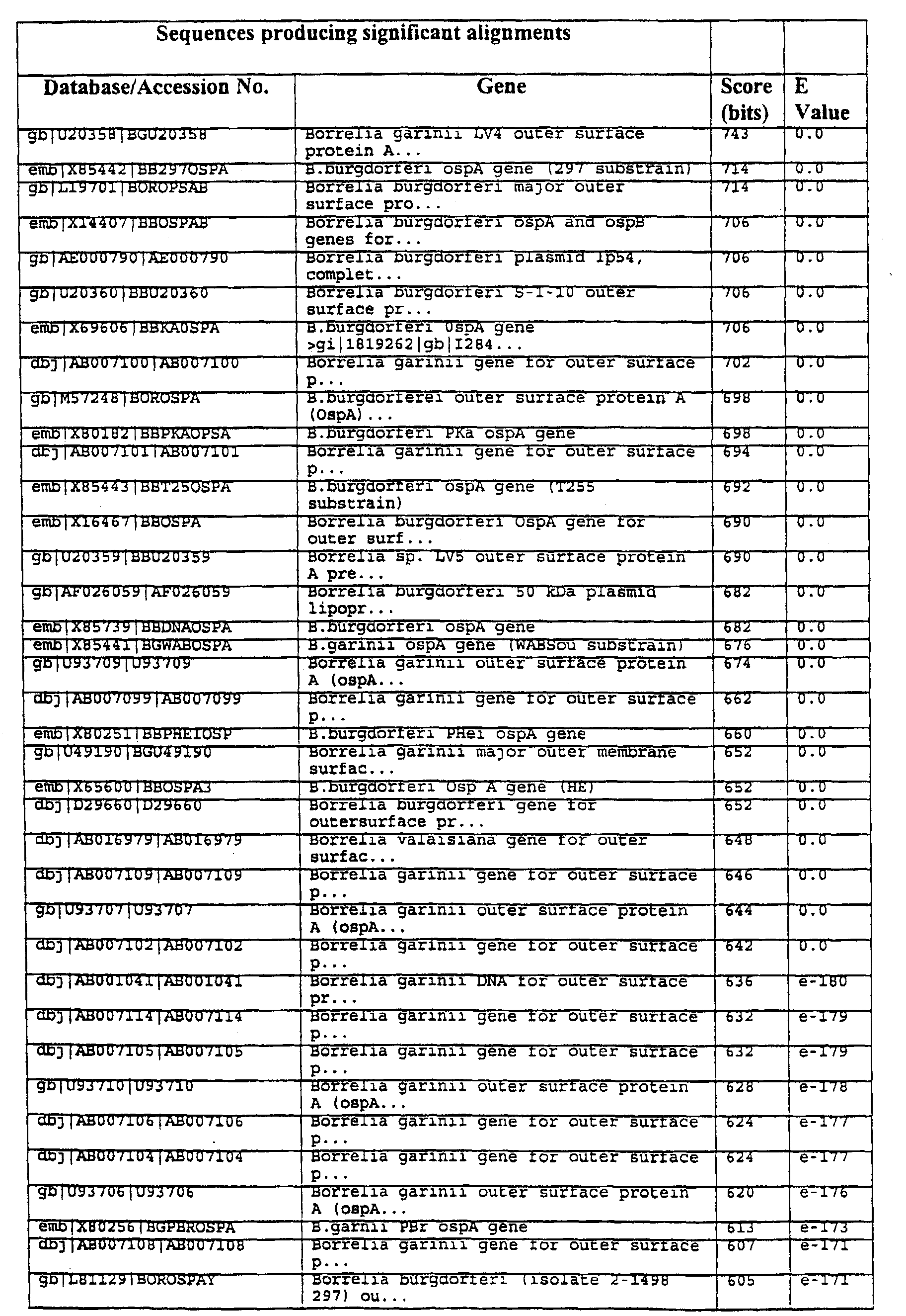

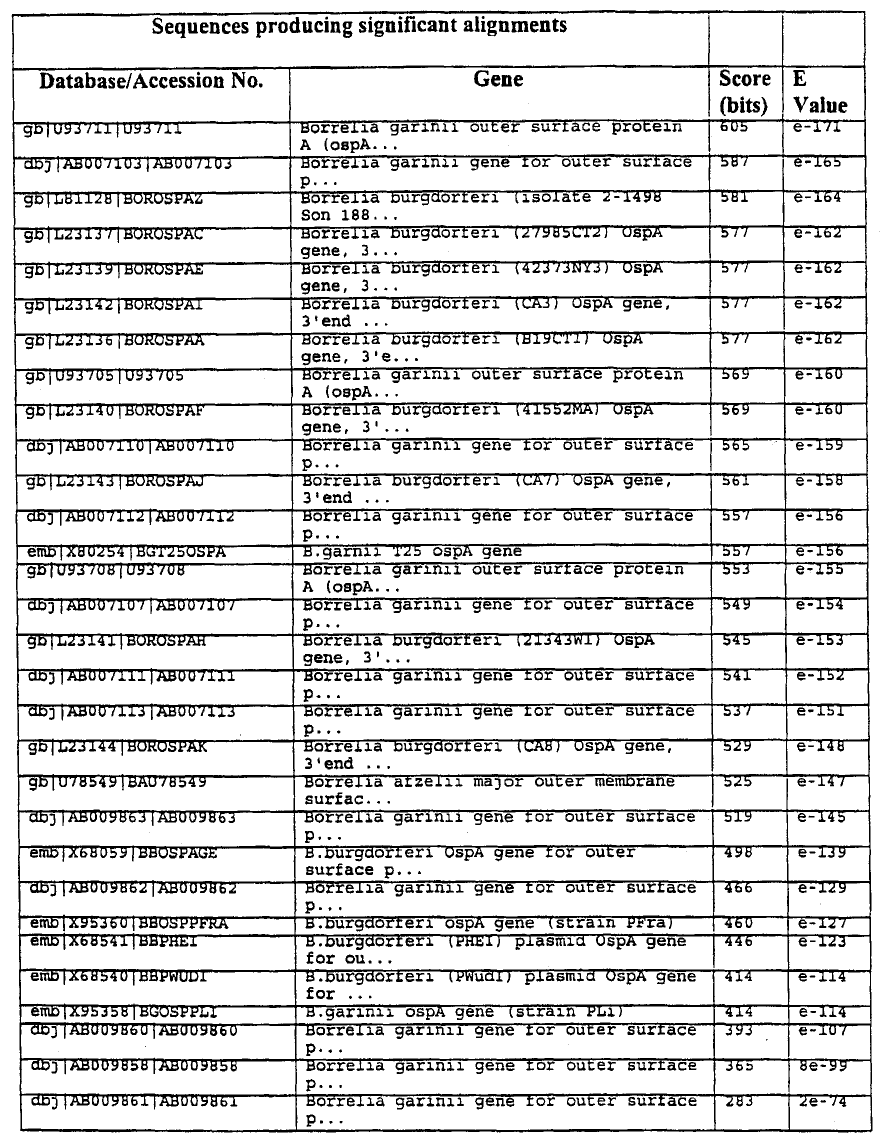

A BLAST search with the *B. burgdorferi* OspC protein gene revealed over 200 related entries. Entries for one hundred sequences sharing at least 82% DNA sequence identity are shown in Table 5 below that provide a source of diversity for family shuffling to obtain improved therapeutics in the treatment of Lyme disease. These genes are cloned by methods such as PCR, shuffled and screened for improved antigens.

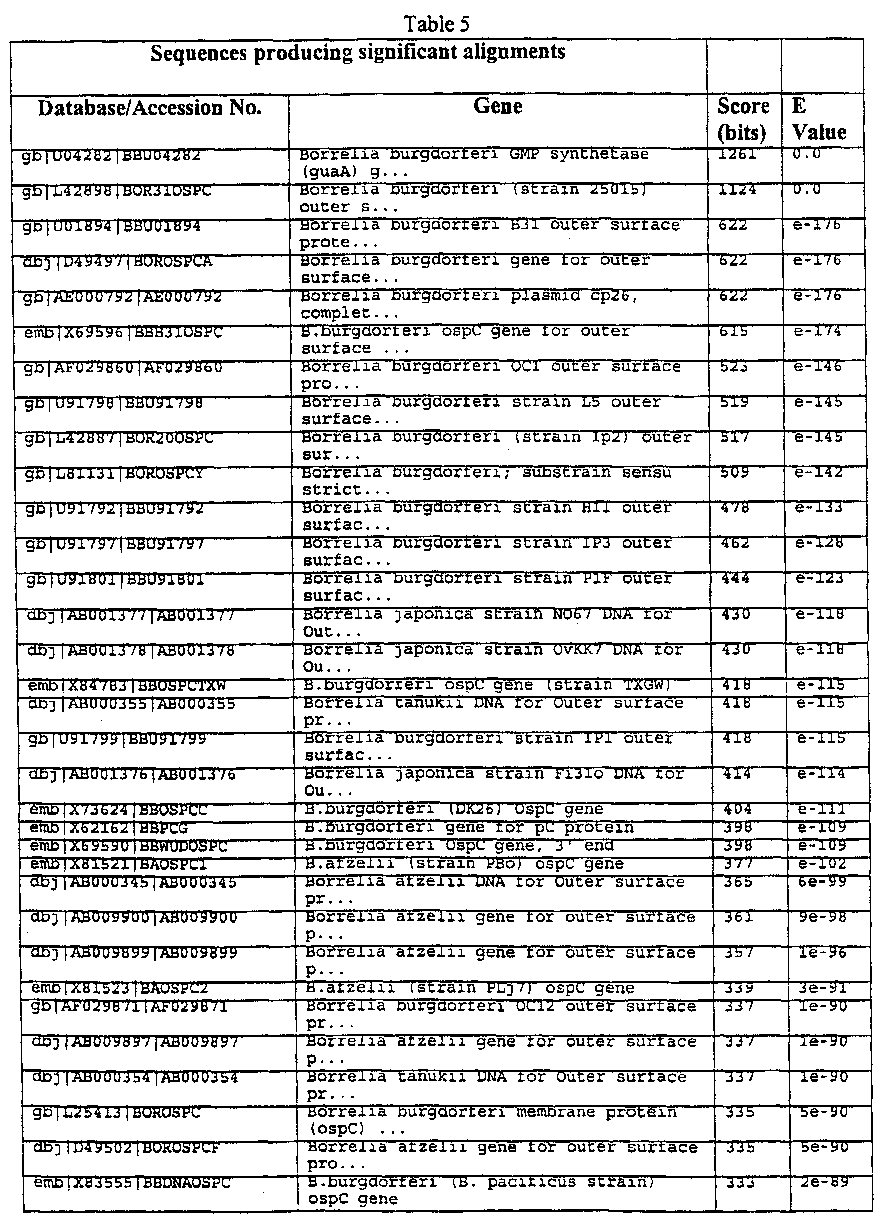

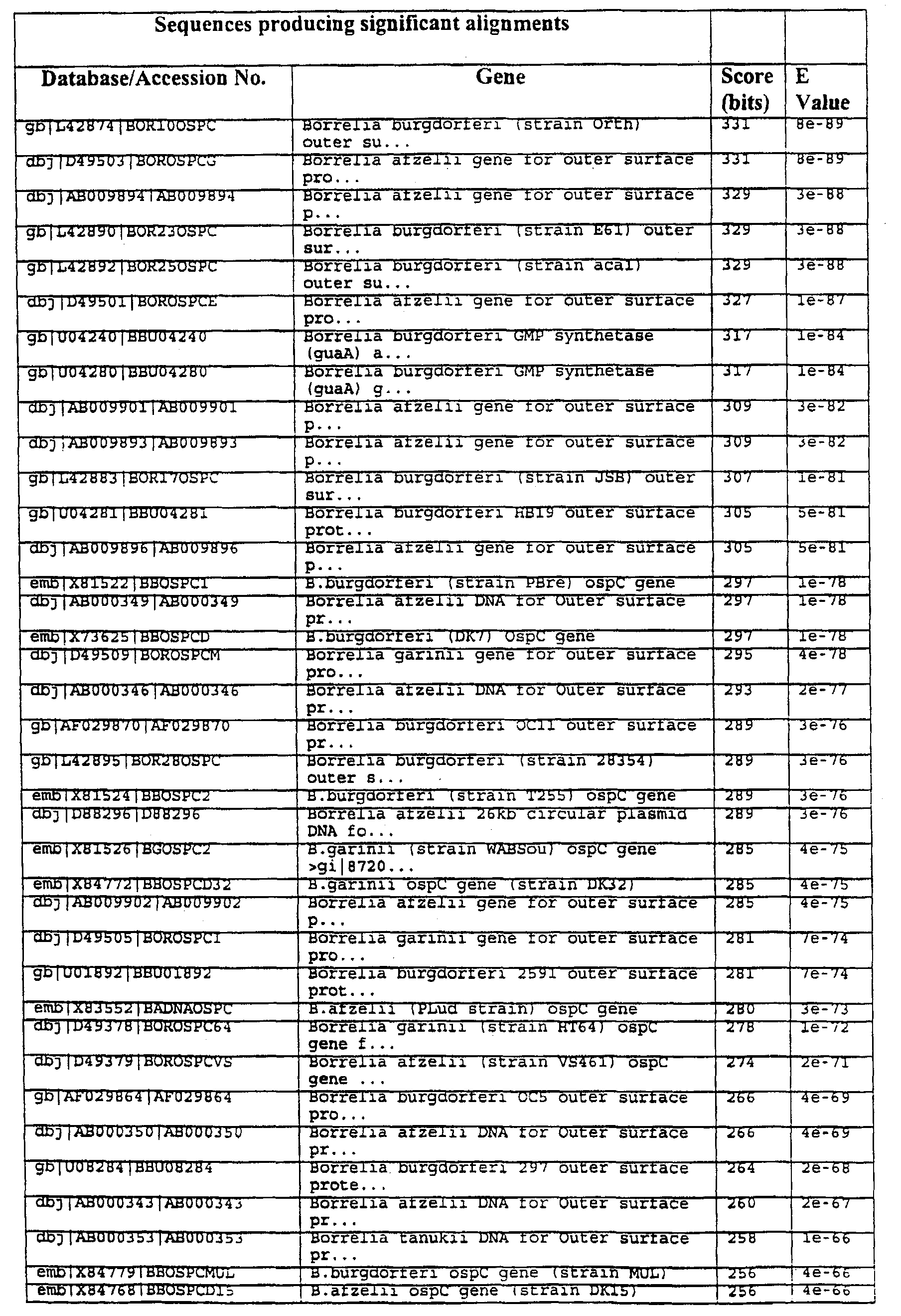

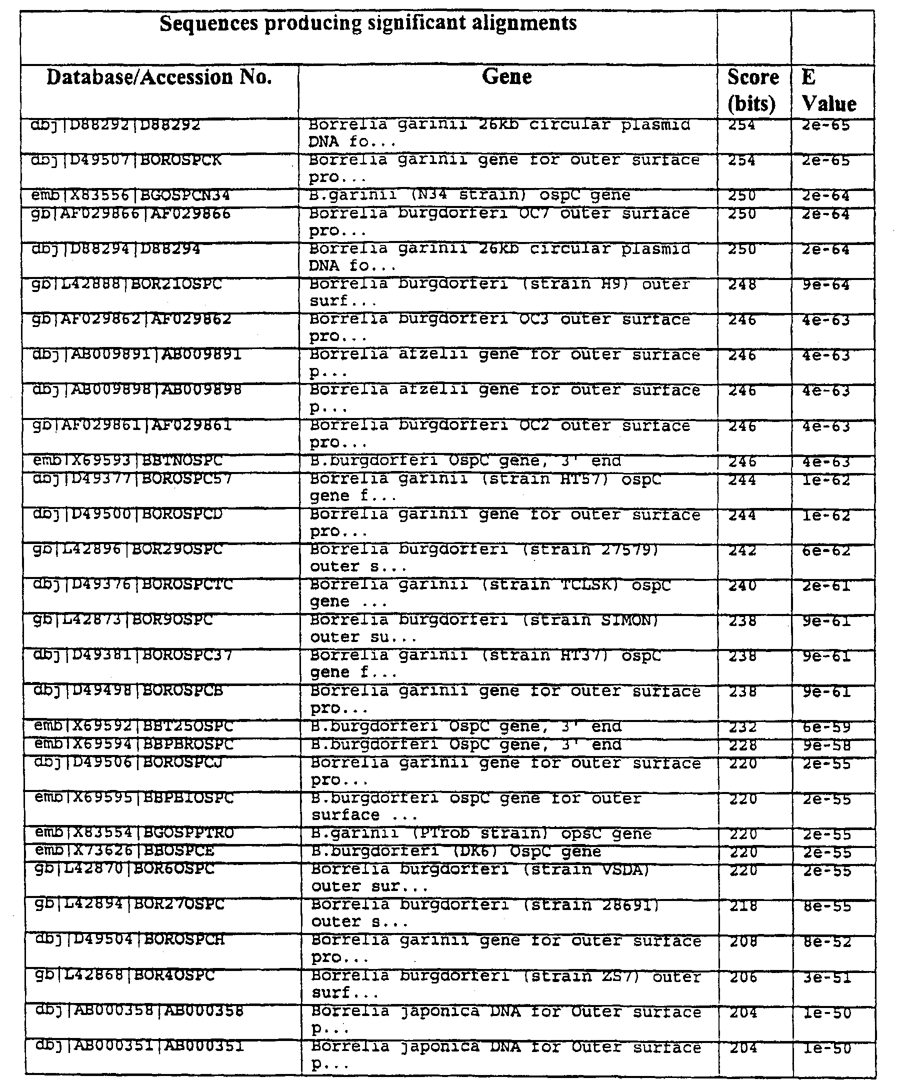

### Example 3

### Evolution of Broad-spectrum Vaccines against Mycobacterium

Tuberculosis is an ancient bacterial disease caused by *Mycobacterium tuberculosis* that continues to be an important public health problem worldwide and calls are being made for an improved effort in eradication (*Morb. Mortal Wkly Rep* (1998 Aug 21; 47(RR-13): 1-6). It infects over 50 million people and over 3 million people will die from tuberculosis this year. The currently available vaccine, Bacille Calmette-Guerin (BCG) is found to be less effective in developing countries and an increasing number of multidrug-resistant (MDR) strains are being isolated.

The major immunodominant antigen of *M. tuberculosis* is the 30-35 kDa (a.k.a. antigen 85, alpha-antigen) which is normally a lipoglycoprotein on the cell surface. Other protective antigens include a 65-kDa heat shock protein, and a 36-kDa proline-rich antigen (*Tascon et al.* (1996) *Nat. Med.* 2: 888-92).

Table 6 shows the output of a BLAST search using the 30-35 kDa major *M. Tuberculosis* antigen (a.k.a. antigen 85, alpha-antigen) coding sequence to identify homologous genes that may be used in a family shuffling format to obtain improved antigens. Many homologous antigens have been cloned and sequenced from a large number of related yet distinct mycobacterial strains. These genes are cloned by methods such as PCR, shuffled and screened for improved antigens.

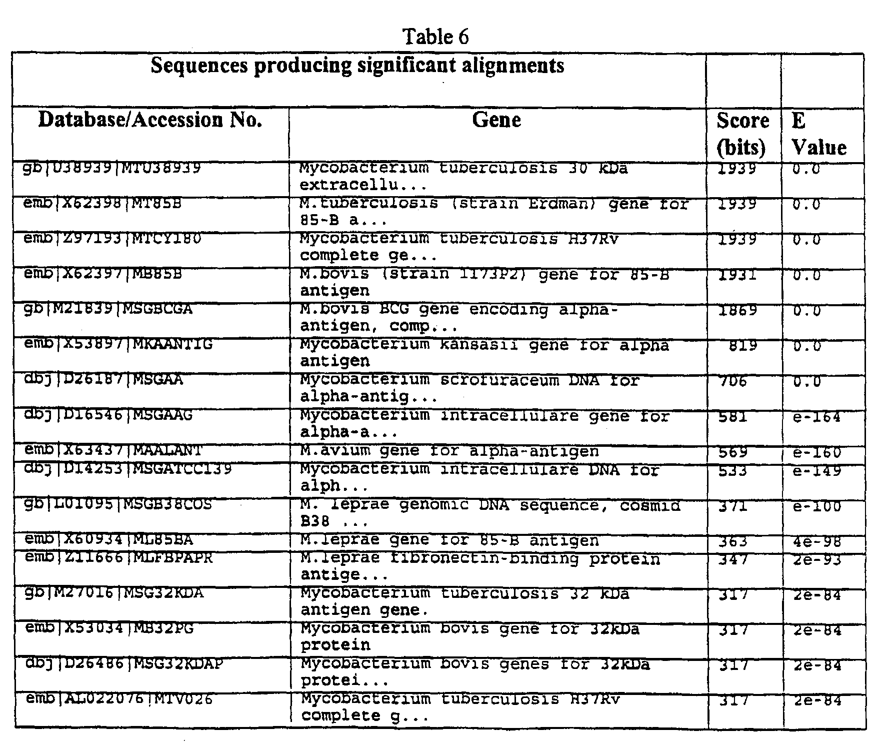

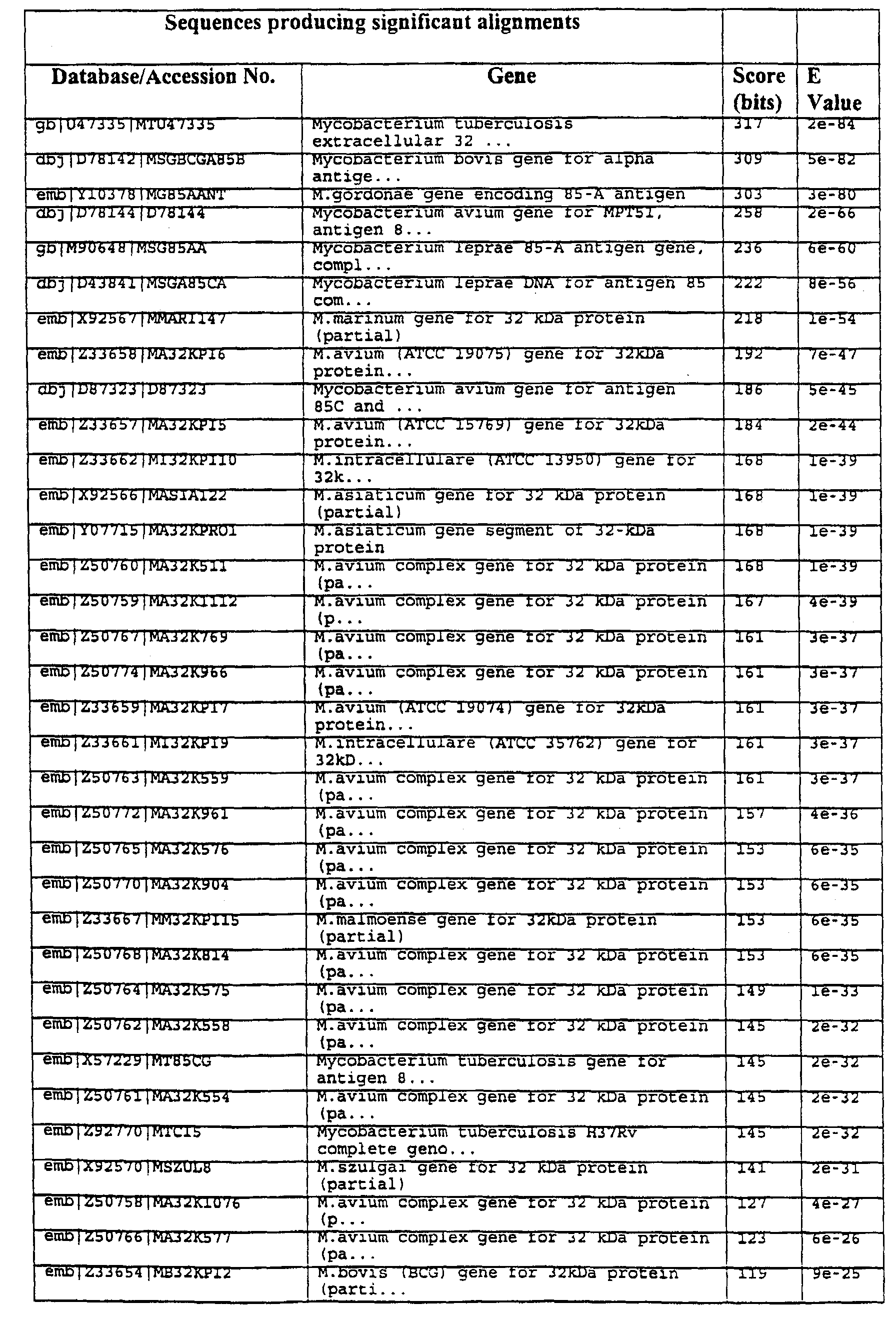

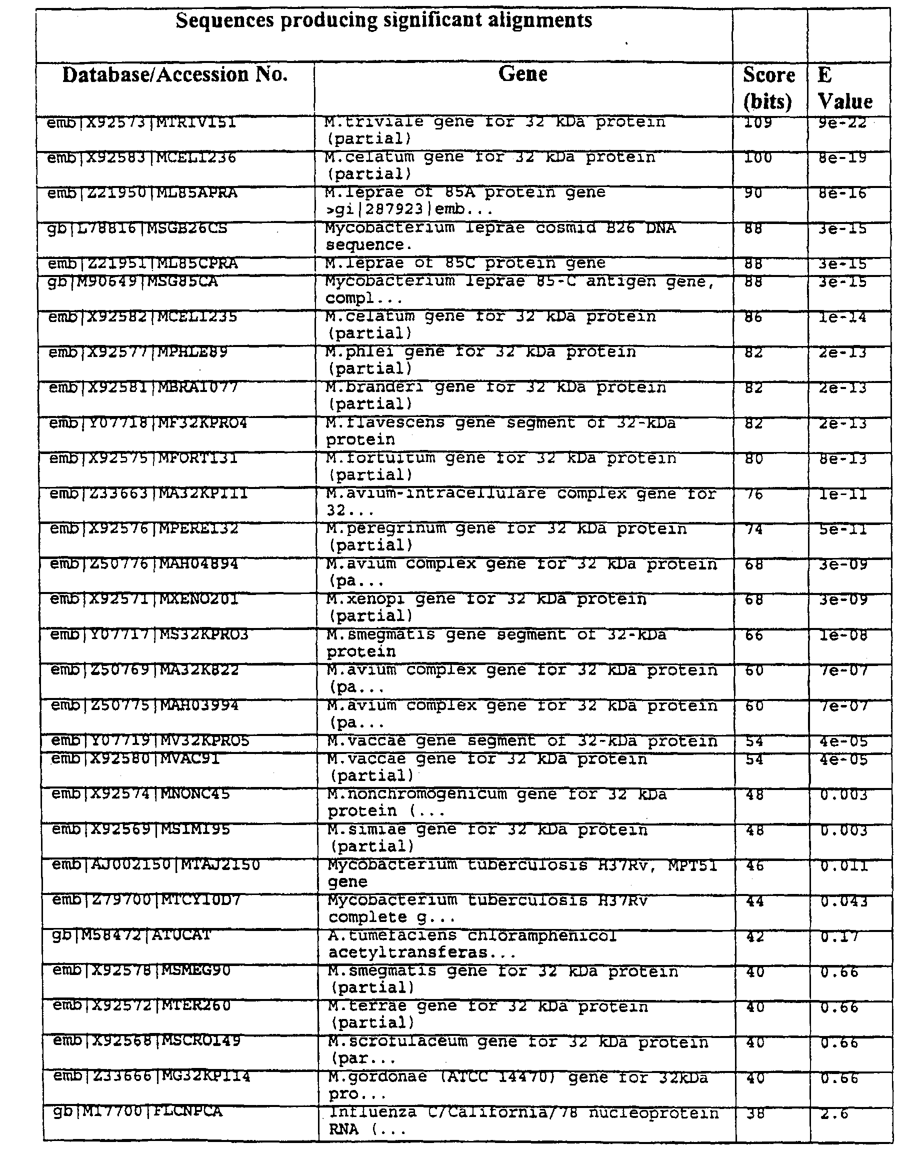

### Example 4

### Evolution of Broad-spectrum Vaccines against Helicobacter pylori

Chronic infection of the gastroduodenal mucosae by *Helicobacter pylori* bacteria is responsible for chronic active gastritis, peptic ulcers, and gastric cancers such as adenocarcinoma and low-grade B-cell lymphoma. An increasing occurrence of antibiotic-resistant strains is limiting this therapy. The use of vaccines to both prevent and treat ongoing infections is being actively pursued (Crabtree JE (1998) *Gut* 43: 7-8; Axon AT (1998) *Gut* 43 Suppl I: S70-3; Dubois *et al.* (1998) *Infect. Immun.* 66: 4340-6; Tytgat GN (1998) *Aliment. Pharmacol. Ther.* 12 Suppl 1: 123-8; Blaser MJ (1998) BMJ 316: 1507-10; Marchetti *et al.* (1998) *Vaccine* 16: 33-7; Kleanthous *et al.* (1998) *Br. Med. Bull.* 54: 229-41; Wermeille *et al.* (1998) *Pharm. World Sci.* 20: 1-17.

Identification of appropriate *Helicobacter* antigens for use in preventive and therapeutic vaccines can include two-dimensional gel electrophoresis, sequence analysis, and serum profiling *(McAtee et al.* (1998) *Clin. Diagn. Lab. Immunol.* 5:537-42; McAtee *et al.* (1998) *Helicobacter* 3: 163-9). Antigenic differences between related *Helicobacter* species and strains can limit the use of vaccines for prevention and treatment of infections (Keenan *et al.* (1998) *FEMS Microbiol Lett.* 161: 21-7).

In this Example, DNA family shuffling of related yet immunologically distinct antigens allows for the isolation of complex chimeric antigens that can provide a broad cross-reactive protection against many related strains and species of *Helicobacter.* Mouse models of persistent infection by mouse-adapted *H. pylori* strains that have been used to evaluate therapeutic use of vaccines against infection are used to evaluate shuffled antigens (Crabtree JE (1998) *Gut* 43: 7-8; Axon AT (1998) *Gut* 43 Suppl 1:S70-3).

The vacuolating cytotoxin (VacA) and cytotoxin associated gene products (CagA) have been evaluated as a vaccine against *H. pylori* infection in animal models which supports the application of this approach in humans.

Table 7 shows the results of a BLAST search using the *H. pylori* VacA gene to identify homologous genes that can be used in a family shuffling format to obtain improved antigens. Homologous antigens have been cloned and sequenced from a number of related yet distinct *H. pylori* strains and additional natural diversity can be obtained by cloning antigen genes from other strains. These genes and others or fragments thereof are cloned by methods such as PCR, shuffled and screened for improved antigens.

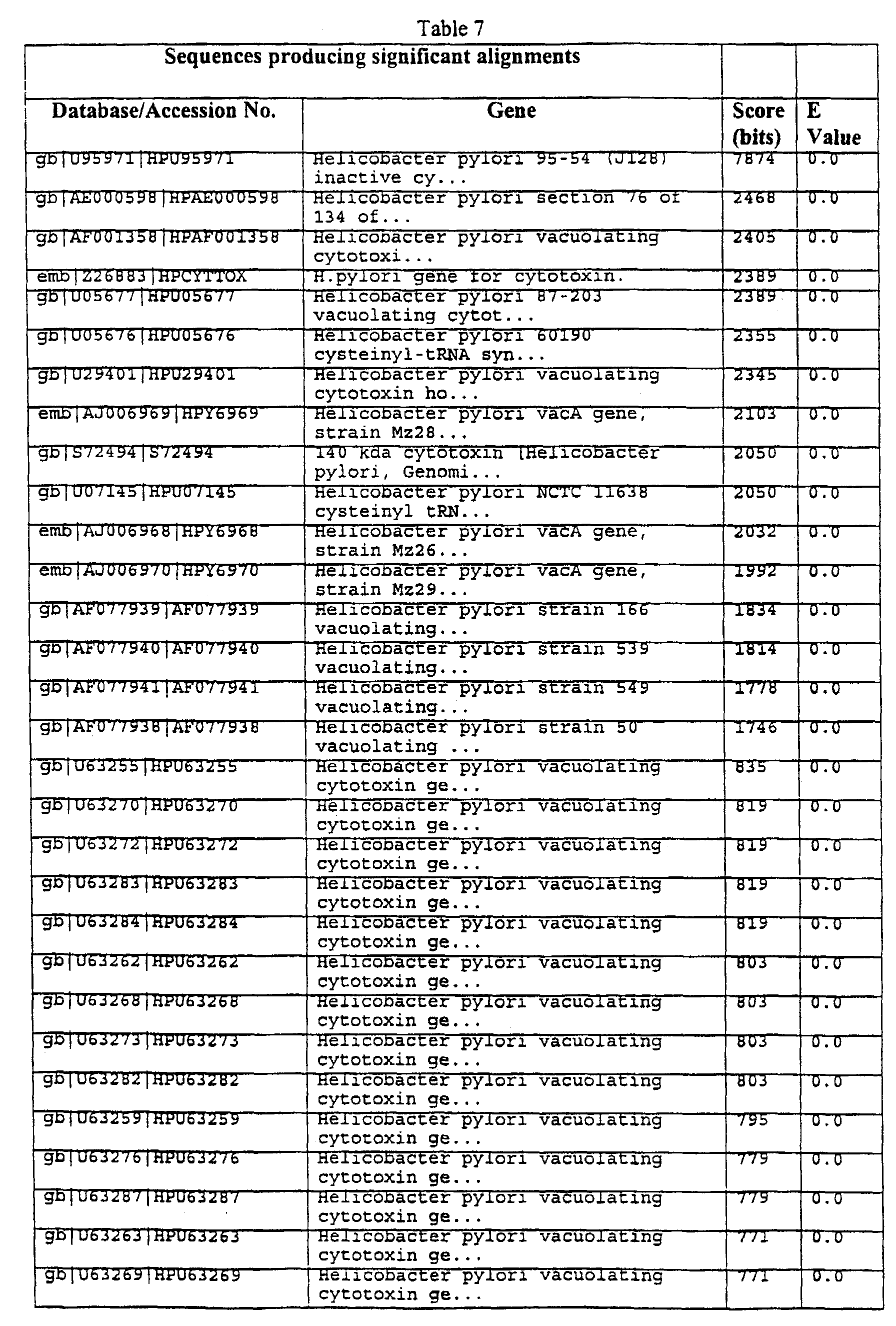

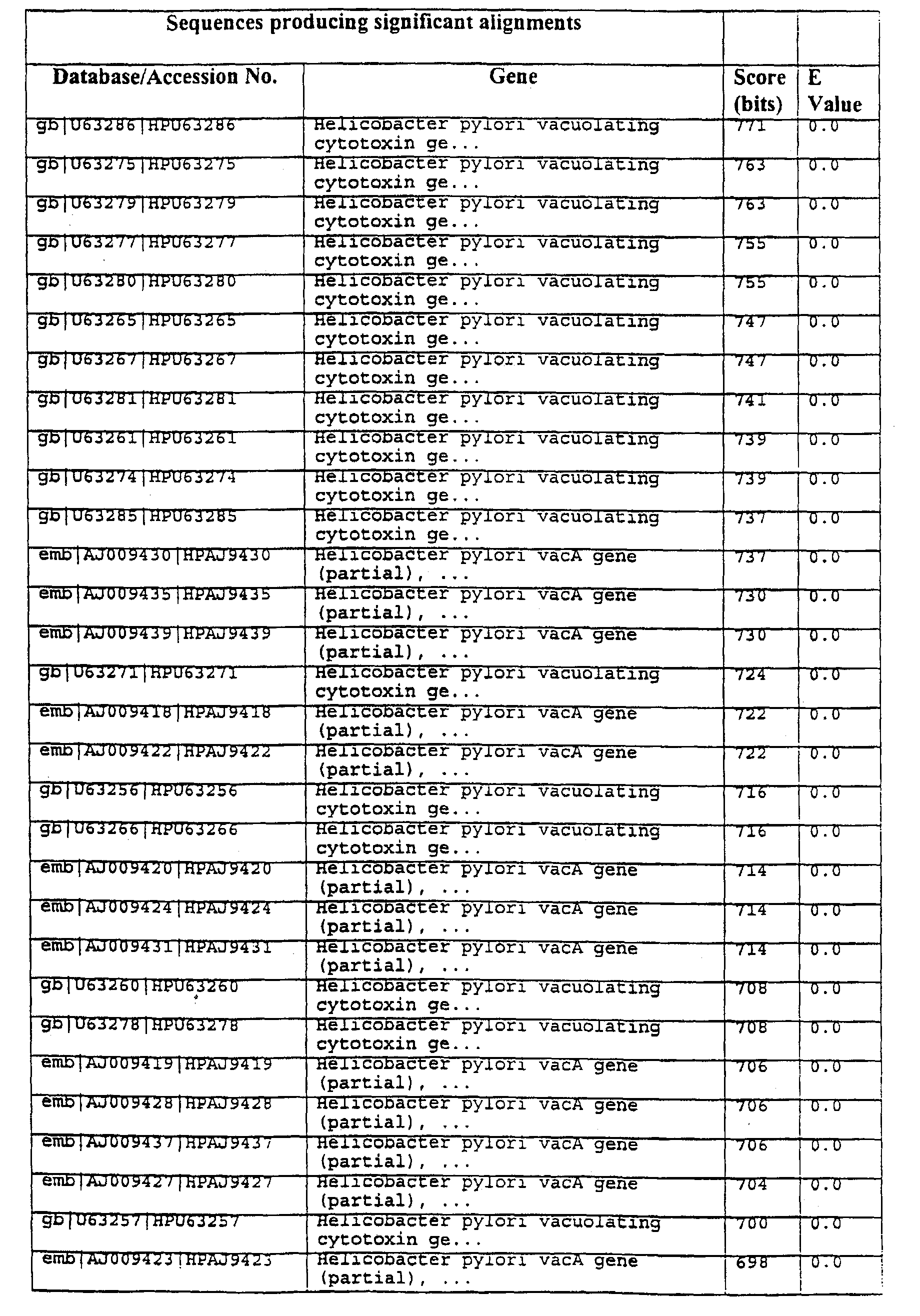

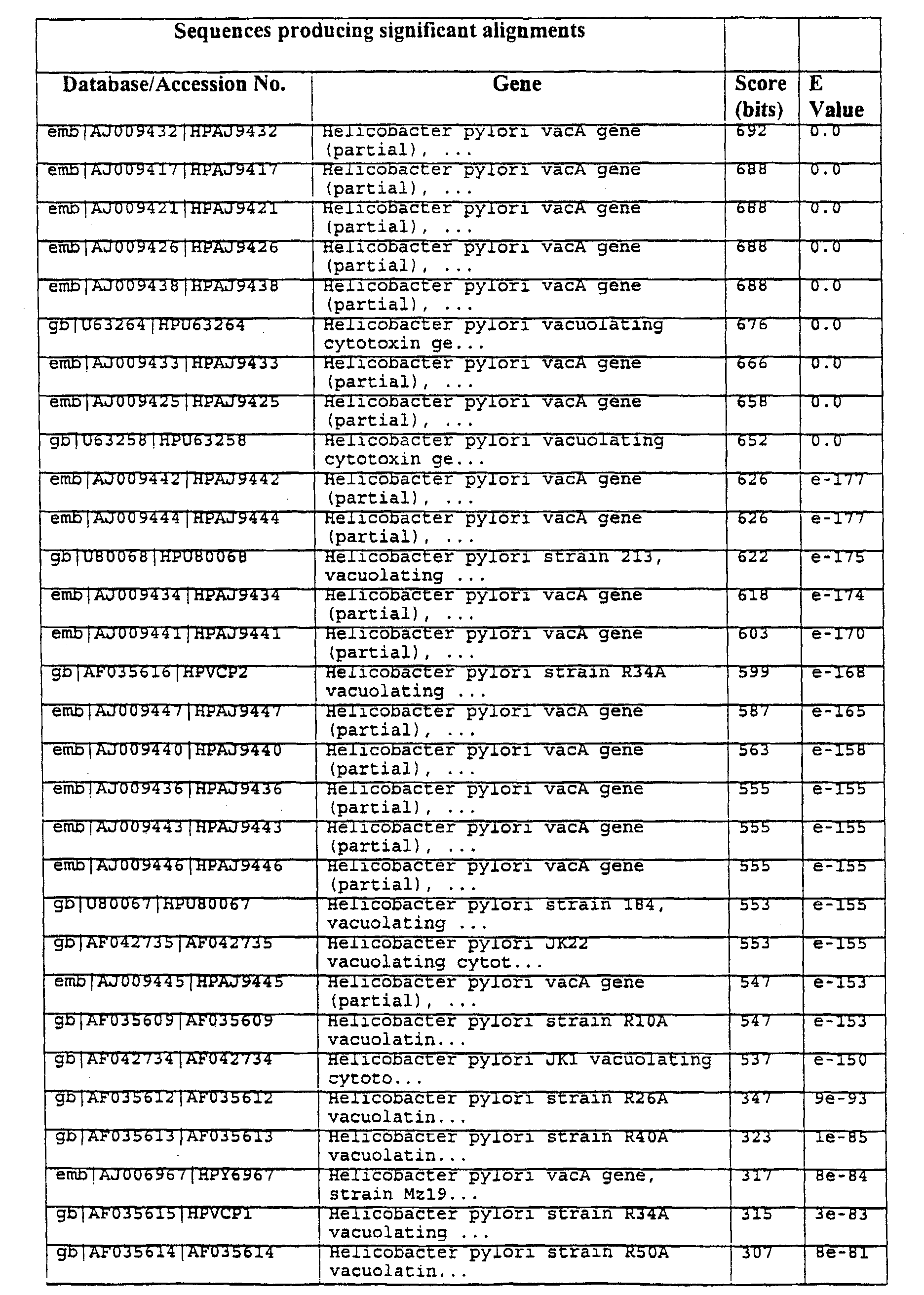

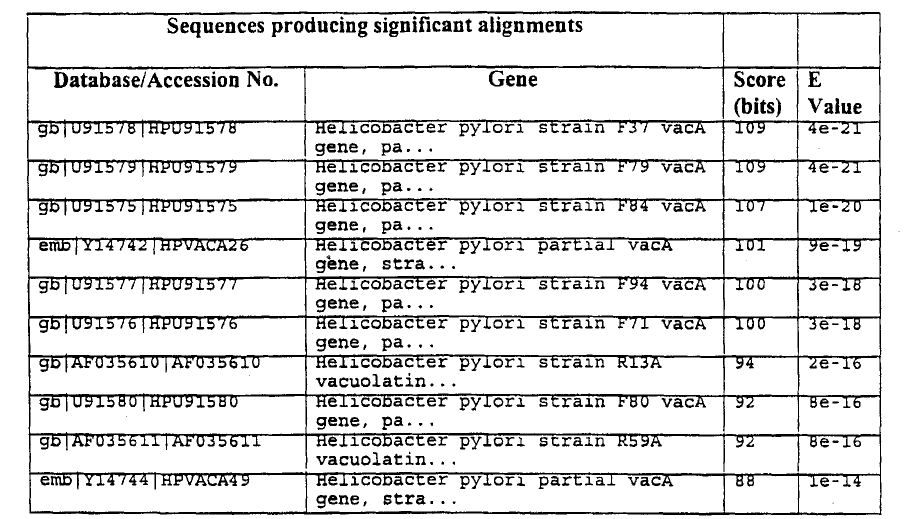

Table 8 shows the results of a BLAST search using the *H. pylori* CagA gene to identify homologous genes that can be used in a family shuffling format to obtain improved antigens. Homologous antigens have been cloned and sequenced from a number of related yet distinct *H. pylori* strains and additional natural diversity can be obtained by cloning antigen genes from other strains. These genes and others or fragments thereof are be cloned by methods such as PCR, shuffled and screened for improved antigens.

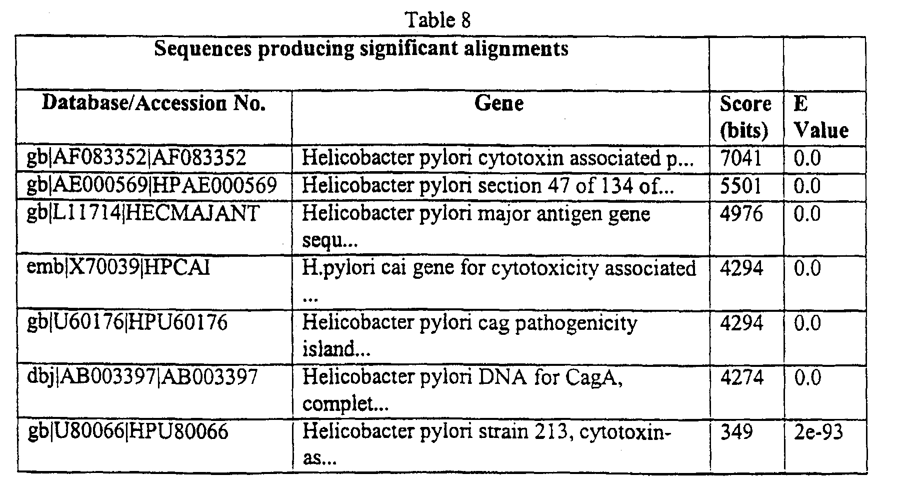

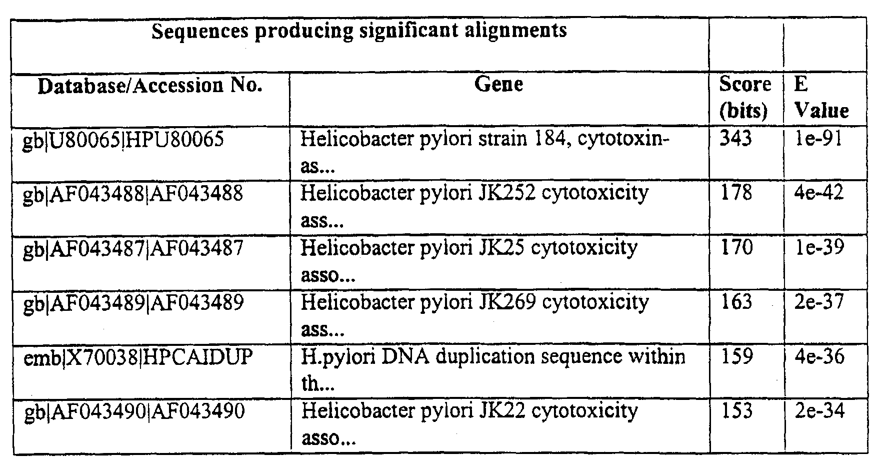

### Example 5

### Development Of Broad-Spectrum Vaccines Against Malaria

This Example describes the use of DNA shuffling to generate improved vaccines against malaria infection. An excellent target for evolution by DNA shuffling is the *Plasmodium falciparum* merozoite surface protein, MSP 1 (Hui *et al.* (1996) *Infect. Immun.* 64: 1502-1509). MSP 1 is expressed on the surface of merozoites as an integral membrane protein. It is cleaved by parasite proteases just before and concomitant with rupture and release from infected cells. The cleavage appears to be obligatory for full function in MSP 1 binding to RBC receptors. The cleaved fragments remain attached to the membrane of the merozoite. Other membrane proteins on merozoites also participate in the attachment and specific invasion events. MSP1 is a proven candidate for inclusion in a vaccine against the asexual blood stage of malaria.

The genes encoding MSP I can be isolated from various isolates of Plasmodium falciparum merozoites by PCR technology. Related naturally existing genes can be additionally used to increase the diversity of the starting genes. A library of shuffled MSP1 genes is generated by DNA shuffling, and this library is screened for induction of efficient immune responses.

The screening can be done by injecting individual variants into test animals, such as mice or monkeys. Either purified recombinant proteins, or DNA vaccines or viral vectors encoding the relevant genes are injected. Typically, a booster injection is given 2-3 weeks after the first injection. Thereafter, the sera of the test animals are collected and these sera are analyzed for the presence of antibodies that reduce invasion of merozoites into uninfected erythrocytes (RBC). RBC are infected by the merozoite, immediately inside the RBC, the merozoite differentiates into a ring and this matures to a schizont that contains several nascent daughter merozoites, which then burst out of the infected cell, destroying it, and go on to attach and invade another RBC. *In vivo,* the merozoite is likely only extracellular for seconds. *In vitro,* any blockade of this event can dramatically reduce the level of reinfection. Antibodies against MSP 1 bind to the surface of merozoites that are released from schizont infected RBC when they rupture and thereby reduce the ability of these merozoites to attach and engage cognate RBC receptors on the uninfected RBC surface. Merozoite attachment is reduced, merozoite entry into new RBC is reduced, and the numbers of newly invaded cells detected at the early ring stage is therefore reduced if the culture is examined several hours after the blockade of invasion test. In some assay formats a surrogate of merozoite invasion inhibition is to note the appearance of agglutinated merozoites, although this is an indirect measure of antibodies that cause reduced invasion.

The shuffled antigens that induce the most potent antibody responses reducing invasion of merozoites into uninfected erythrocytes are selected for further testing and can be subjected to new rounds of shuffling and selection. In subsequent studies, the capacity of these antigens to induce antibodies in man is investigated. Again, either purified recombinant antigens, or DNA vaccines or viral vectors encoding the relevant genes are injected and the protective immune responses are analyzed.

### Example 6

### Development Of Broad-Spectrum Vaccines Against Viral Pathogens

This Example describes the use of DNA shuffling to obtain vaccines that can induce an immune response against multiple isolates of viral pathogens.

### A. Venezuelan equine encephalitis virus (VEE)

VEE belongs to the alphavirus genus, which are generally transmitted by mosquitoes. However, VEE is an unusual alphavirus in that it is also highly infectious by aerosol inhalation for both humans and rodents. The disease manifestations in humans range from subclinical or mild febrile disease to serious infection and inflammation of the central nervous system. Virus clearance coincides that of production of specific anti-VEE antibodies, which are believed to be the primary mediators of protective immune responses (Schmaljohn *et al.* (1982) *Nature* 297: 70). VEE is an unusual virus also because its primary target outside the central nervous system is the lymphoid tissue, and therefore, replication defective variants may provide means to target vaccines or pharmaceutically useful proteins to the immune system.

At least seven subtypes of VEE are known that can be identified genetically and serologically. Based on epidemiological data, the virus isolates fall into two main categories: I-AB and I-C strains, which are associated with VEE epizootics/epidemics, and the remaining serotypes, which are associated primarily with enzootic vertebrate-mosquito cycles and circulate in specific ecological zones (Johnston and Peters, In *Fields Virology,* Third Edition, eds. B.N. Fields *et al.,* Lippincott-Raven Publishers, Philadelphia, 1996).

The envelope protein (E) appears to be the major antigen in inducing neutralizing Abs. Accordingly, DNA shuffling is used to obtain a library of recombinant E proteins by shuffling the corresponding genes derived from various strains of VEE. These libraries and individuals chimeras/mutants thereof are subsequently screened for their capacity to induce widely cross-reacting and protective Ab responses.

### B. Flaviviruses

Japanese encephalitis virus (JE), Tick-borne encephalitis virus (TBE) and Dengue virus are arthropod-borne viruses belonging to the Flavivirus family, which comprises 69 related viruses. The heterogeneity of the viruses within the family is a major challenge for vaccine development. For example, there are four major serotypes of Dengue virus, and a tetravalent vaccine that induces neutralizing Abs against all four serotypes is necessary. Moreover, non-neutralizing antibodies induced by infection or vaccination by one Dengue virus may cause enhancement of the disease during a subsequent infection by another serotype. Therefore, cross-protective, broad spectrum vaccines for TBE and JE would provide significant improvements to the existing vaccines. In this Example, the ability of DNA shuffling to efficiently generate chimeric and mutated genes is used to generate cross-protective vaccines.

### 1. Japanese encephalitis virus

Japanese encephalitis virus (JE) is a prototype of the JE antigenic complex, which comprises St. Louis encephalitis virus, Murray Valley encephalitis virus, Kunjin virus and West Nile virus (Monath and Heinz, In *Fields Virology,* Third Edition, eds. B.N. Fields *et al.,* Lippincott-Raven Publishers, Philadelphia, pp 961-1034, 1996). Infections caused by JE are relatively rare, but the case-fatality is 5-40% because no specific treatment is available. JE is widely distributed in China, Japan, Philippines, far-eastern Russia and India providing a significant threat to those traveling in these areas. Currently available JE vaccine is produced from brain tissues of mice infected with single virus isolate. Side effects are observed in 10% to 30% of the vaccinees.

To obtain chimeric and/or mutated antigens that provide a protective immune response against all or most of the viruses within the JE complex, DNA shuffling is performed on viral envelope genes. The amino acid identity within the JE complex varies between 72% and 93%. In addition, significant antigenic variation has been observed among JE strains by neutralization assays, agar gel diffusion, antibody absorption and monoclonal antibody analysis (Oda (1976) *Kobe J. Med. Sci.* 22: 123; Kobyashi *et al.* (1984) *Infect. Immun.* 44: 117). Moreover, the amino acid divergence of the envelope protein gene among 13 strains from different Asian countries is as much as 4.2% (Ni and Barrett (1995) *J. Gen. Virol.* 76: 401). The resulting library of recombinant polypeptides encoded by the shuffled genes is screened to identify those that provide a cross-protective immune response.

### 2. Tick-borne encephalitis virus

The tick-borne encephalitis virus complex comprises 14 antigenically related viruses, eight of which cause human disease, including Powassan, Louping ill and Tick-bome encephalitis virus (TBE) (Monath and Heinz, In *Fields Virology,* Third Edition, eds. B.N. *Fields et al.,* Lippincott-Raven Publishers, Philadelphia, pp. 961-1034, 1996). TBE has been recognized in all Central and Eastern European countries, Scandinavia and Russia, whereas Powassan occurs in Russia, Canada and the United States. The symptoms vary from flu-like illness to severe meningitis, meningoencephalitis and meningoencephalitis with a fatality rate of 1% to 2% (Gresikova and Calisher, In Monath ed., *The arboviruses: ecology and epidemiology,* vol. IV, Boca Raton, FL, CRC Press, pp. 177-203, 1988).

Family DNA shuffling is used to generate chimeric envelope proteins derived from the TBE complex to generate crossprotective antigens. The envelope proteins within the family are 77-96% homologous, and viruses can be distinguished by specific mAbs (Holzmann *et al.,* Vaccine, 10, 345, 1992). The envelope protein of Powassan is 78% identical at the amino acid level with that of TBE, and cross-protection is unlikely, although epidemiological data is limited.

Langat virus is used as a model system to analyze protective immune responses *in vivo* (Iacono-Connors *et al.* (1996) *Virus Res.* 43: 125). Langat virus belongs to the TBE complex, and can be used in challenge studies in BSL3 facilities. Serological studies based on recombinant envelope proteins are performed to identify immunogen variants that induce high levels of antibodies against envelope proteins derived from most or all viruses of the TBE complex.

### 3. Dengue viruses

Dengue viruses are transmitted though mosquito bites, posing a significant threat to troops and civilian populations particularly in tropical areas. There are four major serotypes of Dengue virus, namely Dengue 1, 2, 3 and 4. A tetravalent vaccine that induces neutralizing antibodies against all four strains of Dengue is required to avoid antibody-mediated enhancement of the disease when the individual encounters the virus of the other strain.

The envelope protein of Dengue virus has been shown to provide an immune response that protects from a future challenge with the same strain of virus. However, the levels of neutralizing antibodies produced are relatively low and protection from live virus challenge is not always observed. For example, mice injected with genetic vaccines encoding envelope protein of Dengue-2 virus developed neutralizing antibodies when analyzed *by in vitro* neutralization assays, but the mice did not survive the challenge with live Dengue-2 virus (Kochel *et al.* (1997) *Vaccine* 15: 547-552). However, protective immune responses were observed in mice immunized with recombinant vaccinia virus expressing Dengue 4 virus structural proteins (Bray *et al.* (1989) *J. Virol.* 63: 2853). These studies indicate that vaccinations with E proteins work, but significant improvements in the immunogenicity of the protective antigens are required.

In this Example, DNA shuffling is performed on the genes encoding the envelope (E) protein from all four Dengue viruses and their antigenic variants. Family DNA shuffling is used to generate chimeric E protein variants that induce high titer neutralizing antibodies against all serotypes of Dengue. The E proteins of the different dengue viruses share 62% to 77% of their amino acids. Dengue 1 and Dengue 3 are most closely related (77% homologous), followed by Dengue 2 (69%) and Dengue 4 (62%). These homologies are well in the range that allows efficient family shuffling (Crameri *et al.* (1998) *Nature* 391: 288-291 ).

The shuffled antigen sequences are incorporated into genetic vaccine vectors, the plasmids purified, and subsequently injected into mice. The sera are collected from the mice and analyzed for the presence of high levels of cross-reactive antibodies. The best antigens are selected for further studies using *in vivo* challenge models to screen for chimeras/mutants that induce cross-protection against all strains of Dengue.

### C. Improved Expression and Immunogenicity of Hantaan virus Glycoproteins

One of the advantages of genetic vaccines is that vectors expressing pathogen antigens can be generated even when the given pathogen cannot be isolated in culture. An example of such potential situation was an outbreak of severe respiratory disease among rural residents of the Southwestern United States which was caused by a previously unknown hantavirus, Sin Nombre virus (Hjelle *et al.* (1994) *J*. *Virol.* 68: 592). Much RNA sequence information of the virus was obtained well before the virus could be isolated and *characterized in vitro.* In these situations, genetic vaccines can provide means to generate efficient vaccines in a short period of time by creating vectors encoding antigens encoded by the pathogen. However, genetic vaccines can only work if these antigens can be properly expressed in the host.

Hantaan virus belongs to the Bunyavirus family. A characteristic feature of this family is that their glycoproteins typically accumulate at the membranes of the Golgi apparatus when expressed by cloned cDNAs, thereby reducing the efficacy of corresponding genetic vaccines (Matsuoka *et al.* (1991) *Curr. Top. Microb. Immunol.* 169: 161-179). Poor expression of Hantaan virus glycoproteins on the cell surface is also one explanation for poor immune responses following injections of Hantaan virus genetic vaccines.

In this Example, family DNA shuffling is used to generate recombinant Hantaan virus derived glycoproteins that are efficiently expressed in human cells and that can induce protective immune responses against the wild-type pathogen. Nucleic acids that encode the Hantaan virus glycoprotein are shuffled with genes that encode other homologous Bunyavirus glycoproteins. The resulting library is screened to identify proteins that are readily expressed in human cells. The screening is performed using a dual marker expression vector that enables simultaneous analysis of transfection efficiency and expression of fusion proteins that are PIG-linked to the cell surface (Whitehorn *et al.* (1995) *Biotechnology* (N Y) 13:1215-9).

Flow cytometry based cell sorting is used to select Hantaan virus glycoprotein variants that are efficiently expressed in mammalian cells. The corresponding sequences are then obtained by PCR or plasmid recovery. These chimeras/mutants are further analyzed for their capacity to protect wild mice against Hantaan virus infections.

### Example 7

### DNA Shuffling Of HSV-1 And HSV-2 Glycoproteins B And/Or D As Means To Induce Enhanced Protective Immune Responses

This Example describes the use of DNA shuffling to obtain HSV glycoprotein B (gB) and glycoprotein D (gD) polypeptides that exhibit improved ability to induce protective immune responses upon administration to a mammal. Epidemiological studies have shown that prior infections with HSV-1 give partial protection against infections with HSV-2, indicating existence of cross-reactive immune responses. Based on previous vaccination studies, the main immunogenic glycoproteins in HSV appear to be gB and gD, which are encoded by 2.7 kb and 1.2 kb genes, respectively. The gB and gD genes of HSV-1 are about 85% identical to the corresponding gene of HSV-2, and the gB genes of each share little sequence identity with the gD genes. Baboon HSV-2 gB is appr. 75% identical to human HSV-1 or -2 gB, with rather long stretches of almost 90% identity. In addition, 60-75% identity is found in portions of the genes of equine and bovine herpesviruses.

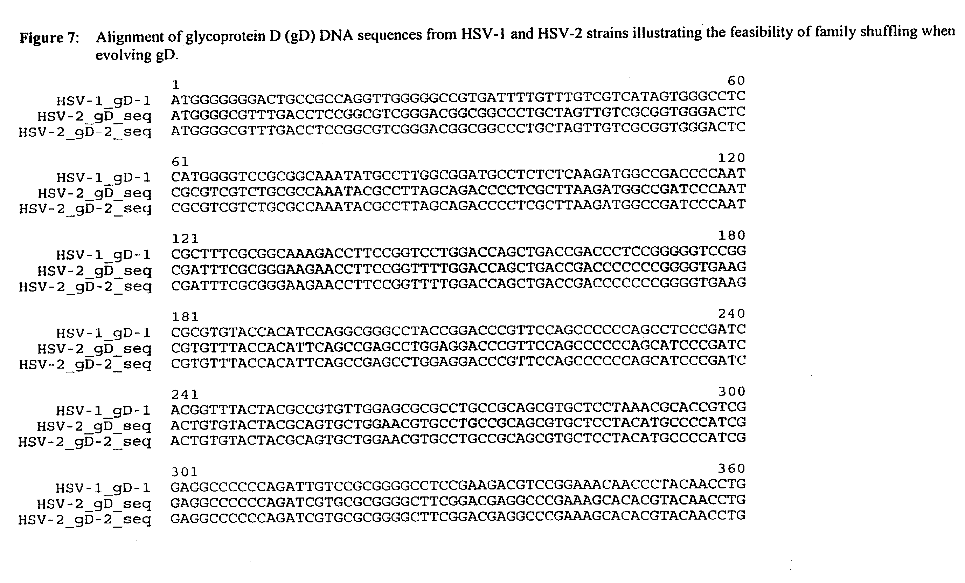

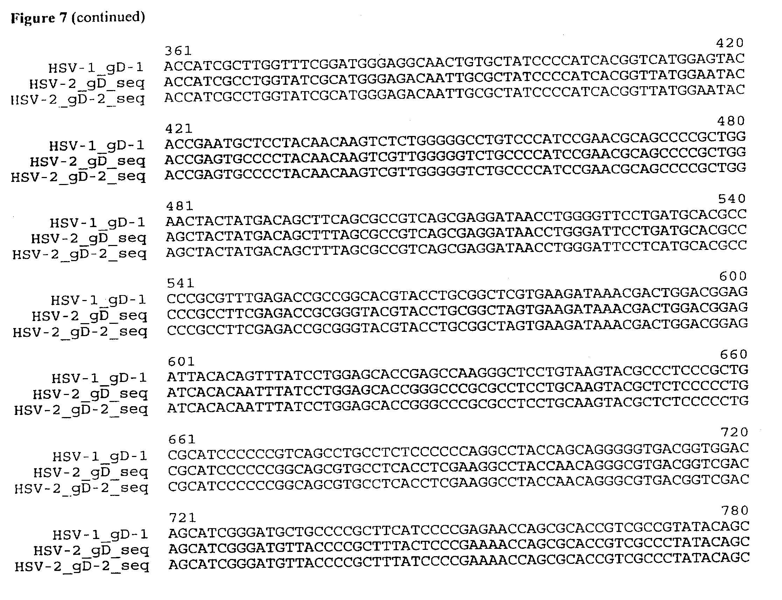

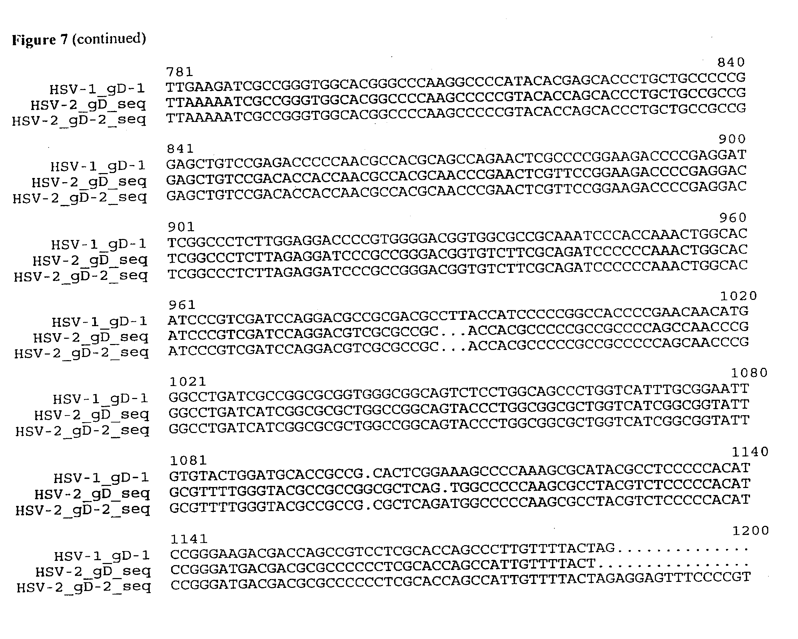

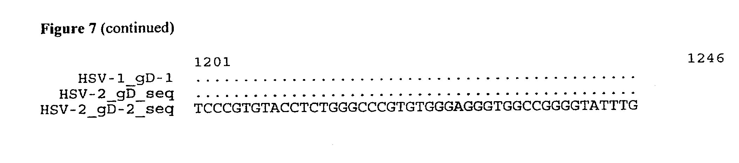

Family shuffling is employed using as substrates nucleic acids that encode gB and/or gD from HSV-1 and HSV-2. Preferably, homologous genes are obtained from HSVs of various strains. An alignment of gD nucleotide sequences from HSV-1 and two strains of HSV-2 is shown in Figure 7. Antigens encoded by the shuffled nucleic acids are expressed and analyzed *in vivo.* For example, one can screen for improved induction of neutralizing antibodies and/or CTL responses against HSV-1/HSV-2. One can also detect protective immunity by challenging mice or guinea pigs with the viruses. Screening can be done using pools or individuals clones.

### Example 8

### Evolution Of HIV Gp120 Proteins For Induction Of Broad Spectrum Neutralizing Ab Responses

This Example describes the use of DNA shuffling to generate immunogens that crossreact among different strains of viruses, unlike the wild-type immunogens. Shuffling two kinds of envelope sequences can generate immunogens that induce neutralizing antibodies against a third strain.

Antibody-mediated neutralization of HIV-1 is strictly type-specific. Although neutralizing activity broadens in infected individuals over time, induction of such antibodies by vaccination has been shown to be extremely difficult. Antibody-mediated protection from HIV-1 infection in vivo correlates with antibody-mediated neutralization of virus *in vitro.*

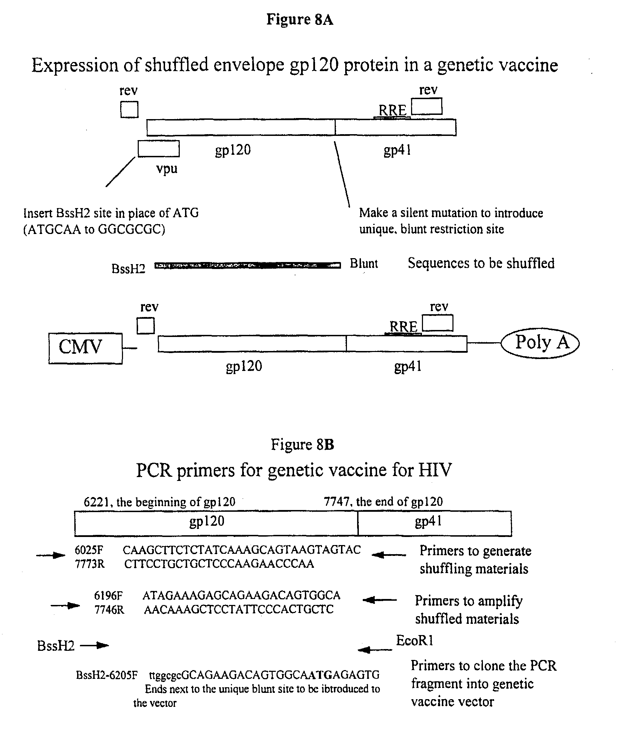

Figure 8 illustrates the generation of libraries of shuffled gp120 genes. gp120 genes derived from HIV-1DH12 and HIV-IIIIB(NL43) are shuffled. The chimeric/mutant gp 120 genes are then analyzed for their capacity to induce antibodies that have broad spectrum capacity to neutralize different strains of HIV. Individual shuffled gp120 genes are incorporated into genetic vaccine vectors, which are then introduced to mice by injection or topical application onto the skin. These antigens can also be delivered as purified recombinant proteins. The immune responses are measured by analyzing the capacity of the mouse sera to neutralize HIV growth *in vitro.* Neutralization assays are performed against HIV-1DH12, HIV-1IIIB and HIV-189.6. The chimeras/mutants that demonstrate broad spectrum neutralization are chosen for further rounds of shuffling and selection. Additional studies are performed in monkeys to illustrate the capacity of the shuffled gp120 genes to provide protection for subsequent infection with immunodeficiency virus.

### Example 9

### Antigen Shuttling of the Hepadnavirus Envelope Protein

The Hepatitis B virus (HBV) is one of a member of a family of viruses called hepadnaviruses. This Example describes the use of genomes and individual genes from this family are used for DNA shuffling, which results in antigens having improved properties.

### A. Shuffling of Hepadnavirus envelope protein genes

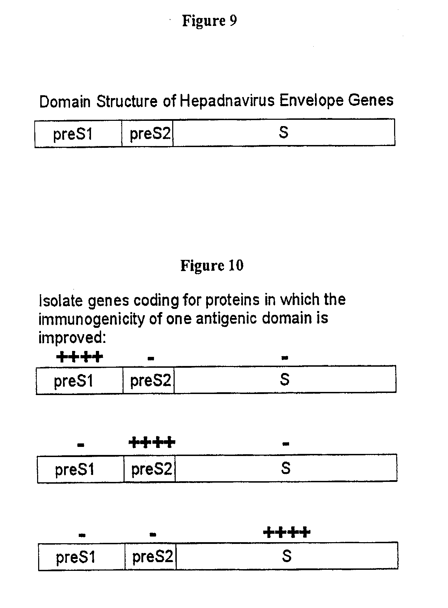

The envelope protein of the HBV assembles to form particles that carry the antigenic structures collectively known as the Hepatitis B surface antigen (HBsAg; this term is also used to designate the protein itself). Antibodies to the major antigenic site, designated the "a" epitope (which is found in the envelope domain called S), are capable of neutralizing the virus. Immunization with the HBsAg-bearing protein thus serves as a vaccine against viral infection. The HBV envelope also contains other antigenic sites that can protect against viral infection and are potentially vital components of an improved vaccine. The epitopes are part of the envelope protein domains known as preS1 and preS2 (Figure 9).

DNA shuffling of the envelope gene from several members of the hepadnavirus family is used to obtain more immunogenic proteins. Specifically, the genes from the following hepatitis viruses are shuffled:
- the human HBV viruses, subtypes *ayw* and *adw2*
- a hepatitis virus isolated from chimpanzee
- a hepatitis virus isolated from gibbon
- a hepatitis virus isolated from woodchuck

If desired, genes from other genotypes of the human virus are available for inclusion in the DNA shuffling reactions. Likewise, other animal hepadnaviruses are available.

To promote the efficiency of the formation of chimeras resulting from DNA shuffling, some artificial genes are made:
- In one case, a synthetic gene is made that contains the HBV envelope sequences, except for those codons which specify amino acids found in the chimpanzee and gibbon genes. For those codons, the chimpanzee or gibbon sequence is used.
- In a second case, a synthetic gene is synthesized in which the preS2 gene sequence from the human HBV *adw2* strain is fused with the woodchuck S region.
- In a third case, all the oligonucleotides required to chemically synthesize each of the hepadnavirus envelope genes are mixed in approximately equal quantities and allowed to anneal to form a library of sequences.

After DNA shuffling of the hepadnavirus envelope genes, either or both of two strategies are used to obtain improved HBsAg antigens.

Strategy A: Antigens are screened by immunizing mice using two possible methods. The genes are injected in the form of DNA vaccines, i.e., shuffled envelope genes carried by a plasmid that comprises the genetic regulatory elements required for expression of the envelope proteins. Alternatively, the protein is prepared from the shuffled genes and used as the immunogen.

The sequences that give rise to greater immunogenicity for either the preS1-, preS2- or S-bome HBV antigens are selected for a second round of shuffling (Figure 10). For the second round, the best candidates are chosen based on their improved antigenicity and their other properties such as higher expression level or more efficient secretion. Screening and further rounds of shuffling are continued until a maximum optimization for one of the antigenic regions is obtained.

The individually optimized genes are then used as a combination vaccine for the induction of optimal responses to preS1-, preS2- and S-bom epitopes.

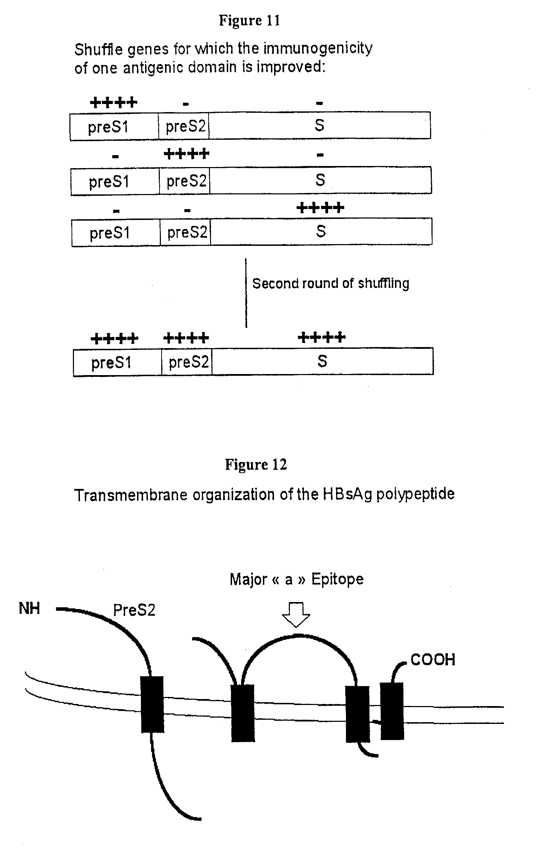

Strategy B: After isolation of the individually optimized genes as in Strategy A, the preS1, preS2 and S candidates are shuffled together, or in a pairwise fashion, in further rounds to obtain genes which encode proteins that demonstrate improved immunogenicity for at least two regions containing HBsAg epitopes (Figure 11).

### B. Use of HBsAg to carry epitopes from unrelated antigens

Several of the characteristics of the HBsAg make it a useful protein to carry epitopes drawn from other, unrelated antigens. The epitopes can be either B epitopes (which induce antibodies) or T epitopes drawn from the class I type (which stimulate CD8⁺ T lymphocytes and induced cytotoxic cells) or class II type (which induce helper T lymphocytes and are important in providing immunological memory responses.

### 1. B cell epitopes

Amino acid sequences of potential B epitopes are chosen from any pathogen. Such sequences are often known to induce antibodies, but the immunogenicity is weak or otherwise unsatisfactory for preparation of a vaccine. These sequences can also be mimotopes, which have been selected based on their ability to have a certain antigenicity or immunogenicity.

The amino acid coding sequences are added to a hepadnavirus envelope gene. The heterologous sequences can either replace certain envelope sequences, or be added in addition to all the envelope sequences. The heterologous epitope sequences can be placed at any position in the envelope gene. A preferred position is the region of the envelope gene that encodes the major "a" epitope of the HBsAg (Figure 12). This region is likely to be exposed on the external side of the particles formed by the envelope protein, and thus will expose the heterologous epitopes.

DNA shuffling is carried out on the envelope gene sequences, keeping the sequence of the heterologous epitopes constant. Screening is carried out to choose candidates that are secreted into the culture medium after transfection of plasmids from the shuffled library into cells in tissue culture.

Clones that encode a secreted protein are then tested for immunogenicity in mice either as a DNA vaccine or as a protein antigen, as described above. Clones that give an improved induction of antibodies to the heterologous epitopes are chosen for further rounds of DNA shuffling. The process is continued until the immunogenicity of the heterologous epitope is sufficient for use as a vaccine against the pathogen from which the heterologous epitopes were derived.

### 2. Class I epitopes

MHC Class I epitopes are relatively short, linear peptide sequences that are generally between 6 and 12 amino acids amino acids in length, most often 9 amino acids in length. These epitopes are processed by antigen-presenting cells either after synthesis of the epitope within the cell (usually as part of a larger protein) or after uptake of soluble protein by the cells.

Polynucleotide sequences that encode one or more class I epitopes are inserted into the sequence of a hepadnavirus envelope gene either by replacing certain envelope sequences, or by inserting the epitope sequences into the envelope gene. This is typically done by modifying the gene before DNA shuffling or by including in the shuffling reaction certain oligonucleotide fragments that encode the heterologous epitopes as well as sufficient flanking hepadnavirus sequences to be incorporated into the shuffled products.

Preferably, the heterologous class I epitopes are placed into different positions in the several hepadnavirus genes used for the DNA shuffling reaction. This will optimize the chances for finding chimeric gene carrying the epitopes in an optimal position for efficient presentation.

### 3. Class II epitopes

MHC Class II epitopes are generally required to be part of a protein which is taken up by antigen presenting cells, rather than synthesized within the cell. Preferably, such epitopes are incorporated into a carrier protein such as the HBV envelope that can be produced in a soluble form or which can be secreted if the gene is delivered in the form of a DNA vaccine.

Polynucleotides that encode heterologous class II epitopes are inserted into regions of the hepadnavirus envelope genes that are not involved in the transmembrane structure of the protein. DNA shuffling is performed to obtain a secreted protein that also carries the class II epitopes. When injected as a protein, or when the gene is delivered as a DNA vaccine, the protein can be taken up by antigen presenting cells for processing of the class II epitopes.

### Example 10

### Evolution of broad spectrum vaccines against Hepatitis C Virus.

Antigenic heterogeneity of different strains of Hepatitis C Virus (HCV) is a major problem in development of efficient vaccines against HCV. Antibodies or CTLs specific for one strain of HCV typically do not protects against other strains. Multivalent vaccine antigens that simultaneously protect against several strains of HCV would be of major importance when developing efficient vaccines against HCV.

The HCV envelope genes, which encode envelope proteins E1 and E2, have been shown to induce both antibody and lymphoproliferative responses against these antigens (Lee *et al.* (1998) *J. Virol.* 72: 8430-6), and these responses can be optimized by DNA shuffling. The hypervariable region 1 (HVR1) of the envelope protein E2 of HCV is the most variable antigenic fragment in the whole viral genome and is primarily responsible for the large inter- and intra-individual heterogeneity of the infecting virus (Puntoriero *et al.* (1998) *EMBO J.* 17: 3521-33). Therefore, the gene encoding E2 is a particularly useful target for evolution by DNA shuffling.

DNA shuffling of HCV antigens, such as nucleocapsid or envelope proteins E1, E2, provides a means to generate multivalent HCV vaccines that simultaneously protect against several strains of HCV. These antigens are shuffled using the family DNA shuffling approach. The starting genes will be obtained from various natural isolates of HCV. In addition, related genes from other viruses can be used to increase the number of different recombinants that are generated. A library of related, chimeric variants of HCV antigens are then generated and this library will be screened for induction of widely crossreactive immune responses. The screening can be done directly *in vivo* by injecting individual variants into test animals, such as mice or monkeys. Either purified recombinant proteins or DNA vaccines encoding the relevant genes are injected. Typically, a booster injection is given 2-3 weeks after the first injection. Thereafter, the sera of the test animals are collected and these sera are tested for the presence of antibodies that react against multiple HCV virus isolates.

Before the *in vivo* testing is initiated, the antigens can be pre-enriched *in vitro* for antigens that are recognized by polyclonal antisera derived from previously infected patients or test animals. Alternatively, monoclonal antibodies that are specific for various strains of HCV are used. The screening is performed using phage display or ELISA assays. For example, the antigen variants are expressed on bacteriophage M13 and the phage are then incubated on plates coated with antisera derived from patients or test animals infected with various HCV isolates. The phage that bind to the antibodies are then eluted and further analyzed in test animals for induction of crossreactive antibodies.

### Example 11

### Evolution of chimeric allergens that induce broad immune responses and have reduced risk of inducing anaphylactic reactions

Specific immunotherapy of allergy is performed by injecting increasing amounts of the given allergens into the patients. The therapy typically alters the types of allergen-specific immune responses from a dominating T helper 2 (T_{H}2) type response to a dominating T helper 1 (T_{H}1 ) type response. However, because allergic patients have increased levels of IgE antibodies specific for the allergens, the immunotherapy of allergy involves a risk of IgE receptor mediated anaphylactic reactions.

T helper (T_{H}) cells are capable of producing a large number of different cytokines, and based on their cytokine synthesis pattern T_{H} cells are divided into two subsets (Paul and Seder (1994) *Cell* 76: 241-251). T_{H}1 cells produce high levels of IL-2 and IFN-gamma and no or minimal levels of IL-4, IL-5 and IL-13. In contrast, T_{H}2 cells produce high levels of IL-4, IL-5 and IL-13, whereas IL-2 and IFN-gamma production is minimal or absent. T_{H}1 cells activate macrophages, dendritic cells and augment the cytolytic activity of CD8+ cytotoxic T lymphocytes and NK cells *(Id.),* whereas T_{H}2 cells provide efficient help for B cells and they also mediate allergic responses due to the capacity of T_{H}2 cells to induce IgE isotype switching and differentiation of B cells into IgE secreting cell (De Vries and Punnonen (1996) In *Cytokine regulation of humoral immunity: basic and clinical aspects.* Eds. Snapper, C.M., John Wiley & Sons, Ltd., West Sussex, UK, pp. 195-215

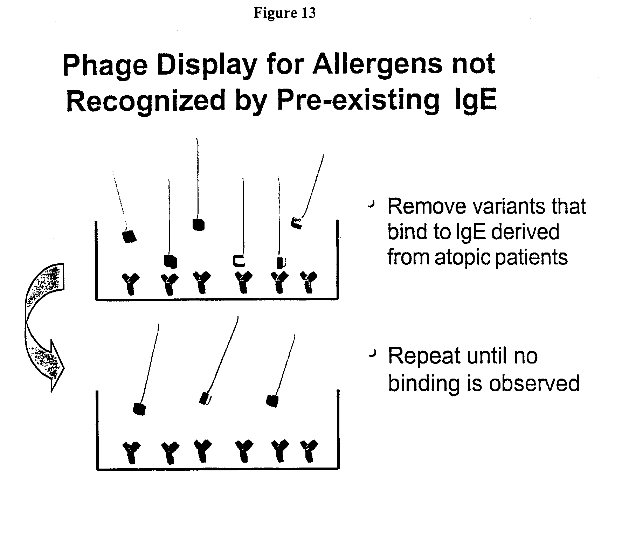

This Example describes methods to generate chimeric allergens that can broadly modulate allergic immune responses. This can be achieved by DNA shuffling of related allergen genes to generate chimeric genes. In addition, chimeric/mutated allergens are less likely to be recognized by preexisting IgE antibodies of the patients. Importantly, allergen variants that are not recognized by IgE antibodies can be selected using patient sera and negative selection (Figure 13).

As one example, chimeric allergen variants of Der p2, Der f2, Tyr p2 Lep d2 and Gly d2 allergens are generated. These house dust mite allergens are very common in exacerbating allergic and asthmatic symptoms, and improved means to downregulate such allergic immune responses are desired. House dust mites can be used as sources of the genes. The corresponding genes are shuffled using family DNA shuffling and a shuffled library is generated. Phage display is used to exclude allergens that are recognized by antibodies from allergic individuals. It is particularly important is to exclude variants that are recognized by IgE antibodies. Phage expressing the allergen variants are incubated with pools of sera derived from allergic individuals. The phage that are recognized by IgE antibodies are removed, and the remaining allergens are further tested *in vitro* and *in vivo* for their capacity to activate allergen-specific human T cells (Figure 14). Because immunotherapy of allergy is believed to function through induction of a dominating T_{H}1 response as compared to allergic T_{H}2 response, efficient T cell activation and induction of a T_{H}1 type response by allergen variants is used as a measure of the efficacy of the allergens to modulate allergic T cell responses.

The optimal allergen variants are then further tested *in vivo* by studying skin responses after injections to the skin. A strong inflammatory response around the injection site is an indication of efficient T cell activation, and the allergen variants that induce the most efficient delayed type T cell response (typically observed 24 hours after the injection) are the best candidates for further studies *in vivo* to identify allergens that effectively downregulate allergic immune responses. Accordingly, these allergen variants re analyzed for their capacity to inhibit allergic responses in allergic, atopic and asthmatic individuals. The screening of allergen variants is further illustrated in Figure 13 and Figure 14.

### Example 12

### Evolution of cancer antigens that induce efficient anti-tumor immune responses

Several cancer cells express antigens that are present at significantly higher levels on the malignant cells than on other cells in the body. Such antigens provide excellent targets for preventive cancer vaccines and immunotherapy of cancer. The immunogenicity of such antigens can be improved by DNA shuffling. In addition, DNA shuffling provides means to improve expression levels of cancer antigens.

This Example describes methods to generate cancer antigens that can efficiently induce anti-tumor immune responses by DNA shuffling of related cancer antigen genes. Libraries of shuffled melanoma-associated glycoprotein (gp100/pmel17) genes (Huang *et al.* (1998) *J. Invest. Dermatol.* 111: 662-7) are generated. The genes can be isolated from melanoma cells obtained from various patients, who may have mutations of the gene, increasing the diversity in the starting genes. In addition, a gp100 gene can be isolated from other mammalian species to further increase the diversity of starting genes. A typical method for the isolation of the genes is RT-PCR. The corresponding genes are shuffled using single gene DNA shuffling or family DNA shuffling and a shuffled library is generated.

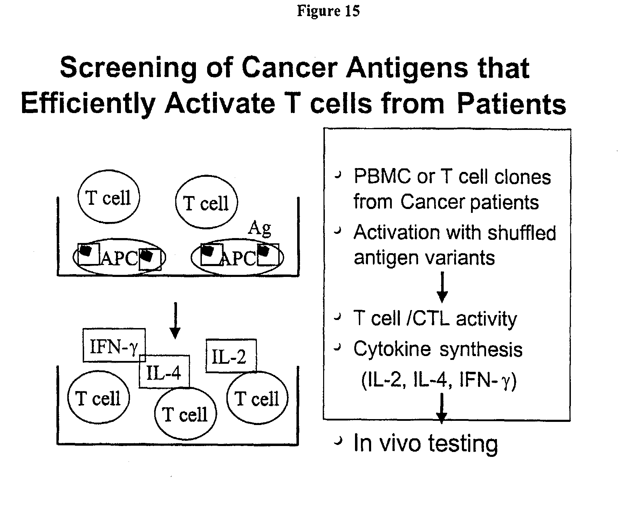

The shuffled gp 100 variants, either pools or individual clones, are subsequently injected into test animals, and the immune responses are studied (Figure 15). The shuffled antigens are either expressed in *E. coli* and recombinant, purified proteins are injected, or the antigen genes are used as components of DNA vaccines. The immune response can be analyzed for example by measuring anti-gp100 antibodies, as previous studies indicate that the antigen can induce specific antibody responses (Huang *et al., supra*.). Alternatively, the test animals that can be challenged by malignant cells expressing gp100. Animals that have been efficiently immunized will generate cytotoxic T cells specific for gp100 and will survive the challenge, whereas in non-immunized or poorly immunized animals the malignant cells will efficiently grow eventually resulting in lethal expansion of the cells. Furthermore, antigens that induce cytotoxic T cells that have the capacity to kill cancer cells can be identified by measuring the capacity of T cells derived from immunized animals to kill cancer cells *in vitro.* Typically the cancer cells are first labeled with radioactive isotopes and the release of radioactivity is an indication of tumor cell killing after incubation in the presence of T cells from immunized animals. Such cytotoxicity assays are known in the art.

The antigens that induce highest levels of specific antibodies and/or can protect against the highest number of malignant cells can be chosen for additional rounds of shuffling and screening. Mice are useful test animals because large numbers of antigens can be studied. However, monkeys are a preferred test animal, because the MHC molecules of monkeys are very similar to those of humans.

To screen for antigens that have optimal capacity to activate antigen-specific T cells, peripheral blood mononuclear cells from previously infected or immunized humans individuals can be used. This is a particularly useful method, because the MHC molecules that will present the antigenic peptides are human MHC molecules. Shuffled cancer antigens that induce cytotoxic T cells that have the capacity to kill cancer cells can be identified by measuring the capacity of T cells derived from immunized animals to kill cancer cells *in vitro.* Typically the cancer cells are first labeled with radioactive isotopes and the release of radioactivity is an indication of tumor cell killing after incubation in the presence of T cells from immunized animals. Such cytotoxicity assays are known in the art.

### Example 13

### Evolution of autoantigens that induce efficient immune responses

Autoimmune diseases are characterized by an immune response directed against self antigens expressed by the host. Autoimmune responses are generally mediated by T_{H}1 cells that produce high levels of IL-2 and IFN-gamma. Vaccines that can direct autoantigen specific T cells towards T_{H}2 phenotype producing increased levels of IL-4 and IL-5 would be beneficial. For such vaccines to work, the vaccine antigens have to be able to efficiently activate specific T cells. DNA shuffling can be used to generate antigens that have such properties. To optimally induce T_{H}2 cell differentiation it may be beneficial to coadminister cytokines that have been shown to enhance T_{H}2 cell activation and differentiation, such as IL-4 (Racke *et al.* (1994) *J. Exp. Med.* 180: 1961-66).

This Example describes methods for generating autoantigens that can efficiently induce immune responses. DNA shuffling is performed on related autoantigen genes. For example, libraries of shuffled myelin basic proteins, or fragments thereof (Zamvil and Steinman (1990) *Ann. Rev. Immunol.* 8: 579-621); Brocke *et al.* (1996) *Nature* 379: 343-46) are generated. MBP is considered to be an important autoantigen in patients with multiple sclerosis (MS). The genes encoding MBP from at least bovine, mouse, rat, guinea pig and human have been isolated providing an excellent starting point for family shuffling. A typical method for the isolation of the genes is RT-PCR. The shuffled MBP variants, either pools or individual clones, are subsequently injected into test animals, and the immune responses are studied. The shuffled antigens are either expressed in *E*. *coli* and recombinant, purified proteins are injected, or the antigen genes are used as components of DNA vaccines or viral vectors. The immune response can be analyzed for example by measuring anti-MBP antibodies by ELISA. Alternatively, the lymphocytes derived from immunized test animals are activated with MBP, and the T cell proliferation or cytokine synthesis is studies. A sensitive assays for cytokine synthesis is ELISPOT (McCutcheon *et al.* (1997) *J. Immunol. Methods* 210: 149-66). Mice are useful test animals because large numbers of antigens can be studied. However, monkeys are a preferred test animal, because the MHC molecules of monkeys are very similar to those of humans.

To screen for antigens that have optimal capacity to activate MBP specific T cells peripheral blood mononuclear cells from patients with MS can also be used. This is a particularly useful method, because the MHC molecules that will present the antigenic peptides are human MHC molecules. Shuffled antigens that activate MBP specific T cells can be identified by measuring the capacity of T cells derived from MS patients to proliferate or produce cytokines upon culture in the presence of the antigen variants. Such assays are known in the art. One such assay is ELISPOT (McCutcheon *et al., supra.*). An indication of the efficacy of an MBP variant to activate specific T cells is also the degree of skin inflammation when the antigen is injected into the skin of a patient with MS. Strong inflammation is correlated with strong activation of antigen-specific T cells. Improved activation of MBP specific T cells, particularly in the presence of IL-4, is likely to result in enhanced T_{H}2 cell responses, which are beneficial in the treatment of MS patients.

### Example 14

### Method of Optimizing the Immunogenicity of Hepatitis B Surface Antigen

This Example describes methods by which the envelope protein sequence of the hepatitis B virus can be evolved to provide a more immunogenic surface antigen. Such a protein is important for vaccination of low responders and for immunotherapy of chronic hepatitis B.

### Background

Current HBV vaccines (Merck, SKB) are based on the immunogenicity of the viral envelope protein and contain the Major (or Small) form of the envelope protein produced as particles in yeast. These particles induce antibodies to the major surface antigen (HBsAg) which can protect against infection when antibody levels are at least 10 milli-International Units per milliliter (mU/ml). These recombinant protein preparations are not capable of inducing humoral immunity in chronic carriers (some 300 million cases worldwide) the induction of which would be important to control virus spread. Moreover, certain individuals respond poorly to the vaccine (up to 30-50% of vaccinees in some groups) and do not develop protective levels of antibody. The inclusion of the natural epitope sequences contained in the Middle or Large forms of the viral envelope protein has been used as a method to increase the immunogenicity of vaccine preparations. An alternative method is to introduce new (*i.e.*, not present in the natural virus sequence) helper T-cell epitopes into the HBsAg sequence using DNA shuffling technology.

### Method

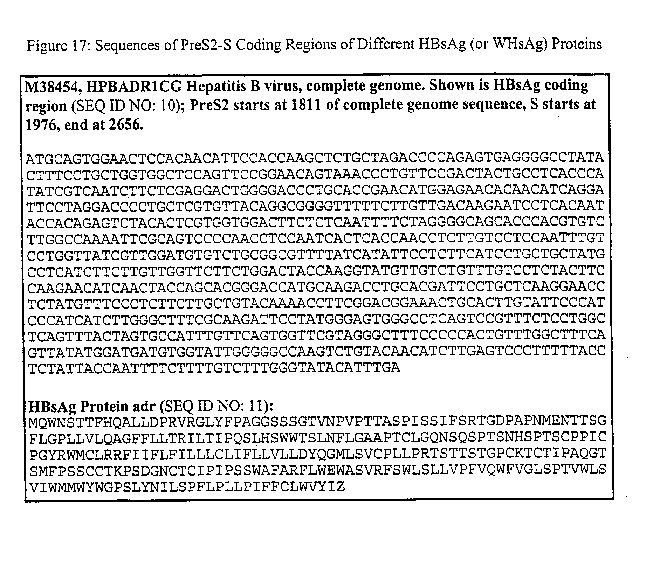

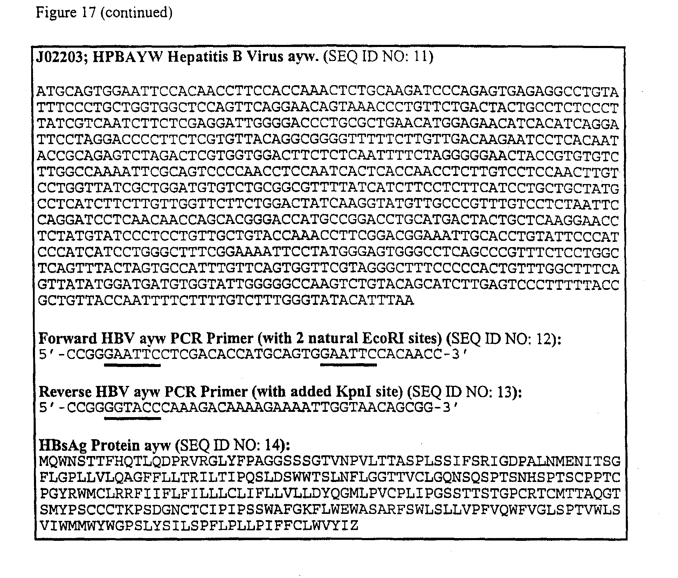

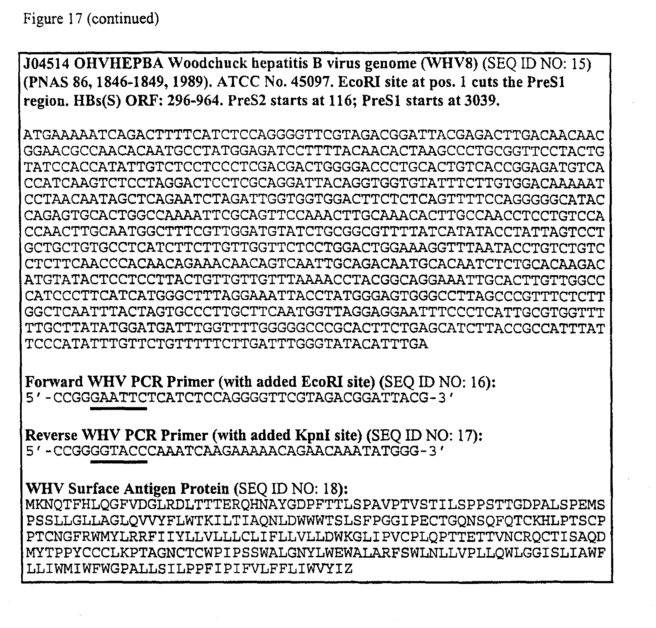

DNA sequences of HBsAg from different subtypes of HBV (*e.g.*, ayw and adr) and the related woodchuck hepatitis virus are prepared for shuffling. Comparison of the genes encoding these proteins suggests that recombination would occur at least ten times within 850 base pairs when shuffling the ayw and woodchuck hepatitis virus (WHV) DNA sequences. Nucleotide and amino acid sequences of portions of different subtypes of HBV are shown in Figure 17.

The sequence of the main HBsAg B-cell antigenic site (the "a" epitope) can be retained in the protein sequence by including the coding sequences of the external "a" loop in the final protein preparation. Peptide analogue(s) for the "a" epitope ofHBsAg have been described (Neurath *et al.* (1984) *J. ViroL Methods* 9:341-346), and the immunogenicity of the "a" epitope has been demonstrated (Bhatnagar *et al.* (1982) *Proc. Natl, Acad. Sci. USA* 79: 4400-4404). HBsAg and WHsAg share the major "a" determinant, and chimps can be protected by both antigens (Cote *et al.* (1986) *J. Virol.* 60: 895-901). Likewise, important CTL epitopes can be included in the protein in a defined way.

One can also easily introduce B or T (helper or CTL) epitopes from other antigens into the shuffled HBsAg sequence. This may focus the immune response to certain epitopes, independent of other potentially dominant epitopes from the same protein. Furthermore, the availability of the "a" loop on the HBsAg may provide a region of the envelope protein into which other artificial antigens or mimotopes could be included.

In all cases where a novel HBV envelope sequence is prepared to include a specific epitope (from HBV, another pathogen or a tumor cell), shuffling of the surrounding sequences in the HBV envelope will serve to optimize expression of the protein and help to ensure that the immune response is directed to the desired epitope.

Several methods of analyzing and utilizing shuffled HBsAg sequences are described below.

### A. Modulating expression levels of HBsAg

Shuffled HBsAg sequences are introduced into cells in culture and the ability to direct expression of secreted HBsAg (measured with clinical kits for HBsAg expression) is evaluated. This can be used to identify shuffled HBsAg sequences which exhibit optimized HBsAg expression levels. Such coding sequences are particularly interesting for DNA vaccination.

### B. Circumventing low responsiveness to the HBsAg

Shuffled HBsAg sequences are evaluated for their ability to induce an immune response to the clinically relevant HBsAg epitopes. This can be done using mice of the H-2s and H-2f haplotypes, which respond poorly or not at all to HBsAg protein immunization. In these experiments, one can verify that antibodies are generated to the main "a" epitope in the S protein, and a second protective epitope in the PreS2 region (a linear sequence).

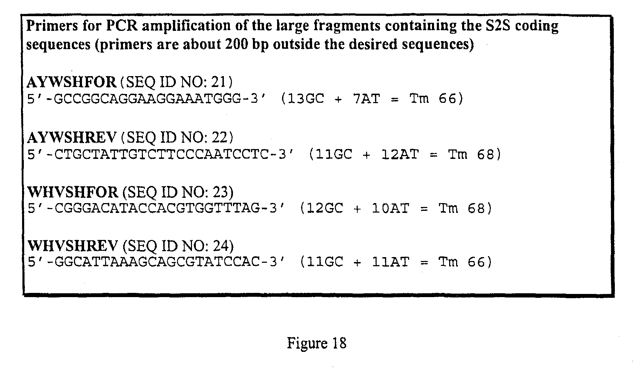

The PreS2 and S coding sequences for the envelope protein (HBsAg) from the HBV ayw subtype (plasmid pCAG-M-Kan; Whalen) and the WHV (plasmid pWHV8 from ATCC) are amplified from the two plasmids by PCR and shuffled. Examples of suitable primers for PCR amplification are shown in Figure 18. The shuffled library of sequences is cloned into an HBsAg-expression vector and individual colonies are chosen for preparation of plasmid DNA. The DNA is administered to the test animals and vectors which induce the desired immune response are identified and recovered.

### C. Presentation of natural HBsAg CTL epitopes by evolved HBsAg proteins

This example describes methods of using the evolved HBsAg protein to present natural HBsAg CTL epitopes. Shuffling is used to increase overall immunogenicity of the HBsAg protein, as discussed above. However, some of the evolved HBsAg sequences are replaced with class I or class II epitope sequences from the natural HBsAg protein in order to stimulate immunoreactivity specifically to these natural viral epitopes. Alternatively, the natural viral epitopes can be added to the evolved protein without loss of immunogenicity of the evolved HBsAg.

### D. Expression of tumor-devided CTL, epitopes by evolved HBsAg proteins

This example describes methods of using the evolved HBsAg protein is used to express tumor-derived CTL epitopes. The overall immunogenicity of the HBsAg protein is increased by shuffling. However, some of the evolved HBsAg sequences are replaced with class I or class II epitope sequences from tumor cells in order to stimulate immunoreactivity specifically to these natural viral epitopes. Alternatively, the tumor cells epitopes can be added to the evolved protein without loss of immunogenicity of the evolved HBsAg.

### E. Expression of minotope sequences by the HBsAg

This example describes the use of an evolved HBsAg protein for expression of mimotope sequences. Again, the evolved HBsAg protein is used to increase overall immunogenicity of the protein. However, some of the evolved HBsAg sequences are replaced with mimotope sequences to stimulate immunoreactivity specifically to the natural sequence which cross reacts with the mimotope. Alternatively, the mimotope sequences can be added to the evolved protein without loss of immunogenicity of the evolved HBsAg.

### Example 15

### Fusion Proteins Of The HBsAg Polypeptide and HIV gp120 Protein

This Example describes the preparation of fusion proteins ("chimeras") formed from the HBsAg polypeptide and the extracellular fragment gp120 of the HIV envelope protein, and their use as vaccines.

### Background

When used as a vaccine, recombinant monomeric gp120 has failed to induce antibodies that have strong neutralizing activity with primary isolates of the HIV virus. It has been suggested that oligomeric forms of the HIV envelope protein which expose certain regions of the tertiary structure would be better able to elicit virus-neutralizing antibodies (Parrin *et al.* (1997) *Immunol. Lett.* 57: 105-112; VanCott *et al.* (1997) *J. Virol.* 71: 4319-4330;

In this Example, DNA shuffling is applied to this problem, in order to obtain gp 120 polypeptides which adopt conformations slightly different from those of previous preparations of recombinant gp 120. To allow the individual gp 120 molecules to interact as oligomers, a fusion is prepared between gp120 sequences (on the N-terminus of the fusion) and HBsAg sequences (on the C-terminal of the fusion).

The N-terminal peptide sequence of the S region of the HBsAg polypeptide is a transmembrane structure which is locked into the membrane of the endoplasmic reticulum. The actual N-terminus of the S region as well as the preS2 sequences are located in the lumenal part of the ER. They are found on the outside of the final HBsAg particles. By placing the gp120 sequences on the N-terminus of the HBsAg preS2 or S sequences, the gp120 sequences are also located on the outside of the particles. The gp120 molecules can thus be brought together in three-dimensional space to interact as in the virus.

Since the exact conformation of the final chimera which will have the most appropriate immunogenicity cannot be predicted, DNA shuffling is employed. The sequences of the HBsAg polypeptide, which functions as a scaffold, and of gp120 are both shuffled. Screening of the shuffled products can be performed by ELISA assay using antibodies (polyclonal or monoclonal) which have previously been determined to have virus neutralizing activity.

### Method

The sequences encoding the gp 120 fragment of the HIV envelope protein are preferably prepared as a synthetic gene to include codons which are optimal for gene expression in mammals. The gp120 sequence will typically include a signal sequence on its N-terminal end.

The gp120 sequences are inserted into the preS2 region of an HBsAg-expressing plasmid. In the preS2 region of the plasmid pMKan and its derivatives, an *Eco*RI site and an *Xho*I site are available for cloning. The gp120 sequences can be inserted between these two sites, which brings the gp120 closer to the start of the S coding sequences, or into the *Eco*RI site alone, which leaves a spacer sequence of about 50 amino acids between the gp120 sequence and the start of the S region of the HBsAg. These two different cloning strategies will give rise to chimeric molecules in which the gp120 sequences are located at different distances from the transmembrane region of the HBsAg sequence. This may be advantageous in allowing the gp 120 sequences to adopt conformations which are more suitable immunogens than monomeric gp120. '

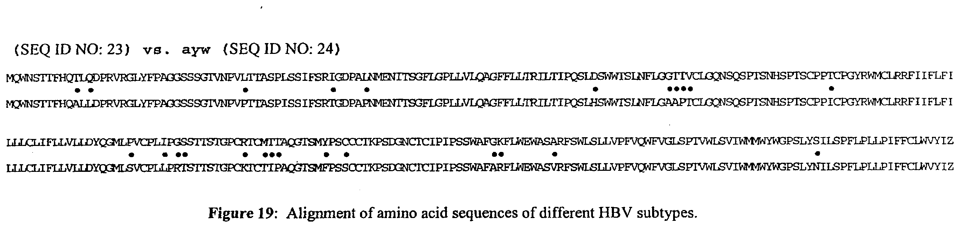

DNA shuffling of the entire chimeric sequence is carried out. Family shuffling is preferred; this involves the preparation of several gp120-HBsAg fusion proteins in which different gp120 and HBsAg (or WHV) sequences are used. An alignment of HBsAg nucleotide sequences is shown in Figure 19. After shuffling of the different sequences, the products are cloned into an expression vector such as pMKan. Pools of clones from the library of shuffled products are transfected into cultured cells and the secretion of chimeric proteins is assayed with broadly reactive antibodies to gp120. Positive clones can be further evaluated with particular antibodies that have demonstrated HIV neutralizing activity, for example the anti-CD4 binding domain recombinant human monoclonal antibody, IgGlb12 (Kessler *et al.* (1997) *AIDS Res. Hum. Retroviruses.* 1: 13: 575-582; Roben *et al.* (1994) *J. Virol.* 68: 4821-4828). Candidate clones can then be used to immunize mice and the antiserum obtained is evaluated for HIV virus-neutralizing activity in *in vitro* assays.

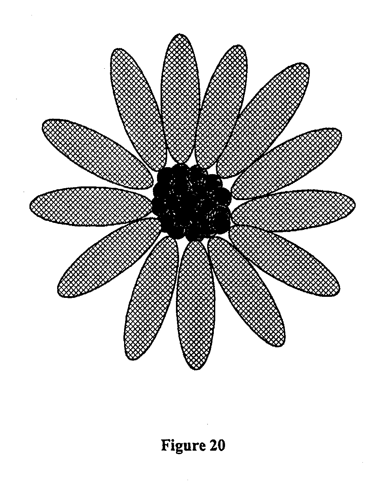

Because the gp120 molecule (approx. 1100 amino acids) is larger in size than the monomeric HBsAg preS2+S protein (282 amino acids), it is likely that not every HBsAg monomer in an aggregated particle will contain a gp120 sequence. Internal initiation of protein synthesis can take place on the HBsAg coding sequences at the initiator methionine that marks the beginning of the S region. Thus, the chimeric molecule (which contains the gp120 sequences) will be mixed in the cell with the S region and the multimeric particles should assemble with an appropriate number of chimeric polypeptides and native HBsAg S monomers. Alternatively, an S-expressing plasmid can be mixed with the plasmid expressing the chimera, or a single plasmid which expresses the chimera and the S form can be constructed. A diagram of the resulting particles is shown in Figure 20.

### Example 16

### DNA Shuffling Of HSV-1 And HSV-2 Glycoproteins B And/Or D As Means To Induce Enhanced Protective Immune Responses

This Example describes the use of DNA shuffling to obtain HSV glycoprotein B (gB) and glycoprotein D (gD) polypeptides that exhibit improved ability to induce protective immune responses upon administration to a mammal. Epidemiological studies have shown that prior infections with HSV-1 give partial protection against infections with HSV-2, indicating existence of cross-reactive immune responses. Based on previous vaccination studies, the main immunogenic glycoproteins in HSV appear to be gB and gD, which are encoded by 2.7 kb and 1.2 kb genes, respectively. The gB and gD genes of HSV-1 are about 85% identical to the corresponding gene of HSV-2, and the gB genes of each share little sequence identity with the gD genes. Baboon HSV-2 gB is appr. 75% identical to human HSV-1 or -2 gB, with rather long stretches of almost 90% identity. In addition, 60-75% identity is found in portions of the genes of equine and bovine herpesviruses.

Family shuffling is employed using as substrates nucleic acids that encode gB and/or gD from HSV-1 and HSV-2. Preferably, homologous genes are obtained from HSVs of various strains. An alignment of gD nucleotide sequences from HSV-1 and two strains of HSV-2 is shown in Figure 7. Antigens encoded by the shuffled nucleic acids are expressed and analyzed *in vivo.* For example, one can screen for improved induction of neutralizing antibodies and/or CTL responses against HSV-1/HSV-2. One can also detect protective immunity by challenging mice or guinea pigs with the viruses. Screening can be done using pools or individuals clones.

### Example 17

### Evolution Of HIV Gp120 Proteins For Induction Of Broad Spectrum Neutralizing Ab Responses

This Example describes the use of DNA shuffling to generate immunogens that crossreact among different strains of viruses, unlike the wild-type immunogens. Shuffling two kinds of envelope sequences can generate immunogens that induce neutralizing antibodies against a third strain.

Antibody-mediated neutralization of HIV-1 is strictly type-specific. Although neutralizing activity broadens in infected individuals over time, induction of such antibodies by vaccination has been shown to be extremely difficult. Antibody-mediated protection from HIV-1 infection in vivo correlates with antibody-mediated neutralization of virus in vitro.

Figure 8 illustrates the generation of libraries of shuffled gp120 genes. gp120 genes derived from HIV-1DH12 and HIV-IIIIB(NL43) are shuffled. The chimeric/mutant gp120 genes are then analyzed for their capacity to induce antibodies that have broad spectrum capacity to neutralize different strains of HIV. Individual shuffled gp120 genes are incorporated into genetic vaccine vectors, which are then introduced to mice by injection or topical application onto the skin. These antigens can also be delivered as purified recombinant proteins. The immune responses are measured by analyzing the capacity of the mouse sera to neutralize HIV growth *in vitro.* Neutralization assays are performed against HIV-1DH12, HIV-IIIIB and HIV-189.6. The chimeras/mutants that demonstrate broad spectrum neutralization are chosen for further rounds of shuffling and selection. Additional studies are performed in monkeys to illustrate the capacity of the shuffled gp120 genes to provide protection for subsequent infection with immunodeficiency virus.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference for all purposes.

## Claims

1. An recombinant multivalent antigenic polypeptide that comprises a first antigenic determinant of a first polypeptide and at least a second antigenic determinant from a second polypeptide.

2. The multivalent antigenic polypeptide of claim 1, wherein the polypeptide comprises at least a third antigenic determinant from a third polypeptide.

3. The multivalent antigenic polypeptide of claim 1, wherein the first and second polypeptides are selected from the group consisting of cancer antigens, antigens associated with autoimmunity disorders, antigens associated with inflammatory conditions, antigens associated with allergic reactions, and antigens from infectious agents.

4. The multivalent antigenic polypeptide of claim 3, wherein the antigens are from a virus, a parasite, or a bacteria.

5. The multivalent antigenic polypeptide of claim 4, wherein the antigens are from a virus selected from the group consisting of a Venezuelan equine encephalitis virus or a related alphavirus, a virus of the Japanese encephalitis virus complex, a virus of the tick-borne encephalitis virus complex, a Dengue virus, a Hanta virus, an HIV, a hepatitis B virus, a hepatitis C virus, and a *Herpes simplex* virus.

6. The multivalent antigenic polypeptide of claim 5, wherein the antigens are envelope proteins.

7. The multivalent antigenic polypeptide of claim 4, wherein the antigens are from a bacteria and are selected from the group consisting of a Yersinia V antigen, a *Staphylococcus aureus* enterotoxin, a *Streptococcus pyogenes* enterotoxin, a *Vibrio cholera* toxin, an enterotoxigenic *Escherichia coli* heat labile enterotoxin, a OspA and a OspC polypeptide from a *Borrelia* species, an Antigen 85 polypeptide from a *Mycobacterium* species, a VacA and a CagA polypeptide from *Helicobacter pylori*, and an MSP antigen from *Plasmodium falciparum.*

8. The multivalent antigenic polypeptide of claim 1, wherein the multivalent antigenic polypeptide exhibits reduced affinity to IgE from a mammal compared to the first or second polypeptides.

9. The multivalent antigenic polypeptide of claim 1, wherein the first antigenic determinant and the second antigenic determinant are from different serotypes of a pathogenic organism.

10. The multivalent antigenic polypeptide of claim 1, wherein the first antigenic determinant and the second antigenic determinant are from different species of pathogenic organism.

11. The multivalent antigenic polypeptide of claim 1, wherein the first polypeptide and the second polypeptide are allergens.

12. The multivalent antigenic polypeptide of claim 11, wherein the allergens are dust mite allergens, grass pollen allergens, birch pollen allergens, ragweed pollen allergens, hazel pollen allergens, cockroach allergens, rice allergens, olive tree pollen allergens, fungal allergens, mustard allergens, and bee venom.

13. The multivalent antigenic polypeptide of claim 1, wherein the first polypeptide and the second polypeptide are associated with an inflammatory or autoimmune disease.

14. The multivalent antigenic polypeptide of claim 13, wherein the first polypeptide and the second polypeptide are autoantigens associated with a disease selected from the group consisting of multiple sclerosis, scleroderma, systemic sclerosis, systemic lupus erythematosus, hepatic autoimmune disorder, skin autoimmune disorder, insulin-dependent diabetes mellitus, thyroid autoimmune disorder, and rheumatoid arthritis.

15. The multivalent antigenic polypeptide of claim 1, wherein the first polypeptide and the second polypeptide are cancer antigens or sperm antigens.

16. A recombinant antigen library comprising recombinant nucleic acids that encode antigenic polypeptides, wherein the library is obtained by recombining at least first and second forms of a nucleic acid which comprises a polynucleotide sequence that encodes a disease-associated antigenic polypeptide, wherein the first and second forms differ from each other in two or more nucleotides, to produce a library of recombinant nucleic acids.

17. The recombinant antigen library of claim 16, wherein the first and second polypeptides are toxins.

18. A method of obtaining a polynucleotide that encodes a recombinant antigen having improved ability to induce an immune response to a disease condition, the method comprising:
(1) recombining at least first and second forms of a nucleic acid which comprises a polynucleotide sequence that encodes an antigenic polypeptide that is associated with the disease condition, wherein the first and second forms differ from each other in two or more nucleotides, to produce a library of recombinant nucleic acids; and
(2) screening the library to identify at least one optimized recombinant nucleic acid that encodes an optimized recombinant antigenic polypeptide that has improved ability to induce an immune response to the disease condition.

19. The method of claim 18, wherein the method further comprises:
(3) recombining at least one optimized recombinant nucleic acid with a further form of the nucleic acid, which is the same or different from the first and second forms, to produce a further library of recombinant nucleic acids;
(4) screening the further library to identify at least one further optimized recombinant nucleic acid that encodes a polypeptide that has improved ability to induce an immune response to the disease condition; and
(5) repeating (3) and (4), as necessary, until the further optimized recombinant nucleic acid encodes a polypeptide that has improved ability to induce an immune response to the disease condition.

20. The method of claim 18, wherein the disease-associated polypeptides are selected from the group consisting of cancer antigens, antigens associated with autoimmunity disorders, antigens associated with inflammatory conditions, antigens associated with allergic reactions, and antigens associated with infectious agents.

21. The method of claim 18, wherein the disease condition is an infectious disease and the first and second forms of the nucleic acid each encode an antigen of a different serotype of a pathogenic agent.

22. The method of claim 21, wherein the first and second forms of the nucleic acid are each from a different species of pathogen.

23. The method of claim 21, wherein the screening is accomplished by:
introducing into a test animal either:
a) the library of recombinant nucleic acids, or
b) recombinant polypeptides encoded by the library of recombinant nucleic acids;
introducing the pathogenic agent into the test animal; and
determining whether the test animal is resistant to challenge by the pathogenic agent.

24. The method of claim 23, wherein the pathogenic agent introduced into the test animal is of a different serotype than that used as a source of the first and second forms of the nucleic acid.

25. The method of claim 23, wherein the library is subdivided into a plurality of pools, each of which pools is introduced into a test animal to identify those pools that include an optimized recombinant nucleic acid that encodes a polypeptide which has improved ability to induce an immune response to the pathogenic agent.

26. The method of claim 25, wherein the pools that include an optimized recombinant nucleic acid are further subdivided into a plurality of subpools, each of which subpools is introduced into a test animal to identify those pools that include an optimized recombinant nucleic acid that encodes a polypeptide which has improved ability to induce an immune response to the pathogenic agent.

27. The method of claim 18, wherein the optimized recombinant nucleic acid encodes a multivalent antigenic polypeptide and the screening is accomplished by:
expressing the library of recombinant nucleic acids in a phage display expression vector such that the recombinant antigen is expressed as a fusion protein with a phage polypeptide that is displayed on a phage particle surface;
contacting the phage with a first antibody that is specific for a first serotype of the pathogenic agent and selecting those phage that bind to the first antibody;
contacting those phage that bind to the first antibody with a second antibody that is specific for a second serotype of the pathogenic agent and selecting those phage that bind to the second antibody;
wherein those phage that bind to the first antibody and the second antibody express a multivalent antigenic polypeptide.

28. The method of claim 27, wherein the screening further comprises contacting those phage that bind to the first and second antibodies with one or more additional antibodies, each of which is specific for an additional serotype of the pathogenic agent, and selecting those phage that bind to the respective additional antibodies.

29. The method of claim 27, wherein the phage display expression vector comprises a suppressible stop codon between the recombinant nucleic acid and the phage polypeptide, whereby expression in a host cell which comprises a corresponding suppressor tRNA results in production of the fusion protein and expression in a host cell which lacks a corresponding suppressor tRNA results in production of the recombinant antigen not as a fusion protein.

30. The method of claim 18, wherein the optimized recombinant antigen exhibits an enhanced expression level in a host cell and the screening is accomplished by expression of each recombinant nucleic acid in the host cell and subjecting the host cells to flow cytometry-based cell sorting to obtain those host cells that display the recombinant antigen on the host cell surface.

31. The method of claim 18, wherein the improved property is selected from the group consisting of:
improved immunogenicity;
enhanced cross-reactivity against different forms of the disease-associated antigenic polypeptide;
reduced toxicity;
improved adjuvant activity *in vivo*; and
improved production of the immunogenic polypeptide.

32. The method of claim 31, wherein the improved property is enhanced cross-reactivity against different forms of the disease-associated polypeptide and the first and second forms of the nucleic acid are from a first and a second form of the disease-associated polypeptide.

33. The method of claim 32, wherein the first and second forms of the disease-associated polypeptide are obtained from at least a first and second species of a pathogenic agent and the optimized recombinant nucleic acid encodes a recombinant polypeptide that induces a protective response against both species of the pathogenic agent.

34. The method of claim 33, wherein the recombinant polypeptide induces a protective response against at least one additional species of the pathogenic agent.

35. The method of claim 33, wherein the pathogenic agent is a toxin.

36. The method of claim 33, wherein the pathogenic agent is a virus or a cell.

37. The method of claim 33, wherein the disease-associated polypeptide is a *Yersinia* V-antigen.

38. The method of claim 37, wherein the at least first and second forms of a nucleic acid are obtained from at least a first and second species of *Yersinia.*

39. The method of claim 38, wherein the *Yersinia* species are selected from the group consisting of *Y. pestis, Y. enterocolitica,* and *Y. pseudotuberculosis.*

40. The method of claim 33, wherein the pathogenic agent is a bacterial toxin.

41. The method of claim 18, wherein the disease condition is cancer and the screening step involves introducing the optimized recombinant nucleic acids into a genetic vaccine vector and testing library members for ability to inhibit proliferation of cancer cells or inducing death of cancer cells.

42. The method of claim 41, wherein the optimized recombinant nucleic acid comprises a nucleotide sequence that encodes a tumor specific antigen.

43. The method of claim 41, wherein the optimized recombinant nucleic acid comprises a nucleotide sequence that encodes a molecule which is capable of inhibiting proliferation of cancer cells.

44. The method of claim 18, wherein the disease condition is an inflammatory response which has an unknown or no antigen specificity and the screening step involves one or more of the following:
a) determining the ability of the genetic vaccine vector to induce cytokine production by PBMC, synovial fluid cells, purified T cells, monocytes/macrophages, dendritic cells, or T cell clones;
b) determining the ability of the genetic vaccine vector to induce T cell activation or proliferation; and
c) determining the ability of the genetic vaccine vector to induce T cell differentiation to T_{H}1 or T_{H}2 cells.

45. The method of claim 18, wherein the disease condition is an autoimmune response.

46. The method of claim 45, wherein the optimized recombinant antigenic polypeptide shifts the immune response from a T_{H}1-mediated response to a T_{H}2-mediated response.

47. The method of claim 18, wherein the disease condition is an allergic immune response.

48. The method of claim 47, wherein the optimized recombinant antigenic polypeptide shifts the immune response from a T_{H}2-mediated response to a T_{H}1-mediated response.

49. The method of claim 47, wherein the optimized recombinant antigenic polypeptide induces an immune response **characterized by** predominant IgG and IgM expression and reduced IgE expression.

50. The method of claim 47, wherein the optimized recombinant antigenic polypeptide is not recognized by pre-existing IgE molecules present in sera of atopic mammals.

51. The method of claim 50, wherein the optimized recombinant antigenic polypeptide retains T cell epitopes that are involved in modulating a T cell response.

52. A method of obtaining a recombinant viral vector which has an enhanced ability to induce an antiviral response in a cell, the method comprising the steps of:
(1) recombining at least first and second forms of a nucleic acid which comprise a viral vector, wherein the first and second forms differ from each other in two or more nucleotides, to produce a library of recombinant viral vectors;
(2) transfecting the library of recombinant viral vectors into a population of mammalian cells;
(3) staining the cells for the presence of Mx protein; and
(4) isolating recombinant viral vectors from cells which stain positive for Mx protein, wherein recombinant viral vectors from positive staining cells exhibit enhanced ability to induce an antiviral response.

53. The method of claim 52, wherein the viral vector comprises an influenza viral genomic nucleic acid.
